# EUROPEAN PATENT SPECIFICATION

(11) **EP 2 069 306 B1**
(45) Date of publication and mention of the grant of the patent: **12.05.2010**
(21) Application number: 07803403.0
(22) Date of filing: 11.09.2007
(51) Int. Cl.: C07D 215/12, C07D 215/18, C07D 215/20

(54) **QUINOLINYLMETHYL COMPOUNDS**
CHINOLINYLMETHYLVERBINDUNGEN
COMPOSÉS DE QUINOLINYLMÉTHYLE

(30) Priority: 12.09.2006 US 844023 P
(43) Date of publication of application: 17.06.2009
(73) Proprietor: BASF SE, 67056 Ludwigshafen (DE)
(72) Inventor: PUHL, Michael, 68623 Lampertheim (DE); PARRA RAPADO, Liliana, 77654 Offenburg (DE); RACK, Michael, 69214 Eppelheim (DE); LOHMANN, Jan Klaas, 67063 Ludwigshafen (DE); GRAMMENOS, Wassilios, 67071 Ludwigshafen (DE); VON DEYN, Wolfgang, 67435 Neustadt (DE); LANGEWALD, Jürgen, 68165 Mannheim (DE); BAUMANN, Ernst, 67373 Dudenhofen (DE); OLOUMI-SADEGHI, Hassan, Raleigh, NC 27614 (US); ANSPAUGH, Douglas D., Apex, NC 27502 (US); CULBERTSON, Deborah L., Fuquay Varina, NC 27526 (US)
(86) International application number: PCT/EP2007/059538
(87) International publication number: WO 2008/031824

(56) References cited:
- WO-A-2005/033081
- WO-A-2006/097488

## Description

The present invention relates to novel biphenyl-4-sulfonicacid (quinolin-4-ylmethyl)-amide compounds and the N-oxides, and salts thereof and their use for combating arthropod pests and nematodes, and also to compositions comprising such compounds as active component. The present invention also relates to compounds of formula (I) for use in a method for controlling arthropod pests or nematodes.

Animal pests and in particular arthropods and nematodes destroy growing and harvested crops and attack wooden dwelling and commercial structures, causing large economic loss to the food supply and to property. While a large number of pesticidal agents are known, due to the ability of target pests to develop resistance to said agents, there is an ongoing need for new agents for combating arthropods and nematodes. It is therefore an object of the present invention to provide compounds having a good pesticidal activity and show a broad activity spectrum against a large number of different animal pests, especially against difficult to control insects, arachnids and nematodes.

WO 2005/033081 describes fungicidal 4-pyridinylmethylsulfonamide derivatives. Those compounds may carry a benzene ring fused to the pyridine moiety.

WO 2006/097489 (PCT/EP/2006/060753) describes various 4-pyridylmethylamides of biphenylsulphonic acid, wherein the biphenyl moiety may carry substituents at the phenyl ring of the biphenyl moiety at the sulfonamide group. The compounds are used for combating arthropodal pests and for protecting materials against infestation and/or destruction by said pests.

WO 2006/097488 (PCT/EP/2006/060752) inter alia describes arthropodal quinolone compounds of the formula (A), 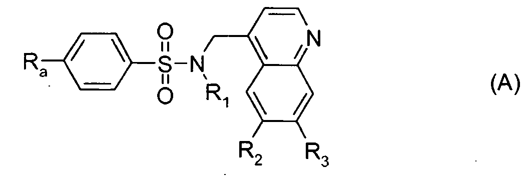 wherein R₂ and R₃ are both hydrogen, halogen, methoxy or trifluoromethoxy, R₁ is hydrogen or methyl and Rₐ is selected from phenyl which may be unsubstituted or may carry one substituent selected from chloro, C₁-C₄-alkyl, methoxy, trifluoromethoxy or phenyl.

US 60/782429 describes specific quinoline methylsulfonamides carrying a biphenyl moiety at the sulfonamide group wherein the phenylene moiety of biphenyl is unsubstituted.

There is an ongoing need to provide compounds which are useful for combating harmful arthropodes such as insects and arachnids. It is desirable that the compounds have an improved action and/or a broader activity spectrum against harmful arthropdes.

Surprisingly it has been found that this object is achieved by quinoline compounds of formula (I) 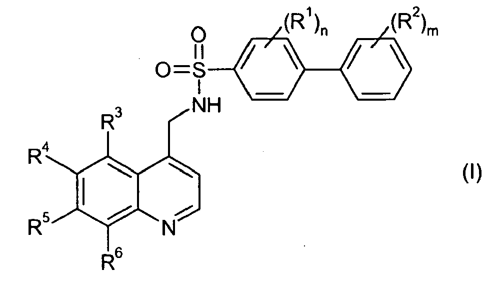 wherein
- R¹, R²: are each independently halogen, hydroxy, cyano, amino, nitro, C₁-C₆-alkyl, C₂-C₆-alkenyl, C₂-C₆-alkynyl, C₃-C₇-cydoalkyl, C₃-C₇-cydoalkyl-C₁-C₄-alkyl, C₁-C₆-alkoxy, C₂-C₆-alkenyloxy, C₂-C₆-alkynyloxy, C₁-C₄-alkoxy-C₁-C₄-alkoxy, C₃-C₇-cydoalkyl-C₁-C₄-alkoxy, C(OH)(CF₃)₂, C₁-C₆-haloalkyl, C₂-C₆-haloalkenyl, C₁-C₆-haloalkoxy, C₂-C₆-haloalkenyloxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, C(R^{a})=O or C(R^{a})=NOR^{b};
- Rₐ: is hydrogen or C₁-C₄-alkyl;
- R^{b}: is hydrogen, C₁-C₄-alkyl, C₂-C₄-alkenyl, C₂-C₄-alkynyl, C₁-C₄-haloalkyl, or C₂-C₄-haloalkenyl;
- R³, R⁴, R⁵, R⁶: are each independently hydrogen, halogen, cyano, amino, nitro, hydroxy, C₁-C₆-alkyl, C₁-C₆-alkoxy, C₁-C₆-haloalkyl, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, or C(=O)OR^{c};
- Rc: is hydrogen, C₁-C₆-alkyl, C₂-C₆-alkenyl, or C₂-C₆-alkynyl;
- m: is 0, 1, 2, 3, 4 or 5;
- n: is 1 or 2;
and the N-oxides or salts thereof.

Accordingly, the present invention realtes to quinoline compounds of the general formula (I) and the N-oxides and salts thereof.

The compounds of the present invention are also useful for combating arthropod pests and nematode pests. Therefore the present invention also relates to the use of the quinoline compounds of the general formula (I) and their N-oxides and salts for combating arthropod pests or nematode pests.

The present invention furthermore relates to compounds of formula (I) for use in a method for controlling arthropod pests or nematodes, which comprises contacting the insect, arachnid or nematode or their food supply, habitat, breeding ground or their locus with at least one compound of formula (I) and/or a N-oxide or a salt thereof. Furthermore, the present invention relates to a method for protecting growing plants from attack or infestation by arthropod pests or nematodes, which comprises applying to the plants, or to the soil or water in which they are growing, at least one compound of the formula (I) and/or a N-oxide or an agriculturally acceptable salt thereof. In a preferred embodiment of the above mentioned methods at least one compound of formula (I) and/or N-oxide or the salt thereof or a composition comprising them is applied in an amount of from 5 g/ha to 2000 g/ha, calculated as the compound of formula (I).

Furthermore, the present invention relates to a method for protecting seeds with at least one compound of formula (I) and/or a N-oxide or an agriculturally acceptable salt thereof or a composition containing at least one of these compounds (I) in pesticidally effective amounts. Preferably, at least one compound of formula (I) and/or a N-oxide or an agriculturally acceptable salt thereof or a composition comprising at least one of these compounds is applied in an amount of from 0.1 g to 10 kg per 100 kg of seeds.

Accordingly, a further object of the present invention is seed, comprising at least one compound of formula (I) and/or a N-oxide or an agriculturally acceptable salt thereof, preferably in an amount of from 0.1 g to 10 kg per 100 kg of seeds, calculated as the compound of formula (I).

The present invention also relates to compounds of formula (I) for use in a method for treating, controlling, preventing or protecting animals against infestation or infection by parasites which comprises administering or applying to the animals a parasiticidally effective amount of at least one compound of formula (I) and/or a N-oxide or a veterinarily acceptable salt thereof.

The present invention also relates to synergistic pesticidal mixtures, comprising a compound of formula (I) and/or a N-oxide or a salt thereof and a pesticide selected from the organo(thio)phosphates, carbamates, pyrethroids, growth regulators, neonicotinoids, nicotinic receptor agonists/antagonists compounds, GABA antagonist compounds, macrocyclic lactone insecticides, METI I, II and III compounds, oxidative phosphorylation inhibitor compounds, moulting disruptor compounds, mixed function oxidase inhibitor compounds, sodium channel blocker compounds, benclothiaz, bifenazate, cartap, flonicamid, pyridalyl, pymetrozine, sulfur, thiocyclam, flubendiamide, cyanopyrafen, flupyrazofos, cyflumetofen, amidoflumet, anthranilamides and N-R'-2,2-dihalo-1-R"-cyclopropanecarboxamide-2-(2,6-dichloro-α,α,α-trifluoro-p-tolyl)hydrazone or N-R'-2,2-di(R"')-propionamide-2-(2,6-dichloro-α,α,α-trifluoro-p-tolyl)-hydrazone, wherein R' is methyl or ethyl, halo is chloro or bromo, R" is hydrogen or methyl and R"' is methyl or ethyl.

Depending on the substitution pattern, the compounds of the formula (I) and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

Agriculturally useful salts of the compounds (I) encompass especially the salts of those cations or the acid addition salts of those acids whose cations and anions, respectively, have no adverse effect on the pesticidal action of the compounds (I). Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting compounds of formula (I) with an acid of the corresponding anion, preferably of hydrochloric acid, hydrobromic acid, sulfuric acid, phosphoric acid or nitric acid.

Veterinarily acceptable salts of the compounds of formula (I) encompass especially the salts of those cations or the acid addition salts which are known and accepted in the art for the formation of salts for veterinary use. Suitable acid addition salts, e.g. formed by compounds of formula (I) containing a basic nitrogen atom, e.g. an amino group, include salts with inorganic acids, for example hydrochlorids, sulphates, phosphates, and nitrates and salts of organic acids for example acetic acid, maleic acid, e.g. the monoacid salts or diacid salts of maleic acid, dimaleic acid, fumaric acid, e.g. the monoacid salts or diacid salts of fumaric acid, difumaric acid, methane sulfenic acid, methane sulfonic acid, and succinic acid.

In the definitions of the variables given above, collective terms are used which are generally representative for the substituents in question. The term Cₙ-Cₘ indicates the number of carbon atoms possible in each case in the substituent or substituent moiety in question.

The term "halogen" as used herein refers to fluoro, chloro, bromo and iodo.

The term "alkyl" as used herein refers to a straight-chained or branched saturated hydrocarbon group having 1 to 6 carbon atoms, for example methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

The term "haloalkyl" as used herein refers to a straight-chained or branched alkyl group having 1 to 6 carbon atoms (as mentioned above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 5-fluoro-1-pentyl, 5-chloro-1-pentyl, 5-bromo-1-pentyl, 5-iodo-1-pentyl, 5,5,5-trichloro-1-pentyl, undecafluoropentyl, 6-fluoro-1-hexyl, 6-chloro-1-hexyl, 6-bromo-1-hexyl, 6-iodo-1-hexyl, 6,6,6-trichloro-1-hexyl or dodecafluorohexyl.

Accordingly "alkoxy" and "alkylthio" as used herein refer to straight-chain or branched alkyl groups having 1 to 6 carbon atoms (as mentioned above) bonded through an oxygen atom or a sulfur atom respectively, at any position in the alkyl group. Examples include methoxy, ethoxy, propoxy, isopropoxy, methylthio, ethylthio, propylthio, isopropylthio, and n-butylthio.

Similarly, "alkylsulfinyl" and "alkylsulfonyl" refer to straight-chain or branched alkyl groups having 1 to 6 carbon atoms (as mentioned above) bonded through a -S(=O)-moiety or a -S(=O)₂- moiety, respectively, at any position in the alkyl group. Examples include methylsulfinyl and methylsulfonyl.

Similarly, "haloalkoxy" and "haloalkylthio" (or haloalkylsulfenyl, respectively) refer to straight-chain or branched alkyl groups having 1 to 6 carbon atoms (as mentioned above) bonded through an oxygen atom or a sulfur atom, respectively, at any bond in the alkyl group, where some or all of the hydrogen atoms in these groups may be replaced by halogen atoms as mentioned above, for example C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy, further C₁-C₂-haloalkylthio, such as chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethytthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like.

The term "C₁-C₄-alkoxy-C₁-C₄-alkoxy" as used herein refers to alkoxy having 1 to 4 carbon atoms (as mentioned above), wherein one hydrogen atom of the alkoxy radical is replaced by a C₁-C₄-alkoxy group.

The term "alkenyl" as used herein intends a branched or unbranched unsaturated hydrocarbon group having 2 to 6 carbon atoms and a double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl; 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;

The term "alkynyl" as used herein refers to a branched or unbranched unsaturated hydrocarbon group having 2 to 6 carbon atoms and containing at least one triple bond, such as ethynyl, propynyl, 1-butynyl, 2-butynyl, and the like.

Similarly, "alkenyloxy" and "alkynyloxy" refer to straight-chain or branched alkenyl or alkynyl groups, respectively (as mentioned above) bonded through an oxygen atom, at any carbon of the alkenyl group, or at any carbon atom of the alkynyl group, e.g. allyloxy or propargyloxy.

The term "cycloalkyl" as used herein refers to monocyclic 3- to 7-membered saturated carbon atom rings, such as cyclopropyl, cyclobutyl, cyclopentyl, cyclohexyl, or cycloheptyl.

The term "C₃-C₇-cydoalkyl-C₁-C₄-alkyl" as used herein refers to alkyl having 1 to 4 carbon atoms (as mentioned above), wherein one hydrogen atom of the alkyl radical is replaced by a C₃-C₇-cycloalkyl group.

Similarly, the term "C₃-C₇-cycloalkyl-C₁-C₄-alkoxy" as used herein refers to alkoxy having 1 to 4 carbon atoms (as mentioned above), wherein one hydrogen atom of the alkoxy radical is replaced by_a C₃-C₇-cycloalkyl group.

With respect to the intended use of the compounds of formula (I), particular preference is given to the following meanings of the substituents R¹ to R⁶ and the variables n and m, in each case on their own or in combination:
Preference is given to compounds of the formula (I), wherein R¹ is selected independently from the group consisting of halogen, cyano, C₁-C₆-alkyl, C₁-C₆-haloalkyl, C₂-C₆-alkenyl, C₂-C₆-haloalkenyl, C₂-C₆-alkynyl, C₁-C₆-alkoxy, C₁-C6-haloalkoxy, C₂-C₆-alkenyloxy, C₂-C₆-haloalkenyloxy, C₂-C₆-alkynyloxy, C₁-C₆-alkylthio, C₁-C₆-haloalkylthio, C₁-C₆-alkylsulfonyl or C₁-C₆-haloalkylsulfonyl. Particular preference is given to compounds of the formula (I), in which R¹ is selected independently from the group consisting of halogen, in particular F, Cl, Br; C₁-C₂-alkyl, in particular CH₃, C₁-C₂-haloalkyl, especially C₁-C₂-fluoroalkyl, such as CHF₂, CF₃, C₁-C₂-alkoxy, in particular OCH₃, and C₁-C₂-haloalkoxy, especially C₁-C₂-fluoroalkoxy, such as OCHF₂ and OCF₃;
Preference is given to compounds of the formula (I), in which m is 1, 2 or 3;
Preference is given to compounds of the formula (I), in which R² is selected independently of one another from the group consisting of halogen, cyano, C₁-C₆-alkyl, C₁-C₆-haloalkyl, C₂-C₆-alkenyl, C₂-C₆-haloalkenyl, C₂-C₆-alkynyl, C₁-C₆-alkoxy, C₁-C₆-haloalkoxy, C₂-C₆-alkenyloxy, C₂-C₆-haloalkenyloxy,C₂-C₆-alkynyloxy, C₁-C₆-alkylthio, C₁-C₆-haloalkylthio, C₁-C₆-alkylsulfonyl or C₁-C₆-haloalkylsulfonyl. Particular preference is given to compounds of the formula (I), in which R² is selected independently from the group consisting of halogen, in particular F, Cl, Br; C₁-C₂-alkyl, in particular CH₃, C₁-C₃-haloalkyl, especially C₁-C₃-fluoroalkyl, such as CHF₂, CF₃ and CF(CF₃)₂; C₁-C₂-alkoxy, in particular OCH₃,and C₁-C₃-haloalkoxy, especially C₁-C₃-fluoroalkoxy, such as OCH2. OCF₃, OCF(CF₃)₂;
Preference is given to compounds of the formula (I), in which R³, R⁴, R⁵, R⁶ are each independently selected from the group consisting of hydrogen, halogen, C₁-C₆-alkyl, C₁-C₆-alkoxy, C₁-C₆-haloalkyl, C₁-C₆-haloalkoxy, or C₁-C₆-alkylthio.

A very preferred embodiment of the present invention relates to compounds of formula (I), wherein R³, R⁴, R⁵, R⁶ are each hydrogen.

Another preferred embodiment of the present invention relates to compounds of the formula (I), wherein n is 1.

A very preferred embodiment of the present invention relates to compounds of formula (I), wherein the substituent R¹ is attached to the phenyl group in meta position relative to the sulfonamid moiety. This compound is also referred to as compound of formula (I.1), wherein the biphenyl moiety has the substitution pattern as in the following scheme, and R¹, R², R³, R⁴, R⁵, R⁶ and m are as defined hereinabove for compounds of formula (I):

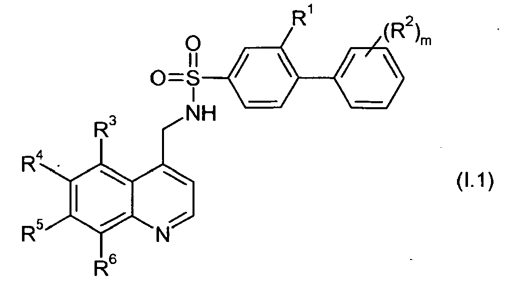

A skilled person will readily understand that the preferences given for R¹ to R⁶ and m in connection with compounds of formula (I) also apply for formulae (I.1) and (I.1a) as defined hereinafter.

In the compounds of formula (I.1) preference is given to those wherein R¹ is selected from the group fluoro, chloro, bromo, methyl, methoxy, trifluoromethyl, methylthio, trifluoromethylthio, and trifluormethoxy.

Preferred embodiments of such compounds include:
Compounds of formula (I.1) wherein R³, R⁴, R⁵ and R⁶ are as defined hereinabove and m is 0;
Compounds of formula (I.1) wherein R², R³, R⁴, R⁵ and R⁶ are as defined hereinabove and m is 1;
Compounds of formula (I.1) wherein R², R³, R⁴, R⁵ and R⁶ are as defined hereinabove and m is 2;
Compounds of formula (I.1) wherein R², R³, R⁴, R⁵ and R⁶ are as defined hereinabove and m is 3.

Particular preference is also given to compounds of formula (I.1) wherein R¹ is F, Cl or Br.

Further preference is given to compounds of formula (I.1) wherein R² is selected independently from the group consisting of halogen, cyano, nitro, C₁-C₆-alkyl, C₁-C₆-haloalkyl and C₁-C₆-alkoxy.

Among the compounds of formula (I.1), particular preference is given to compounds of formula (I.1), wherein R¹ is attached to the phenyl group in meta position relative to the sulfonamid moiety, n is 1 and R³, R⁴, R⁵, R⁶ are each hydrogen, in the following referred to as quinoline compounds of formula (I.1.a) 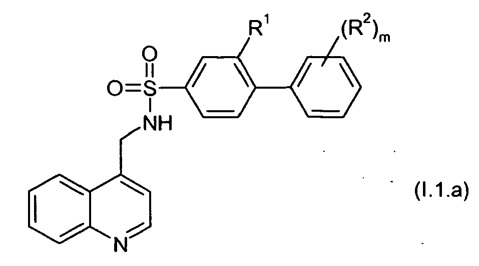 wherein
R¹, R² and m have the meanings given above, especially those mentioned as being preferred.

Another very preferred embodiment of the present invention relates to compounds of formula (I), wherein the substituent R¹ is attached to the phenyl group in ortho position relative to the sulfonamid moiety. This compound is also referred to as compound of formula (I).2, wherein the biphenyl moiety has the substitution pattern as in the following scheme, and R¹, R², R³, R⁴, R⁵, R⁶ and m are as defined hereinabove for compounds of formula (I):

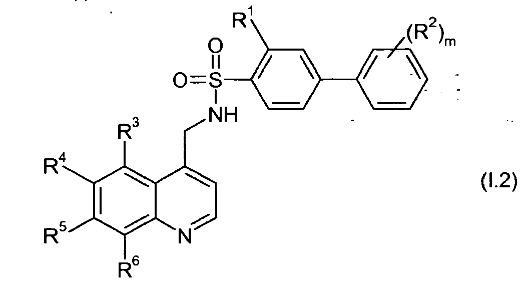

A skilled person will readily understand that the preferences given for R¹ to R⁶ and m in connection with compounds of formula (I) also apply for formulae (1.2) and (I.2.a) as defined hereinafter.

In the compounds of formula (1.2) preference is given to those wherein R¹ is selected from the group fluoro, chloro, bromo, methyl, methoxy, trifluoromethyl, methylthio, trifluoromethylthio, and trifluormethoxy.

Preferred embodiments of such compounds include:
Compounds of formula (I.2) wherein R³, R⁴, R⁵ and R⁶ are as defined hereinabove and m is 0;
Compounds of formula (1.2) wherein R², R³, R⁴, R⁵ and R⁶ are as defined hereinabove and m is 1;
Compounds of formula (1.2) wherein R², R³, R⁴, R⁵ and R⁶ are as defined hereinabove and m is 2;
Compounds of formula (1.2) wherein R², R³, R⁴, R⁵ and R⁶ are as defined hereinabove and m is 3.

Particular preference is also given to compounds of formula (I.2) wherein R¹ is F, Cl or Br.

Further preference is given to compounds of formula (1.2) wherein R² is selected independently from the group consisting of halogen, cyano, nitro, C₁-C₆-alkyl, C₁-C₆-haloalkyl and C₁-C₆-alkoxy.

Among the compounds of formula (1.2), particular preference is given to compounds of formula (I), wherein R¹ is attached to the phenyl group in ortho position relative to the sulfonamid moiety, n is 1 and R³, R⁴, R⁵, R⁶ are each hydrogen, in the following referred to as quinoline compounds of formula (I.2.a), 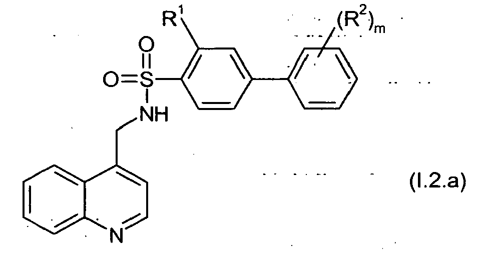 wherein
R¹, R² and m have the meanings given above, especially those mentioned as being preferred.

Examples of suitable quinoline moieties Q are given in the following table A 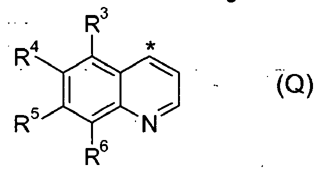 In formula (Q) * denotes the bonding site to the backbone.

**Table A**

| Quinoline moiety no. | R³ | R⁴ | R⁵ | R⁶ |
|---|---|---|---|---|
| Q-1 | H | H | H | H |
| Q-2 | F | H | H | H |
| Q-3 | H | F | H | H |
| Q-4 | H | H | F | H |
| Q-5 | H | H | H | F |
| Q-6 | - Cl | H | H | H |
| Q-7 | H | Cl | H | H |
| Q-8 | H | H | Cl | H |
| Q-9 | H | H | H | Cl |
| Q-10 | CH₃ | H | H | H |
| Q-11 | H | CH₃ | H | H |
| Q-12 | H | H | CH₃ | H |
| Q-13 | H | H | H | CH₃ |
| Q-14 | OCH₃ | H | H | H |
| Q-15 | H | OCH₃ | H | H |
| Q-16 | H | H | OCH₃ | H |
| Q-17 | H | H | H | OCH₃ |
| Q-18 | CF₃ | H | H | H |
| Q-19 | H | CF₃ | H | H |
| Q-20 | H | H | CF₃ | H |
| Q-21 | H | H | H | CF₃ |
| Q-22 | OCF₃ | H | H | H |
| Q-23 | H | OCF₃ | H | H |
| Q-24 | H | H | OCF₃ | H |
| Q-25 | H | H | H | OCF₃ |
| Q-26 | Cl | Cl | H | H |
| Q-27 | Cl | H | Cl | H |
| Q-28 | Cl | H | H | Cl |
| Q-29 | H | Cl | Cl | H |
| Q-30 | H | Cl | H | Cl |
| Q-31 | H | H | Cl | Cl |
| Q-32 | F | F | H | H |
| Q-33 | F | H | F | H |
| Q-34 | F | H | H | F |
| Q-35 | H | F | F | H |
| Q-36 | H | F | H | F |
| Q-37 | H | H | F | F |
| Q-38 | CH₃ | CH₃ | H | H |
| Q-39 | CH₃ | H | CH₃ | H |
| Q-40 | CH₃ | H | H | CH₃ |
| Q-41 | H | CH₃ | CH₃ | H |
| Q-42 | H | CH₃ | H | CH₃ |
| Q-43 | H | H | CH₃ | CH₃ |
| Q-44 | OCH₃ | OCH₃ | H | H |
| Q-45 | OCH₃ | H | OCH₃ | H |
| Q-46 | OCH₃ | H | H | OCH₃ |
| Q-47 | H | OCH₃ | OCH₃ | H |
| Q-48 | H | OCH₃ | H | OCH₃ |
| Q-49 | H | H | OCH₃ | OCH₃ |
| Q-50 | CF₃ | CF₃ | H | H |
| Q-51 | CF₃ | H | CF₃ | H |
| Q-52 | CF₃ | H | H | CF₃ |
| Q-53 | H | CF₃ | CF₃ | H |
| Q-54 | H | CF₃ | H | CF₃ |
| Q-55 | H | H | CF₃ | CF₃ |

With respect to their use, particular preference is given to the compounds of formula (I) compiled in the tables below. Moreover, the groups mentioned for a substituent in the tables are on their own, independently of the combination in which they are mentioned, a particularly preferred embodiment of the substituent in question.

### Table 1

Compounds of the formula (I.A) wherein Q denotes Q-1 and the combination of (R¹)ₙ and (R²)ₘ in each case corresponds to one line of Table B.

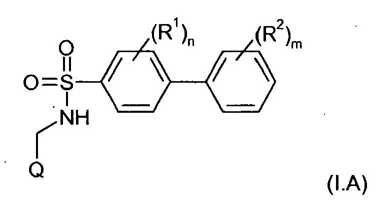

**Table B**

| No. | (R¹)ₙ | (R²)ₘ | | No. | (R¹)ₙ | (R²)ₘ |
|---|---|---|---|---|---|---|
| A-1 | 2-F | --- | | A-9 | 3-CH₃ | --- |
| A-2 | 2-Cl | --- | | A-10 | 3-CF₃ | --- |
| A-3 | 2-CH₃ | --- | | A-11 | 3-OCH₃ | --- |
| A-4 | 2-CF₃ | --- | | A-12 | 3-OCF₃ | --- |
| A-5 | 2-OCH₃ | --- | | A-13 | 2-F | 2-CH₃ |
| A-6 | 2-OCF₃ | --- | | A-14 | 2-Cl | 2-CH₃ |
| A-7 | 3-F | --- | | A-15 | 2-CH₃ | 2-CH₃ |
| A-8 | 3-Cl | --- | | A-16 | 2-CF₃ | 2-CH₃ |
| A-17 | 2-OCH₃ | 2-CH₃ | | A-47 | 3-OCH₃ | 2-Br |
| A-18 | 2-OCF₃ | 2-CH₃ | | A-48 | 3-OCF₃ | 2-Br |
| A-19 | 3-F | 2-CH₃ | | A-49 | 2-F | 2-Cl |
| A-20 | 3-Cl | 2-CH₃ | | A-50 | 2-Cl | 2-Cl |
| A-21 | 3-CH₃ | 2-CH₃ | | A-51 | 2-CH₃ | 2-Cl |
| A-22 | 3-CF₃ | 2-CH₃ | | A-52 | 2-CF₃ | 2-Cl |
| A-23 | 3-OCH₃ | 2-CH₃ | | A-53 | 2-OCH₃ | 2-Cl |
| A-24 | 3-OCF₃ | 2-CH₃ | | A-54 | 2-OCF₃ | 2-Cl |
| A-25 | 2-F | 2-CH₂CH₃ | | A-55 | 3-F | 2-Cl |
| A-26 | 2-Cl | 2-CH₂CH₃ | | A-56 | 3-Cl | 2-Cl |
| A-27 | 2-CH₃ | 2-CH₂CH₃ | | A-57 | 3-CH₃ | 2-Cl |
| A-28 | 2-CF₃ | 2-CH₂CH₃ | | A-58 | 3-CF₃ | 2-Cl |
| A-29 | 2-OCH₃ | 2-CH₂CH₃ | | A-59 | 3-OCH₃ | 2-Cl |
| A-30 | 2-OCF₃ | 2-CH₂CH₃ | | A-60 | 3-OCF₃ | 2-Cl |
| A-31 | 3-F | 2-CH₂CH₃ | | A-61 | 2-F | 2-F |
| A-32 | 3-Cl | 2-CH₂CH₃ | | A-62 | 2-Cl | 2-F |
| A-33 | 3-CH₃ | 2-CH₂CH₃ | | A-63 | 2-CHF3 | 2-F |
| A-34 | 3-CF₃ | 2-CH₂CH₃ | | A-64 | 2-CF₃ | 2-F |
| A-35 | 3-OCH₃ | 2-CH₂CH₃ | | A-65 | 2-OCH₃ | 2-F |
| A-36 | 3-OCF₃ | 2-CH₂CH₃ | | A-66 | 2-OCF₃ | 2-F |
| A-37 | 2-F | 2-Br | | A-67 | 3-F | 2-F |
| A-38 | 2-Cl | 2-Br | | A-68 | 3-Cl | 2-F |
| A-39 | 2-CH₃ | 2-Br | | A-69 | 3-CH₃ | 2-F |
| A-40 | 2-CF₃ | 2-Br | | A-70 | 3-CF₃ | 2-F |
| A-41 | 2-OCH₃ | 2-Br | | A-71 | 3-OCH₃ | 2-F |
| A-42 | 2-OCF₃ | 2-Br | | A-72 | 3-OCF₃ | 2-F |
| A-43 | 3-F | 2-Br | | A-73 | 2-F | 2-CN |
| A-44 | 3-Cl | 2-Br | | A-74 | 2-Cl | 2-CN |
| A-45 | 3-CH₃ | 2-Br | | A-75 | 2-CH₃ | 2-CN |
| A-46 | 3-CF₃ | 2-Br | | A-76 | 2-CF₃ | 2-CN |
| A-77 | 2-OCH₃ | 2-CN | | A-107 | 3-OCH₃ | 2-OCH₃ |
| A-78 | 2-OCF₃ | 2-CN | | A-108 | 3-OCF₃ | 2-OCH₃ |
| A-79 | 3-F | 2-CN | | A-109 | 2-F | 2-CF₃ |
| A-80 | 3-Cl | 2-CN | | A-110 | 2-Cl | 2-CF₃ |
| A-81 | 3-CH₃ | 2-CN | | A-111 | 2-CH₃ | 2-CF₃ |
| A-82 | 3-CF₃ | 2-CN | | A-112 | 2-CF₃ | 2-CF₃ |
| A-83 | 3-OCH₃ | 2-CN | | A-113 | 2-OCH₃ | 2-CHF₃ |
| A-84 | 3-OCF₃ | 2-CN | | A-114 | 2-OCF₃ | 2-CF₃ |
| A-85 | 2-F | 2-NO₂ | | A-115 | 3-F | 2-CF₃ |
| A-86 | 2-Cl | 2-NO₂ | | A-116 | 3-Cl | 2-CF₃ |
| A-87 | 2-CH₃ | 2-NO₂ | | A-117 | 3-CH₃ | 2-CF₃ |
| A-88 | 2-CF₃ | 2-NO₂ | | A-118 | 3-CF₃ | 2-CHF₃ |
| A-89 | 2-OCH₃ | 2-NO₂ | | A-119 | 3-OCH₃ | 2-CF₃ |
| A-90 | 2-OCF₃ | 2-NO₂ | | A-120 | 3-OCF₃ | 2-CF₃ |
| A-91 | 3-F | 2-NO₂ | | A-121 | 2-F | 2-OCF₃ |
| A-92 | 3-Cl | 2-NO₂ | | A-122 | 2-Cl | 2-OCF₃ |
| A-93 | 3-CH₃ | 2-NO₂ | | A-123 | 2-CH₃ | 2-OCF₃ |
| A-94 | 3-CF₃ | 2-NO₂ | | A-124 | 2-CF₃ | 2-OCF₃ |
| A-95 | 3-OCH₃ | 2-NO₂ | | A-125 | 2-OCH₃ | 2-OCF₃ |
| A-96 | 3-OCF₃ | 2-NO₂ | | A-126 | 2-OCF₃ | 2-OCF₃ |
| A-97 | 2-F | 2-OCH₃ | | A-127 | 3-F | 2-OCF₃ |
| A-98 | 2-Cl | 2-OCH₃ | | A-128 | 3-Cl | 2-OCF₃ |
| A-99 | 2-CH₃ | 2-OCH₃ | | A-129 | 3-CH₃ | 2-OCF₃ |
| A-100 | 2-CF₃ | 2-OCH₃ | | A-130 | 3-CF₃ | 2-OCF₃ |
| A-101 | 2-OCH₃ | 2-OCH₃ | | A-131 | 3-OCH₃ | 2-OCF₃ |
| A-102 | 2-OCF₃ | 2-OCH₃ | | A-132 | 3-OCF₃ | 2-OCF₃ |
| A-103 | 3-F | 2-OCH₃ | | A-133 | 2-F | 2-CF(CF₃)₂ |
| A-104 | 3-Cl | 2-OCH3 | | A-134 | 2-Cl | 2-CF(CF₃)₂ |
| A-105 | 3-CH₃ | 2-OCH₃ | | A-135 | 2-CH₃ | 2-CF(CF₃)₂ |
| A-106 | 3-CF₃ | 2-OCH₃ | | A-136 | 2-CF₃ | 2-CF(CF₃)₂ |
| A-137 | 2-OCH₃ | 2-CF(CF₃)₂ | | A-167 | 3-OCH₃ | 3-CH₃ |
| A-138 | 2-OCF₃ | 2-CF(CF₃)₂ | | A-168 | 3-OCF₃ | 3-CH₃ |
| A-139 | 3-F | 2-CF(CF₃)₂ | | A-169 | 2-F | 3-CH₂CH₃ |
| A-140 | 3-Cl | 2-CF(CF₃)₂ | | A-170 | 2-Cl | 3-CH₂CH₃ |
| A-141 | 3-CH₃ | 2-CF(CF₃)₂ | | A-171 | 2-CH₃ | 3-CH₂CH₃ |
| A-142 | 3-CF₃ | 2-CF(CF₃)₂ | | A-172 | 2-CF₃ | 3-CH₂CH₃ |
| A-143 | 3-OCH₃ | 2-CF(CF₃)₂ | | A-173 | 2-OCH₃ | 3-CH₂CH₃ |
| A-144 | 3-OCF₃ | 2-CF(CF₃)₂ | | A-174 | 2-OCF₃ | 3-CH₂CH₃ |
| A-145 | 2-F | 2-SCH₃ | | A-175 | 3-F | 3-CH₂CH₃ |
| A-146 | 2-Cl | 2-SCH₃ | | A-176 | 3-Cl | 3-CH₂CH₃ |
| A-147 | 2-CH₃ | 2-SCH₃ | | A-177 | 3-CH₃ | 3-CH₂CH₃ |
| A-148 | 2-CF₃ | 2-SCH₃ | | A-178 | 3-CF₃ | 3-CH₂CH₃ |
| A-149 | 2-OCH₃ | 2-SCH₃ | | A-179 | 3-OCH₃ | 3-CH₂CH₃ |
| A-150 | 2-OCF₃ | 2-SCH₃ | | A-180 | 3-OCF₃ | 3-CH₂CH₃ |
| A-151 | 3-F | 2-SCH₃ | | A-181 | 2-F | 3-Br |
| A-152 | 3-Cl | 2-SCH₃ | | A-182 | 2-Cl | 3-Br |
| A-153 | 3-CH₃ | 2-SCH₃ | | A-183 | 2-CH₃ | 3-Br |
| A-154 | 3-CF₃ | 2-SCH₃ | | A-184 | 2-CF₃ | 3-Br |
| A-155 | 3-OCH₃ | 2-SCH₃ | | A-185 | 2-OCH₃ | 3-Br |
| A-156 | 3-OCF₃ | 2-SCH₃ | | A-186 | 2-OCF₃ | 3-Br |
| A-157 | 2-F | 3-CH₃ | | A-187 | 3-F | 3-Br |
| A-158 | 2-Cl | 3-CH₃ | | A-188 | 3-Cl | 3-Br |
| A-159 | 2-CH₃ | 3-CH₃ | | A-189 | 3-CH₃ | 3-Br |
| A-160 | 2-CF₃ | 3-CH₃ | | A-190 | 3-CF₃ | 3-Br |
| A-161 | 2-OCH₃ | 3-CH₃ | | A-191 | 3-OCH₃ | 3-Br |
| A-162 | 2-OCF₃ | 3-CH₃ | | A-192 | 3-OCF₃ | 3-Br |
| A-163 | 3-F | 3-CH3 | | A-193 | 2-F | 3-Cl |
| A-164 | 3-Cl | 3-CH₃ | | A-194 | 2-Cl | 3-Cl |
| A-165 | 3-CH₃ | 3-CH₃ | | A-195 | 2-CH₃ | 3-Cl |
| A-166 | 3-CF₃ | 3-CH₃ | | A-196 | 2-CF₃ | 3-Cl |
| A-197 | 2-OCH₃ | 3-Cl | | A-227 | 3-OCH₃ | 3-CN |
| A-198 | 2-OCF₃ | 3-Cl | | A-228 | 3-OCF₃ | 3-CN |
| A-199 | 3-F | 3-Cl | | A-229 | 2-F | 3-NO₂ |
| A-200 | 3-Cl | 3-Cl | | A-230 | 2-Cl | 3-NO₂ |
| A-201 | 3-CH₃ | 3-Cl | | A-231 | 2-CH₃ | 3-NO₂ |
| A-202 | 3-CF₃ | 3-Cl | | A-232 | 2-CF₃ | 3-NO₂ |
| A-203 | 3-OCH₃ | 3-Cl | | A-233 | 2-OCH₃ | 3-NO₂ |
| A-204 | 3-OCF₃ | 3-Cl | | A-234 | 2-OCF₃ | 3-NO₂ |
| A-205 | 2-F | 3-F | | A-235 | 3-F | 3-NO₂ |
| A-206 | 2-Cl | 3-F | | A-236 | 3-Cl | 3-NO₂ |
| A-207 | 2-CH₃ | 3-F | | A-237 | 3-CH₃ | 3-NO₂ |
| A-208 | 2-CF₃ | 3-F | | A-238 | 3-CF₃ | 3-NO₂ |
| A-209 | 2-OCH₃ | 3-F | | A-239 | 3-OCH₃ | 3-NO₂ |
| A-210 | 2-OCF₃ | 3-F | | A-240 | 3-OCF₃ | 3-NO₂ |
| A-211 | 3-F | 3-F | | A-241 | 2-F | 3-OCH₃ |
| A-212 | 3-Cl | 3-F | | A-242 | 2-Cl | 3-OCH₃ |
| A-213 | 3-CH₃ | 3-F | | A-243 | 2-CH₃ | 3-OCH₃ |
| A-214 | 3-CF₃ | 3-F | | A-244 | 2-CF₃ | 3-OCH₃ |
| A-215 | 3-OCH₃ | 3-F | | A-245 | 2-OCH₃ | 3-OCH₃ |
| A-216 | 3-OCF₃ | 3-F | | A-246 | 2-OCF₃ | 3-OCH₃ |
| A-217 | 2-F | 3-CN | | A-247 | 3-F | 3-OCH₃ |
| A-218 | 2-Cl | 3-CN | | A-248 | 3-Cl | 3-OCH₃ |
| A-219 | 2-CH₃ | 3-CN | | A-249 | 3-CH₃ | 3-OCH₃ |
| A-220 | 2-CF₃ | 3-CN | | A-250 | 3-CF₃ | 3-OCH₃ |
| A-221 | 2-OCH₃ | 3-CN | | A-251 | 3-OCH₃ | 3-OCH₃ |
| A-222 | 2-OCF₃ | 3-CN | | A-252 | 3-OCF₃ | 3-OCH₃ |
| A-223 | 3-F | 3-CN | | A-253 | 2-F | 3-CF₃ |
| A-224 | 3-Cl | 3-CN | | A-254 | 2-Cl | 3-CF₃ |
| A-225 | 3-CH₃ | 3-CN | | A-255 | 2-CH₃ | 3-CF₃ |
| A-226 | 3-CF₃ | 3-CN | | A-256 | 2-CF₃ | 3-CF₃ |
| A-257 | 2-OCH₃ | 3-CF₃ | | A-287 | 3-OCF₃ | 3-CF(CF₃)₂ |
| A-258 | 2-OCF₃ | 3-CF₃ | | A-288 | 2-Cl | 3-SCH₃ |
| A-259 | 3-F | 3-CF₃ | | A-289 | 2-CH₃ | 3-SCH₃ |
| A-260 | 3-Cl | 3-CF₃ | | A-290 | 2-CF₃ | 3-SCH₃ |
| A-261 | 3-CH₃ | 3-CF₃ | | A-291 | 2-OCH₃ | 3-SCH₃ |
| A-262 | 3-CF₃ | 3-CF₃ | | A-292 | 2-OCF₃ | 3-SCH₃ |
| A-263 | 3-OCH₃ | 3-CF₃ | | A-293 | 3-F | 3-SCH₃ |
| A-264 | 3-OCF₃ | 3-CF₃ | | A-294 | 3-Cl | 3-SCH₃ |
| A-265 | 2-Cl | 3-OCF₃ | | A-295 | 3-CH₃ | 3-SCH₃ |
| A-266 | 2-CH₃ | 3-OCF₃ | | A-296 | 3-CF₃ | 3-SCH₃ |
| A-267 | 2-CF₃ | 3-OCF₃ | | A-297 | 3-OCH₃ | 3-SCH₃ |
| A-268 | 2-OCH₃ | 3-OCF₃ | | A-298 | 3-OCF₃ | 3-SCH₃ |
| A-269 | 2-OCF₃ | 3-OCF₃ | | A-299 | 2-F | 3-C(=O)CH₃ |
| A-270 | 3-F | 3-OCF₃ | | A-300 | 2-Cl | 3-C(=O)CH₃ |
| A-271 | 3-Cl | 3-OCF₃ | | A-301 | 2-CH₃ | 3-C(=O)CH₃ |
| A-272 | 3-CH₃ | 3-OCF₃ | | A-302 | 2-CF₃ | 3-C(=O)CH₃ |
| A-273 | 3-CF₃ | 3-OCF₃ | | A-303 | 2-OCH₃ | 3-C(=O)CH₃ |
| A-274 | 3-OCH₃ | 3-OCF₃ | | A-304 | 2-OCF₃ | 3-C(=O)CH₃ |
| A-275 | 3-OCF₃ | 3-OCF₃ | | A-305 | 3-F | 3-C(=O)CH₃ |
| A-276 | 2-F | 3-CF(CF₃)₂ | | A-306 | 3-Cl | 3-C(=O)CH₃ |
| A-277 | 2-Cl | 3-CF(CF₃)₂ | | A-307 | 3-CH₃ | 3-C(=O)CH₃ |
| A-278 | 2-CH₃ | 3-CF(CF₃)₂ | | A-308 | 3-CF₃ | 3-C(=O)CH₃ |
| A-279 | 2-CF₃ | 3-CF(CF₃)₂ | | A-309 | 3-OCH₃ | 3-C(=O)CH₃ |
| A-280 | 2-OCH₃ | 3-CF(CF₃)₂ | | A-310 | 3-OCF₃ | 3-C(=O)CH₃ |
| A-281 | 2-OCF₃ | 3-CF(CF₃)₂ | | A-311 | 2-F | 3-C(=NOCH₃)CH₃ |
| A-282 | 3-F | 3-CF(CF₃)₂ | | A-312 | 2-Cl | 3-C(=NOCH₃)CH₃ |
| A-283 | 3-Cl | 3-CF(CF₃)₂ | | A-313 | 2-CH₃ | 3-C(=NOCH₃)CH₃ |
| A-284 | 3-CH₃ | 3-CF(CF₃)₂ | | A-314 | 2-CF₃ | 3-C(=NOCH₃)CH₃ |
| A-285 | 3-CF₃ | 3-CF(CF₃)₂ | | A-315 | 2-OCH₃ | 3-C(=NOCH₃)CH₃ |
| A-286 | 3-OCH₃ | 3-CF(CF₃)₂ | | A-316 | 2-OCF₃ | 3-C(=NOCH₃)CH₃ |
| A-317 | 3-F | 3-C(=NOCH₃)CH₃ | | A-347 | 2-F | 4-Br |
| A-318 | 3-Cl | 3-C(=NOCH₃)CH₃ | | A-348 | 2-Cl | 4-Br |
| A-319 | 3-CH₃ | 3-C(=NOCH₃)CH₃ | | A-349 | 2-CH₃ | 4-Br |
| A-320 | 3-CF₃ | 3-C(=NOCH₃)CH₃ | | A-350 | 2-CF₃ | 4-Br |
| A-321 | 3-OCH₃ | 3-C(=NOCH₃)CH₃ | | A-351 | 2-OCH₃ | 4-Br |
| A-322 | 3-OCF₃ | 3-C(=NOCH₃)CH₃ | | A-352 | 2-OCF₃ | 4-Br |
| A-323 | 2-F | 4-CH₃ | | A-353 | 3-F | 4-Br |
| A-324 | 2-Cl | 4-CH₃ | | A-354 | 3-Cl | 4-Br |
| A-325 | 2-CH₃ | 4-CH₃ | | A-355 | 3-CH₃ | 4-Br |
| A-326 | 2-CF₃ | 4-CH₃ | | A-356 | 3-CF₃ | 4-Br |
| A-327 | 2-OCH₃ | 4-CH₃ | | A-357 | 3-OCH₃ | 4-Br |
| A-328 | 2-OCF₃ | 4-CH₃ | | A-358 | 3-OCF₃ | 4-Br |
| A-329 | 3-F | 4-CH₃ | | A-359 | 2-F | 4-Cl |
| A-330 | 3-Cl | 4-CH₃ | | A-360 | 2-Cl | 4-Cl |
| A-331 | 3-CH₃ | 4-CH₃ | | A-361 | 2-CH₃ | 4-Cl |
| A-332 | 3-CF₃ | 4-CH₃ | | A-362 | 2-CF₃ | 4-Cl |
| A-333 | 3-OCH₃ | 4-CH₃ | | A-363 | 2-OCH₃ | 4-Cl |
| A-334 | 3-OCF₃ | 4-CH₃ | | A-364 | 2-OCF₃ | 4-Cl |
| A-335 | 2-F | 4-CH₂CH₃ | | A-365 | 3-F | 4-Cl |
| A-336 | 2-Cl | 4-CH₂CH₃ | | A-366 | 3-Cl | 4-Cl |
| A-337 | 2-CH₃ | 4-CH₂CH₃ | | A-367 | 3-CH₃ | 4-Cl |
| A-338 | 2-CF₃ | 4-CH₂CH₃ | | A-368 | 3-CF₃ | 4-Cl |
| A-339 | 2-OCH₃ | 4-CH₂CH₃ | | A-369 | 3-OCH₃ | 4-Cl |
| A-340 | 2-OCF₃ | 4-CH₂CH₃ | | A-370 | 3-OCF₃ | 4-Cl |
| A-341 | 3-F | 4-CH₂CH₃ | | A-371 | 2-F | 4-F |
| A-342 | 3-Cl | 4-CH₂CH₃ | | A-372 | 2-Cl | 4-F |
| A-343 | 3-CH₃ | 4-CH₂CH₃ | | A-373 | 2-CH₃ | 4-F |
| A-344 | 3-CF₃ | 4-CH₂CH₃ | | A-374 | 2-CF₃ | 4-F |
| A-345 | 3-OCH₃ | 4-CH₂CH₃ | | A-375 | 2-OCH₃ | 4-F |
| A-346 | 3-OCF₃ | 4-CH₂CH₃ | | A-376 | 2-OCF₃ | 4-F |
| A-377 | 3-F | 4-F | | A-407 | 2-F | 4-OCH₃ |
| A-378 | 3-Cl | 4-F | | A-408 | 2-Cl | 4-OCH₃ |
| A-379 | 3-CH₃ | 4-F | | A-409 | 2-CH₃ | 4-OCH₃ |
| A-380 | 3-CF₃ | 4-F | | A-410 | 2-CF₃ | 4-OCH₃ |
| A-381 | 3-OCH₃ | 4-F | | A-411 | 2-OCH₃ | 4-OCH₃ |
| A-382 | 3-OCF₃ | 4-F | | A-412 | 2-OCF | 4-OCH₃ |
| A-383 | 2-F | 4-CN | | A-413 | 3-F | 4-OCH₃ |
| A-384 | 2-Cl | 4-CN | | A-414 | 3-Cl | 4-OCH₃ |
| A-385 | 2-CH₃ | 4-CN | | A-415 | 3-CH₃ | 4-OCH₃ |
| A-386 | 2-CF₃ | 4-CN | | A-416 | 3-CF₃ | 4-OCH₃ |
| A-387 | 2-OCH₃ | 4-CN | | A-417 | 3-OCH₃ | 4-OCH₃ |
| A-388 | 2-OCF₃ | 4-CN | | A-418 | 3-OCF₃ | 4-OCH₃ |
| A-389 | 3-F | 4-CN | | A-419 | 2-F | 4-OCH₂CH₃ |
| A-390 | 3-Cl | 4-CN | | A-420 | 2-Cl | 4-OCH₂CH₃ |
| A-391 | 3-CH₃ | 4-CN | | A-421 | 2-CH₃ | 4-OCH₂CH₃ |
| A-392 | 3-CF₃ | 4-CN | | A-422 | 2-CF₃ | 4-OCH₂CH₃ |
| A-393 | 3-OCH₃ | 4-CN | | A-423 | 2-OCH₃ | 4-OCH₂CH₃ |
| A-394 | 3-OCF₃ | 4-CN | | A-424 | 2-OCF₃ | 4-OCH₂CH₃ |
| A-395 | 2-F | 4-NO₂ | | A-425 | 3-F | 4-OCH₂CH₃ |
| A-396 | 2-Cl | 4-NO₂ | | A-426 | 3-Cl | 4-OCH₂CH₃ |
| A-397 | 2-CH₃ | 4-NO₂ | | A-427 | 3-CH₃ | 4-OCH₂CH₃ |
| A-398 | 2-CF₃ | 4-NO₂ | | A-428 | 3-CF₃ | 4-OCH₂CH₃ |
| A-399 | 2-OCH₃ | 4-NO₂ | | A-429 | 3-OCH₃ | 4-OCH₂CH₃ |
| A-400 | 2-OCF₃ | 4-NO₂ | | A-430 | 3-OCF₃ | 4-OCH₂CH₃ |
| A-401 | 3-F | 4-NO₂ | | A-431 | 2-F | 4-OCH(CH₃)₂ |
| A-402 | 3-Cl | 4-NO₂ | | A-432 | 2-Cl | 4-OCH(CH₃)₂ |
| A-403 | 3-CH₃ | 4-NO₂ | | A-433 | 2-CH₃ | 4-OCH(CH₃)₂ |
| A-404 | 3-CF3 | 4-NO₂ | | A-434 | 2-CF₃ | 4-OCH(CH₃)₂ |
| A-405 | 3-OCH3 | 4-NO₂ | | A-435 | 2-OCH₃ | 4-OCH(CH₃)₂ |
| A-406 | 3-OCF3 | 4-NO₂ | | A-436 | 2-OCF₃ | 4-OCH(CH₃)₂ |
| A-437 | 3-F | 4-OCH(CH₃)₂ | | A-467 | 2-F | 4-OCF₃ |
| A-438 | 3-Cl | 4-OCH(CH₃)₂ | | A-468 | 2-Cl | 4-OCF₃ |
| A-439 | 3-CH₃ | 4-OCH(CH₃)₂ | | A-469 | 2-CH₃ | 4-OCF₃ |
| A-440 | 3-CF₃ | 4-OCH(CH₃)₂ | | A-470 | 2-CF₃ | 4-OCF₃ |
| A-441 | 3-OCH₃ | 4-OCH(CH₃)₂ | | A-471 | 2-OCH₃ | 4-OCF₃ |
| A-442 | 3-OCF₃ | 4-OCH(CH₃)₂ | | A-472 | 2-OCF₃ | 4-OCF₃ |
| A-443 | 2-F | 4-OC(CH₃)₃ | | A-473 | 3-F | 4-OCF₃ |
| A-444 | 2-CI | 4-OC(CH₃)₃ | | A-474 | 3-Cl | 4-OCF₃ |
| A-445 | 2-CH₃ | 4-OC(CH₃)₃ | | A-475 | 3-CH₃ | 4-OCF₃ |
| A-446 | 2-CF₃ | 4-OC(CH₃)₃ | | A-476 | 3-CF₃ | 4-OCF₃ |
| A-447 | 2-OCH₃ | 4-OC(CH₃)₃ | | A-477 | 3-OCH₃ | 4-OCF₃ |
| A-448 | 2-OCF₃ | 4-OC(CHs)s | | A-478 | 3-OCF₃ | 4-OCF₃ |
| A-449 | 3-F | 4-OC(CH₃)₃ | | A-479 | 2-F | 4-SCH₃ |
| A-450 | 3-Cl | 4-OC(CH₃)₃ | | A-480 | 2-Cl | 4-SCH₃ |
| A-451 | 3-CH₃ | 4-OC(CH₃)₃ | | A-481 | 2-CH₃ | 4-SCH₃ |
| A-452 | 3-CF₃ | 4-OC(CH₃)₃ | | A-482 | 2-CF₃ | 4-SCH₃ |
| A-453 | 3-OCH₃ | 4-OC(CH₃)₃ | | A-483 | 2-OCH₃ | 4-SCH₃ |
| A-454 | 3-OCF₃ | 4-OC(CH₃)₃ | | A-484 | 2-OCF₃ | 4-SCH₃ |
| A-455 | 2-F | 4-CF₃ | | A-485 | 3-F | 4-SCH₃ |
| A-456 | 2-Cl | 4-CF₃ | | A-486 | 3-Cl | 4-SCH₃ |
| A-457 | 2-CH₃ | 4-CF₃ | | A-487 | 3-CH₃ | 4-SCH₃ |
| A-458 | 2-CF₃ | 4-CF₃ | | A-488 | 3-CF₃ | 4-SCH₃ |
| A-459 | 2-OCH₃ | 4-CF₃ | | A-489 | 3-OCH₃ | 4-SCH₃ |
| A-460 | 2-OCF₃ | 4-CF₃ | | A-490 | 3-OCF₃ | 4-SCH₃ |
| A-461 | 3-F | 4-CF₃ | | A-491 | 2-F | 4-C(=O)CH₃ |
| A-462 | 3-Cl | 4-CF₃ | | A-492 | 2-Cl | 4-C(=O)CH₃ |
| A-463 | 3-CH₃ | 4-CF₃ | | A-493 | 2-CH₃ | 4-C(=O)CH₃ |
| A-464 | 3-CF₃ | 4-CF₃ | | A-494 | 2-CF₃ | 4-C(=O)CH₃ |
| A-465 | 3-OCH₃ | 4-CF₃ | | A-495 | 2-OCH₃ | 4-C(=O)CH₃ |
| A-466 | 3-OCF₃ | 4-CF₃ | | A-496 | 2-OCF₃ | 4-C(=O)CH₃ |
| A-497 | 3-F | 4-C(=O)CH₃ | | A-527 | 2-F | 2,4-(CH₃)₂ |
| A-498 | 3-Cl | 4-C(=O)CH₃ | | A-528 | 2-Cl | 2,4-(CH₃)₂ |
| A-499 | 3-CH₃ | 4-C(=O)CH₃ | | A-529 | 2-CH₃ | 2,4-(CH₃)₂ |
| A-500 | 3-CF₃ | 4-C(=O)CH₃ | | A-530 | 2-CF₃ | 2,4-(CH₃)₂ |
| A-501 | 3-OCH₃ | 4-C(=O)CH₃ | | A-531 | 2-OCH₃ | 2,4-(CH₃)₂ |
| A-502 | 3-OCF₃ | 4-C(=O)CH₃ | | A-532 | 2-OCF₃ | 2,4-(CH₃)₂ |
| A-503 | 2-F | 4-C(=NOCH₃)CH₃ | | A-533 | 3-F | 2,4-(CH₃)₂ |
| A-504 | 2-Cl | 4-C(=NOCH₃)CH₃ | | A-534 | 3-Cl | 2,4-(CH₃)₂ |
| A-505 | 2-CH₃ | 4-C(=NOCH₃)CH₃ | | A-535 | 3-CH₃ | 2,4-(CH₃)₂ |
| A-506 | 2-CF₃ | 4-C(=NOCH₃)CH₃ | | A-536 | 3-CF₃ | 2,4-(CH₃)₂ |
| A-507 | 2-OCH₃ | 4-C(=NOCH₃)CH₃ | | A-537 | 3-OCH₃ | 2,4-(CH₃)₂ |
| A-508 | 2-OCF₃ | 4-C(=NOCH₃)CH₃ | | A-538 | 3-OCF₃ | 2,4-(CH₃)₂ |
| A-509 | 3-F | 4-C(=NOCH₃)CH₃ | | A-539 | 2-F | 2,4-(CH₂CH₃)₂ |
| A-51 0 | 3-Cl | 4-C(=NOCH₃)CH₃ | | A-540 | 2-Cl | 2,4-(CH₂CH₃)₂ |
| A-511 | 3-CH₃ | 4-C(=NOCH₃)CH₃ | | A-541 | 2-CH₃ | 2,4-(CH₂CH₃)₂ |
| A-512 | 3-CF₃ | 4-C(=NOCH₃)CH₃ | | A-542 | 2-CF₃ | 2,4-(CH₂CH₃)₂ |
| A-513 | 3-OCH₃ | 4-C(=NOCH₃)CH₃ | | A-543 | 2-OCH₃ | 2,4-(CH₂CH₃)₂ |
| A-514 | 3-OCF₃ | 4-C(=NOCH₃)CH₃ | | A-544 | 2-OCF₃ | 2,4-(CH₂CH₃)₂ |
| A-515 | 2-F | 4-CF(CF₃)₂ | | A-545 | 3-F | 2,4-(CH₂CH₃)₂ |
| A-516 | 2-Cl | 4-CF(CF₃)₂ | | A-546 | 3-Cl | 2,4-(CH₂CH₃)₂ |
| A-517 | 2-CH₃ | 4-CF(CF₃)₂ | | A-547 | 3-CH₃ | 2,4-(CH₂CH₃)₂ |
| A-518 | 2-CF₃ | 4-CF(CF₃)₂ | | A-548 | 3-CF₃ | 2,4-(CH₂CH₃)₂ |
| A-519 | 2-OCH₃ | 4-CF(CF₃)₂ | | A-549 | 3-OCH₃ | 2,4-(CH₂CH₃)₂ |
| A-520 | 2-OCF₃ | 4-CF(CF₃)₂ | | A-550 | 3-OCF₃ | 2,4-(CH₂CH₃)₂ |
| A-521 | 3-F | 4-CF(CF₃)₂ | | A-551 | 2-F | 2,4-Br₂ |
| A-522 | 3-Cl | 4-CF(CF₃)₂ | | A-552 | 2-Cl | 2,4-Br₂ |
| A-523 | 3-CH₃ | 4-CF(CF₃)₂ | | A-553 | 2-CH₃ | 2,4-Br₂ |
| A-524 | 3-CF₃ | 4-CF(CF₃)₂ | | A-554 | 2-CF₃ | 2,4-Br₂ |
| A-525 | 3-OCH₃ | 4-CF(CF₃)₂ | | A-555 | 2-OCH₃ | 2,4-Br₂ |
| A-526 | 3-OCF₃ | 4-CF(CF₃)₂ | | A-556 | 2-OCF₃ | 2,4-Br₂ |
| A-557 | 3-F | 2,4-Br₂ | | A-587 | 2-F | 2,4-(OCH₃)₂ |
| A-558 | 3-Cl | 2,4-Br₂ | | A-588 | 2-Cl | 2,4-(OCH₃)₂ |
| A-559 | 3-CH₃ | 2,4-Br₂ | | A-589 | 2-CH₃ | 2,4-(OCH₃)₂ |
| A-560 | 3-CF₃ | 2,4-Br₂ | | A-590 | 2-CF₃ | 2,4-(OCH₃)₂ |
| A-561 | 3-OCH₃ | 2,4-Br₂ | | A-591 | 2-OCH₃ | 2,4-(OCH₃)₂ |
| A-562 | 3-OCF₃ | 2,4-Br₂ | | A-592 | 2-OCF₃ | 2,4-(OCH₃)₂ |
| A-563 | 2-F | 2,4-Cl₂ | | A-593 | 3-F | 2,4-(OCH₃)₂ |
| A-564 | 2-Cl | 2,4-Cl₂ | | A-594 | 3-Cl | 2,4-(OCH₃)₂ |
| A-565 | 2-CH₃ | 2,4-Cl₂ | | A-595 | 3-CH₃ | 2,4-(OCH₃)₂ |
| A-566 | 2-CF₃ | 2,4-Cl₂ | | A-596 | 3-CF₃ | 2,4-(OCH₃)₂ |
| A-567 | 2-OCH₃ | 2,4-Cl₂ | | A-597 | 3-OCH₃ | 2,4-(OCH₃)₂ |
| A-568 | 2-OCF₃ | 2,4-Cl₂ | | A-598 | 3-OCF₃ | 2,4-(OCH₃)₂ |
| A-569 | 3-F | 2,4-Cl₂ | | A-599 | 2-F | 2,4-(CF₃)₂ |
| A-570 | 3-Cl | 2,4-Cl₂ | | A-600 | 2-Cl | 2,4-(CF₃)₂ |
| A-571 | 3-CH₃ | 2,4-Cl₂ | | A-601 | 2-CH₃ | 2,4-(CF₃)₂ |
| A-572 | 3-CF₃ | 2,4-Cl₂ | | A-602 | 2-CF₃ | 2,4-(CF₃)₂ |
| A-573 | 3-OCH₃ | 2,4-Cl₂ | | A-603 | 2-OCH₃ | 2,4-(CF₃)₂ |
| A-574 | 3-OCF₃ | 2,4-Cl₂ | | A-604 | 2-OCF₃ | 2,4-(CF₃)₂ |
| A-575 | 2-F | 2,4-F₂ | | A-605 | 3-F | 2,4-(CF₃)₂ |
| A-576 | 2-Cl | 2,4-F₂ | | A-606 | 3-Cl | 2,4-(CF₃)₂ |
| A-577 | 2-CH₃ | 2,4-F₂ | | A-607 | 3-CH₃ | 2,4-(CF₃)₂ |
| A-578 | 2-CF₃ | 2,4-F₂ | | A-608 | 3-CF₃ | 2,4-(CF₃)₂ |
| A-579 | 2-OCH₃ | 2,4-F₂ | | A-609 | 3-OCH₃ | 2,4-(CF₃)₂ |
| A-580 | 2-OCF₃ | 2,4-F₂ | | A-610 | 3-OCF₃ | 2,4-(CF₃)₂ |
| A-581 | 3-F | 2,4-F₂ | | A-611 | 2-F | 2,4-(OCF₃)₂ |
| A-582 | 3-Cl | 2,4-F₂ | | A-612 | 2-Cl | 2,4-(OCF₃)₂ |
| A-583 | 3-CH₃ | 2,4-F₂ | | A-613 | 2-CH₃ | 2,4-(OCF₃)₂ |
| A-584 | 3-CF₃ | 2,4-F₂ | | A-614 | 2-CF₃ | 2,4-(OCF₃)₂ |
| A-585 | 3-OCH₃ | 2,4-F₂ | | A-615 | 2-OCH₃ | 2,4-(OCF₃)₂ |
| A-586 | 3-OCF₃ | 2,4-F₂ | | A-616 | 2-OCF₃ | 2,4-(OCF₃)₂ |
| A-617 | 3-F | 2,4-(OCF₃)₂ | | A-647 | 2-F | 2,5-F₂ |
| A-618 | 3-Cl | 2,4-(OCF₃)₂ | | A-648 | 2-Cl | 2,5-F₂ |
| A-619 | 3-CH₃ | 2,4-(OCF₃)₂ | | A-649 | 2-CH₃ | 2,5-F₂ |
| A-620 | 3-CF₃ | 2,4-(OCF₃)₂ | | A-650 | 2-CF₃ | 2,5-F₂ |
| A-621 | 3-OCH₃ | 2,4-(OCF₃)₂ | | A-651 | 2-OCH₃ | 2,5-F₂ |
| A-622 | 3-OCF₃ | 2,4-(OCF₃)₂ | | A-652 | 2-OCF₃ | 2,5-F₂ |
| A-623 | 2-F | 2,5-(CH₃)₂ | | A-653 | 3-F | 2,5-F₂ |
| A-624 | 2-Cl | 2,5-(CH₃)₂ | | A-654 | 3-Cl | 2,5-F₂ |
| A-625 | 2-CH₃ | 2,5-(CH₃)₂ | | A-655 | 3-CH₃ | 2,5-F₂ |
| A-626 | 2-CF₃ | 2,5-(CH₃)₂ | | A-656 | 3-CF₃ | 2,5-F₂ |
| A-627 | 2-OCH₃ | 2,5-(CH₃)₂ | | A-657 | 3-OCH₃ | 2,5-F₂ |
| A-628 | 2-OCF₃ | 2,5-(CH₃)₂ | | A-658 | 3-OCF₃ | 2,5-F₂ |
| A-629 | 3-F | 2,5-(CH₃)₂ | | A-659 | 2-F | 2,5-(OCH₃)₂ |
| A-630 | 3-Cl | 2,5-(CH₃)₂ | | A-660 | 2-Cl | 2,5-(OCH₃)₂ |
| A-631 | 3-CH₃ | 2,5-(CH₃)₂ | | A-661 | 2-CH₃ | 2,5-(OCH₃)₂ |
| A-632 | 3-CF₃ | 2,5-(CH₃)₂ | | A-662 | 2-CF₃ | 2,5-(OCH₃)₂ |
| A-633 | 3-OCH₃ | 2,5-(CH₃)₂ | | A-663 | 2-OCH₃ | 2,5-(OCH₃)₂ |
| A-634 | 3-OCF₃ | 2,5-(CH₃)₂ | | A-664 | 2-OCF₃ | 2,5-(OCH₃)₂ |
| A-635 | 2-F | 2,5-Cl₂ | | A-665 | 3-F | 2,5-(OCH₃)₂ |
| A-636 | 2-Cl | 2,5-Cl₂ | | A-666 | 3-Cl | 2,5-(OCH₃)₂ |
| A-637 | 2-CH₃ | 2,5-Cl₂ | | A-667 | 3-CH₃ | 2,5-(OCH₃)₂ |
| A-638 | 2-CF₃ | 2,5-Cl₂ | | A-668 | 3-CF₃ | 2,5-(OCH₃)₂ |
| A-639 | 2-OCH₃ | 2,5-Cl₂ | | A-669 | 3-OCH₃ | 2,5-(OCH₃)₂ |
| A-640 | 2-OCF₃ | 2,5-Cl₂ | | A-670 | 3-OCF₃ | 2,5-(OCH₃)₂ |
| A-641 | 3-F | 2,5-Cl₂ | | A-671 | 2-F | 2,5-(CF₃)₂ |
| A-642 | 3-Cl | 2,5-Cl₂ | | A-672 | 2-Cl | 2,5-(CF₃)₂ |
| A-643 | 3-CH₃ | 2,5-Cl₂ | | A-673 | 2-CH₃ | 2,5-(CF₃)₂ |
| A-644 | 3-CF₃ | 2,5-Cl₂ | | A-674 | 2-CF₃ | 2,5-(CF₃)₂ |
| A-645 | 3-OCH₃ | 2,5-Cl₂ | | A-675 | 2-OCH₃ | 2,5-(CF₃)₂ |
| A-646 | 3-OCF₃ | 2,5-Cl₂ | | A-676 | 2-OCF₃ | 2,5-(CF₃)₂ |
| A-677 | 3-F | 2,5-(CF₃)₂ | | A-707 | 2-F | 2,6-(CH₃)₂ |
| A-678 | 3-Cl | 2,5-(CF₃)₂ | | A-708 | 2-Cl | 2,6-(CH₃)₂ |
| A-679 | 3-CH₃ | 2,5-(CF₃)₂ | | A-709 | 2-CH₃ | 2,6-(CH₃)₂ |
| A-680 | 3-CF₃ | 2,5-(CF₃)₂ | | A-710 | 2-CF₃ | 2,6-(CH₃)₂ |
| A-681 | 3-OCH₃ | 2,5-(CF₃)₂ | | A-711 | 2-OCH₃ | 2,6-(CH₃)₂ |
| A-682 | 3-OCF₃ | 2,5-(CF₃)₂ | | A-712 | 2-OCF₃ | 2,6-(CH₃)₂ |
| A-683 | 2-F | 2,5-(OCF₃)₂ | | A-713 | 3-F | 2,6-(CH₃)₂ |
| A-684 | 2-Cl | 2,5-(OCF₃)₂ | | A-714 | 3-Cl | 2,6-(CH₃)₂ |
| A-685 | 2-CH₃ | 2,5-(OCF₃)₂ | | A-715 | 3-CH₃ | 2,6-(CH₃)₂ |
| A-686 | 2-CF₃ | 2,5-(OCF₃)₂ | | A-716 | 3-CF₃ | 2,6-(CH₃)₂ |
| A-687 | 2-OCH₃ | 2,5-(OCF₃)₂ | | A-717 | 3-OCH₃ | 2,6-(CH₃)₂ |
| A-688 | 2-OCF₃ | 2,5-(OCF₃)₂ | | A-718 | 3-OCF₃ | 2,6-(CH₃)₂ |
| A-689 | 3-F | 2,5-(OCF₃)₂ | | A-719 | 2-F | 3,5-(CH₃)₂ |
| A-690 | 3-Cl | 2,5-(OCF₃)₂ | | A-720 | 2-Cl | 3,5-(CH₃)₂ |
| A-691 | 3-CH₃ | 2,5-(OCF₃)₂ | | A-721 | 2-CH₃ | 3,5-(CH₃)₂ |
| A-692 | 3-CF₃ | 2,5-(OCF₃)₂ | | A-722 | 2-CF₃ | 3,5-(CH₃)₂ |
| A-693 | 3-OCH₃ | 2,5-(OCF₃)₂ | | A-723 | 2-OCH₃ | 3,5-(CH₃)₂ |
| A-694 | 3-OCF₃ | 2,5-(OCF₃)₂ | | A-724 | 2-OCF₃ | 3,5-(CH₃)₂ |
| A-695 | 2-F | 2,5-(SCH₃)₂ | | A-725 | 3-F | 3,5-(CH₃)₂ |
| A-696 | 2-Cl | 2,5-(SCH₃)₂ | | A-726 | 3-Cl | 3,5-(CH₃)₂ |
| A-697 | 2-CH₃ | 2,5-(SCH₃)₂ | | A-727 | 3-CH₃ | 3,5-(CH₃)₂ |
| A-698 | 2-CF₃ | 2,5-(SCH₃)₂ | | A-728 | 3-CF₃ | 3,5-(CH₃)₂ |
| A-699 | 2-OCH₃ | 2,5-(SCH₃)₂ | | A-729 | 3-OCH₃ | 3,5-(CH₃)₂ |
| A-700 | 2-OCF₃ | 2,5-(SCH₃)₂ | | A-730 | 3-OCF₃ | 3,5-(CH₃)₂ |
| A-701 | 3-F | 2,5-(SCH₃)₂ | | A-731 | 2-F | 3,5-(CF₃)₂ |
| A-702 | 3-Cl | 2,5-(SCH₃)₂ | | A-732 | 2-Cl | 3,5-(CF₃)₂ |
| A-703 | 3-CH₃ | 2,5-(SCH₃)₂ | | A-733 | 2-CH₃ | 3,5-(CF₃)₂ |
| A-704 | 3-CF₃ | 2,5-(SCH₃)₂ | | A-734 | 2-CF₃ | 3,5-(CF₃)₂ |
| A-705 | 3-OCH₃ | 2,5-(SCH₃)₂ | | A-735 | 2-OCH₃ | 3,5-(CF₃)₂ |
| A-706 | 3-OCF₃ | 2,5-(SCH₃)₂ | | A-736 | 2-OCF₃ | 3,5-(CF₃)₂ |
| A-737 | 3-F | 3,5-(CF₃)₂ | | A-767 | 2-F | 3-CH₃, 4-F |
| A-738 | 3-Cl | 3,5-(CF₃)₂ | | A-768 | 2-Cl | 3-CH₃, 4-F |
| A-739 | 3-CH₃ | 3,5-(CF₃)₂ | | A-769 | 2-CH₃ | 3-CH₃, 4-F |
| A-740 | 3-CF₃ | 3,5-(CF₃)₂ | | A-770 | 2-CF₃ | 3-CH₃, 4-F |
| A-741 | 3-OCH₃ | 3,5-(CF₃)₂ | | A-771 | 2-OCH₃ | 3-CH₃, 4-F |
| A-742 | 3-OCF₃ | 3,5-(CF₃)₂ | | A-772 | 2-OCF₃ | 3-CH₃, 4-F |
| A-743 | 2-F | 3,5-(OCF₃)₂ | | A-773 | 3-F | 3-CH₃, 4-F |
| A-744 | 2-Cl | 3,5-(OCF₃)₂ | | A-774 | 3-Cl | 3-CH₃, 4-F |
| A-745 | 2-CH₃ | 3,5-(OCF₃)₂ | | A-775 | 3-CH₃ | 3-CH₃, 4-F |
| A-746 | 2-CF₃ | 3,5-(OCF₃)₂ | | A-776 | 3-CF₃ | 3-CH₃, 4-F |
| A-747 | 2-OCH₃ | 3,5-(OCF₃)₂ | | A-777 | 3-OCH₃ | 3-CH₃, 4-F |
| A-748 | 2-OCF₃ | 3,5-(OCF₃)₂ | | A-778 | 3-OCF₃ | 3-CH₃, 4-F |
| A-749 | 3-F | 3,5-(OCF₃)₂ | | A-779 | 2-F | 2-Cl, 4-F |
| A-750 | 3-Cl | ..3,5-(OCF₃)₂ | | A-780 | 2-Cl | 2-Cl, 4-F |
| A-751 | 3-CH₃ | 3,5-(OCF₃)₂ | | A-781 | 2-CH₃ | 2-Cl, 4-F |
| A-752 | 3-CF₃ | 3,5-(OCF₃)₂ | | A-782 | 2-CF₃ | 2-Cl, 4-F |
| A-753 | 3-OCH₃ | 3,5-(OCF₃)₂ | | A-783 | 2-OCH₃ | 2-Cl, 4-F |
| A-754 | 3-OCF₃ | 3,5-(OCF₃)₂ | | A-784 | 2-OCF₃ | 2-Cl, 4-F |
| A-755 | 2-F | 3-Cl, 4-F | | A-785 | 3-F | 2-Cl, 4-F |
| A-756 | 2-Cl | 3-Cl, 4-F | | A-786 | 3-Cl | 2-Cl, 4-F |
| A-757 | 2-CH₃ | 3-Cl, 4-F | | A-787 | 3-CH₃ | 2-Cl, 4-F |
| A-758 | 2-CF₃ | 3-Cl, 4-F | | A-788 | 3-CF₃ | 2-Cl, 4-F |
| A-759 | 2-OCH₃ | 3-Cl, 4-F | | A-789 | 3-OCH₃ | 2-Cl, 4-F |
| A-760 | 2-OCF₃ | 3-Cl, 4-F | | A-790 | 3-OCF₃ | 2-Cl, 4-F |
| A-761 | 3-F | 3-Cl, 4-F | | A-791 | 2-F | 2-Cl, 4-OCH₃ |
| A-762 | 3-Cl | 3-Cl, 4-F | | A-792 | 2-Cl | 2-CI, 4-OCH₃ |
| A-763 | 3-CH₃ | 3-Cl, 4-F | | A-793 | 2-CH₃ | 2-Cl, 4-OCH₃ |
| A-764 | 3-CF₃ | 3-Cl, 4-F | | A-794 | 2-CF₃ | 2-Cl, 4-OCH₃ |
| A-765 | 3-OCH₃ | 3-Cl, 4-F | | A-795 | 2-OCH₃ | 2-Cl, 4-OCH₃ |
| A-766 | 3-OCF₃ | 3-Cl, 4-F | | A-796 | 2-OCF₃ | 2-Cl, 4-OCH₃ |
| A-797 | 3-F | 2-Cl, 4-OCH₃ | | A-827 | 2-F | 2-F, 4-Br |
| A-798 | 3-Cl | 2-Cl, 4-OCH₃ | | A-828 | 2-Cl | 2-F, 4-Br |
| A-799 | 3-CH₃ | 2-Cl, 4-OCH₃ | | A-829 | 2-CH₃ | 2-F, 4-Br |
| A-800 | 3-CF₃ | 2-Cl, 4-OCH₃ | | A-830 | 2-CF₃ | 2-F, 4-Br |
| A-801 | 3-OCH₃ | 2-Cl, 4-OCH₃ | | A-831 | 2-OCH₃ | 2-F, 4-Br |
| A-802 | 3-OCF₃ | 2-Cl, 4-OCH₃ | | A-832 | 2-OCF₃ | 2-F, 4-Br |
| A-803 | 2-F | 2-Cl, 4-CF(CF₃)₂ | | A-833 | 3-F | 2-F, 4-Br |
| A-804 | 2-Cl | 2-Cl, 4-CF(CF₃)₂ | | A-834 | 3-Cl | 2-F, 4-Br |
| A-805 | 2-CH₃ | 2-Cl, 4-CF(CF₃)₂ | | A-835 | 3-CH₃ | 2-F, 4-Br |
| A-806 | 2-CF₃ | 2-Cl, 4-CF(CF₃)₂ | | A-836 | 3-CF₃ | 2-F, 4-Br |
| A-807 | 2-OCH₃ | 2-Cl, 4-CF(CF₃)₂ | | A-837 | 3-OCH₃ | 2-F, 4-Br |
| A-808 | 2-OCF₃ | 2-Cl, 4-CF(CF₃)₂ | | A-838 | 3-OCF₃ | 2-F, 4-Br |
| A-809 | 3-F | 2-Cl, 4-CF(CF₃)₂ | | A-839 | 2-F | 2-F, 4-CH₃ |
| A-81 0 | 3-Cl | 2-Cl, 4-CF(CF₃)₂ | | A-840 | 2-Cl | 2-F, 4-CH₃ |
| A-811 | 3-CH₃ | 2-Cl, 4-CF(CF₃)₂ | | A-841 | 2-CH₃ | 2-F, 4-CH₃ |
| A-812 | 3-CF₃ | 2-Cl, 4-CF(CF₃)₂ | | A-842 | 2-CF₃ | 2-F, 4-CH₃ |
| A-813 | 3-OCH₃ | 2-Cl, 4-CF(CF₃)₂ | | A-843 | 2-OCH₃ | 2-F, 4-CH₃ |
| A-814 | 3-OCF₃ | 2-Cl, 4-CF(CF₃)₂ | | A-844 | 2-OCF₃ | 2-F, 4-CH₃ |
| A-815 | 2-F | 2-F, 4-Cl | | A-845 | 3-F | 2-F, 4-CH₃ |
| A-816 | 2-Cl | 2-F, 4-Cl | | A-846 | 3-Cl | 2-F, 4-CH₃ |
| A-817 | 2-CH₃ | 2-F, 4-Cl | | A-847 | 3-CH₃ | 2-F, 4-CH₃ |
| A-818 | 2-CF₃ | 2-F, 4-Cl | | A-848 | 3-CF₃ | 2-F, 4-CH₃ |
| A-819 | 2-OCH₃ | 2-F, 4-Cl | | A-849 | 3-OCH₃ | 2-F, 4-CH₃ |
| A-820 | 2-OCF₃ | 2-F, 4-Cl | | A-850 | 3-OCF₃ | 2-F, 4-CH₃ |
| A-821 | 3-F | 2-F, 4-Cl | | A-851 | 2-F | 2-F, 4-CF₃ |
| A-822 | 3-Cl | 2-F, 4-Cl | | A-852 | 2-Cl | 2-F, 4-CF₃ |
| A-823 | 3-CH₃ | 2-F, 4-Cl | | A-853 | 2-CH₃ | 2-F, 4-CF₃ |
| A-824 | 3-CF₃ | 2-F, 4-Cl | | A-854 | 2-CF₃ | 2-F, 4-CF₃ |
| A-825 | 3-OCH₃ | 2-F, 4-Cl | | A-855 | 2-OCH₃ | 2-F, 4-CF₃ |
| A-826 | 3-OCF₃ | 2-F, 4-Cl | | A-856 | 2-OCF₃ | 2-F, 4-CF₃ |
| A-857 | 3-F | 2-F, 4-CF₃ | | A-887 | 2-Cl | 2-CH₃, 4-OCH₃ |
| A-858 | 3-Cl | 2-F, 4-CF₃ | | A-888 | 2-CH₃ | 2-CH₃, 4-OCH₃ |
| A-859 | 3-CH₃ | 2-F, 4-CF₃ | | A-889 | 2-CF₃ | 2-CH₃, 4-OCH₃ |
| A-860 | 3-CF₃ | 2-F, 4-CF₃ | | A-890 | 2-OCH₃ | 2-CH₃, 4-OCH₃ |
| A-861 | 3-OCH₃ | 2-F, 4-CF₃ | | A-891 | 2-OCF₃ | 2-CH₃, 4-OCH₃ |
| A-862 | 3-OCF₃ | 2-F, 4-CF₃ | | A-892 | 3-F | 2-CH₃, 4-OCH₃ |
| A-863 | 2-F | 2-F, 4-OCH₃ | | A-893 | 3-Cl | 2-CH₃, 4-OCH₃ |
| A-864 | 2-Cl | 2-F, 4-OCH₃ | | A-894 | 3-CH₃ | 2-CH₃, 4-OCH₃ |
| A-865 | 2-CH₃ | 2-F, 4-OCH₃ | | A-895 | 3-CF₃ | 2-CH₃, 4-OCH₃ |
| A-866 | 2₋CF₃ | 2-F, 4-OCH₃ | | A-896 | 3-OCH₃ | 2-CH₃, 4-OCH₃ |
| A-867 | 2-OCH₃ | 2-F, 4-OCH₃ | | A-897 | 3-OCF₃ | 2-CH₃, 4-OCH₃ |
| A-868 | 2-OCF₃ | 2-F, 4-OCH₃ | | A-898 | 2-F | 2-CH₃, 4-Cl |
| A-869 | 3-F | 2-F, 4-OCH₃ | | A-899 | 2-Cl | 2-CH₃, 4-Cl |
| A-870 | 3-Cl | 2-F, 4-OCH₃ | | A-900 | 2-CH₃ | 2-CH₃, 4-Cl |
| A-871 | 3-CH₃ | 2-F, 4-OCH₃ | | A-901 | 2-CF₃ | 2-CH₃, 4-Cl |
| A-872 | 3-CF₃ | 2-F, 4-OCH₃ | | A-902 | 2-OCH₃ | 2-CH₃, 4-Cl |
| A-873 | 3-OCH₃ | 2-F, 4-OCH₃ | | A-903 | 2-OCF₃ | 2-CH₃, 4-Cl |
| A-874 | 3-OCF₃ | 2-F, 4-OCH₃ | | A-904 | 3-F | 2-CH₃, 4-Cl |
| A-875 | 2-Cl | 2-F,4-CF(CF₃)₂ | | A-905 | 3-Cl | 2-CH₃, 4-Cl |
| A-876 | 2-CH₃ | 2-F,4-CF(CF₃)₂ | | A-906 | 3-CH₃ | 2-CH₃, 4-Cl |
| A-877 | 2-CF₃ | 2-F, 4-CF(CF₃)₂ | | A-907 | 3-CF₃ | 2-CH₃, 4-Cl |
| A-878 | 2-OCH₃ | 2-F,4-CF(CF₃)₂ | | A-908 | 3-OCH₃ | 2-CH₃, 4-Cl |
| A-879 | 2-OCF₃ | 2-F,4-CF(CF₃)₂ | | A-909 | 3-OCF₃ | 2-CH₃, 4-Cl |
| A-880 | 3-F | 2-F, 4-CF(CF₃)₂ | | A-910 | 2-F | 2-CH₃, 4-F |
| A-881 | 3-Cl | 2-F,4-CF(CF₃)₂ | | A-911 | 2-Cl | 2-CH₃, 4-F |
| A-882 | 3-CH₃ | 2-F, 4-CF(CF₃)₂ | | A-912 | 2-CH₃ | 2-CH₃, 4-F |
| A-883 | 3-CF₃ | 2-F,4-CF(CF₃)₂ | | A-913 | 2-CF₃ | 2-CH₃, 4-F |
| A-884 | 3-OCH₃ | 2-F, 4-CF(CF₃)₂ | | A-914 | 2-OCH₃ | 2-CH₃, 4-F |
| A-885 | 3-OCF₃ | 2-F,4-CF(CF₃)₂ | | A-915 | 2-OCF₃ | 2-CH₃, 4-F |
| A-886 | 2-F | 2-CH₃, 4-OCH₃ | | A-916 | 3-F | 2-CH₃, 4-F |
| A-917 | 3-Cl | 2-CH₃, 4-F | | A-947 | 2-Cl | 2-OCH₃, 4-F |
| A-918 | 3-CH₃ | 2-CH₃, 4-F | | A-948 | 2-CH₃ | 2-OCH₃, 4-F |
| A-919 | 3-CF₃ | 2-CH₃, 4-F | | A-949 | 2-CF₃ | 2-OCH₃, 4-F |
| A-920 | 3-OCH₃ | 2-CH₃, 4-F | | A-950 | 2-OCH₃ | 2-OCH₃, 4-F |
| A-921 | 3-OCF₃ | 2-CH₃, 4-F | | A-951 | 2-OCF₃ | 2-OCH₃, 4-F |
| A-922 | 2-F | 2-CH₃, 4-CF₃ | | A-952 | 3-F | 2-OCH₃, 4-F |
| A-923 | 2-Cl | 2-CH₃, 4-CF₃ | | A-953 | 3-Cl | 2-OCH₃, 4-F |
| A-924 | 2-CH₃ | 2-CH₃, 4-CF₃ | | A-954 | 3-CH₃ | 2-OCH₃, 4-F |
| A-925 | 2-CF₃ | 2-CH₃, 4-CF₃ | | A-955 | 3-CF₃ | 2-OCH₃, 4-F |
| A-926 | 2-OCH₃ | 2-CH₃, 4-CF₃ | | A-956 | 3-OCH₃ | 2-OCH₃, 4-F |
| A-927 | 2-OCF₃ | 2-CH₃, 4-CF₃ | | A-957 | 3-OCF₃ | 2-OCH₃, 4-F |
| A-928 | 3-F | 2-CH₃, 4-CF₃ | | A-958 | 2-F | 2-OCH₃, 4-Cl |
| A-929 | 3-Cl | 2-CH₃, 4-CF₃ | | A-959 | 2-Cl | 2-OCH₃, 4-Cl |
| A-930 | 3-CH₃ | 2-CH₃, 4-CF₃ | | A-960 | 2-CH₃ | 2-OCH₃, 4-Cl |
| A-931 | 3-CF₃ | 2-CH₃, 4-CF₃ | | A-961 | 2-CF₃ | 2-OCH₃, 4-Cl |
| A-932 | 3-OCH₃ | 2-CH₃, 4-CF₃ | | A-962 | 2-OCH₃ | 2-OCH₃, 4-Cl |
| A-933 | 3-OCF₃ | 2-CH₃, 4-CF₃ | | A-963 | 2-OCF₃ | 2-OCH₃, 4-Cl |
| A-934 | 2-F | 2-CH₃, 4-CF(CF₃)₂ | | A-964 | 3-F | 2-OCH₃, 4-Cl |
| A-935 | 2-Cl | 2-CH₃, 4-CF(CF₃)₂ | | A-965 | 3-Cl | 2-OCH₃, 4-Cl |
| A-936 | 2-CH₃ | 2-CH₃, 4-CF(CF₃)₂ | | A-966 | 3-CH₃ | 2-OCH₃, 4-Cl |
| A-937 | 2-CF₃ | 2-CH₃, 4-CF(CF₃)₂ | | A-967 | 3-CF₃ | 2-OCH₃, 4-Cl |
| A-938 | 2-OCH₃ | 2-CH₃, 4-CF(CF₃)₂ | | A-968 | 3-OCH₃ | 2-OCH₃, 4-Cl |
| A-939 | 2-OCF₃ | 2-CH₃, 4-CF(CF₃)₂ | | A-969 | 3-OCF₃ | 2-OCH₃, 4-Cl |
| A-940 | 3-F | 2-CH₃, 4-CF(CF₃)₂ | | A-970 | 2-F | 3-NO₂, 4-CH₃ |
| A-941 | 3-Cl | 2-CH₃, 4-CF(CF₃)₂ | | A-971 | 2-Cl | 3-NO₂, 4-CH₃ |
| A-942 | 3-CH₃ | 2-CH₃, 4-CF(CF₃)₂ | | A-972 | 2-CH₃ | 3-NO₂, 4-CH₃ |
| A-943 | 3-CF₃ | 2-CH₃, 4-CF(CF₃)₂ | | A-973 | 2-CF₃ | 3-NO₂, 4-CH₃ |
| A-944 | 3-OCH₃ | 2-CH₃, 4-CF(CF₃)₂ | | A-974 | 2-OCH₃ | 3-NO₂, 4-CH₃ |
| A-945 | 3-OCF₃ | 2-CH₃, 4-CF(CF₃)₂ | | A-975 | 2-OCF₃ | 3-NO₂, 4-CH₃ |
| A-946 | 2-F | 2-OCH₃, 4-F | | A-976 | 3-F | 3-NO₂, 4-CH₃ |
| A-977 | 3-Cl | 3-NO₂, 4-CH₃ | | A-1007 | 3-Cl | 2-CF₃, 5-F |
| A-978 | 3-CH₃ | 3-NO₂, 4-CH₃ | | A-1008 | 3-CH₃ | 2-CF₃, 5-F |
| A-979 | 3-CF₃ | 3-NO₂, 4-CH₃ | | A-1009 | 3-CF₃ | 2-CF₃, 5-F |
| A-980 | 3-OCH₃ | 3-NO₂, 4-CH₃ | | A-1010 | 3-OCH₃ | 2-CF₃, 5-F |
| A-981 | 3-OCF₃ | 3-NO₂, 4-CH₃ | | A-1011 | 3-OCF₃ | 2-CF₃, 5-F |
| A-982 | 3-F | 2-CF₃, 4-Cl | | A-1012 | 3-F | 2-CF₃, 5-Cl |
| A-983 | 3-Cl | 2-CF₃, 4-Cl | | A-1013 | 3-Cl | 2-CF₃, 5-Cl |
| A-984 | 3-CH₃ | 2-CF₃, 4-Cl | | A-1014 | 3-CH₃ | 2-CF₃, 5-Cl |
| A-985 | 3-CF₃ | 2-CF₃, 4-Cl | | A-1015 | 3-CF₃ | 2-CF₃, 5-Cl |
| A-986 | 3-OCH₃ | 2-CF₃, 4-Cl | | A-1016 | 3-OCH₃ | 2-CF₃, 5-Cl |
| A-987 | 3-OCF₃ | 2-CF₃, 4-Cl | | A-1017 | 3-OCF₃ | 2-CF₃, 5-Cl |
| A-988 | 3-F | 2-Cl, 4-CF₃ | | A-1018 | 3-F | 2-Cl, 5-CF₃ |
| A-989 | 3-Cl | 2-Cl, 4-CF₃ | | A-1019 | 3-Cl | 2-Cl, 5-CF₃ |
| A-990 | 3-CH₃ | 2-Cl, 4-CF₃ | | A-1020 | 3-CH₃ | 2-Cl, 5-CF₃ |
| A-991 | 3-CF₃ | 2-Cl, 4-CF₃ | | A-1021 | 3-CF₃ | 2-Cl, 5-CF₃ |
| A-992 | 3-OCH₃ | 2-Cl, 4-CF₃ | | A-1022 | 3-OCH₃ | 2-Cl, 5-CF₃ |
| A-993 | 3-OCF₃ | 2-Cl, 4-CF₃ | | A-1023 | 3-OCF₃ | 2-Cl, 5-CF₃ |
| A-994 | 3-F | 2-CF₃, 4-OCH₃ | | A-1024 | 3-F | 2-F, 5-CF₃ |
| A-995 | 3-Cl | 2-CF₃, 4-OCH₃ | | A-1025 | 3-Cl | 2-F, 5-CF₃ |
| A-996 | 3-CH₃ | 2-CF₃, 4-OCH₃ | | A-1026 | 3-CH₃ | 2-F, 5-CF₃ |
| A-997 | 3-CF₃ | 2-CF₃, 4-OCH₃ | | A-1027 | 3-CF₃ | 2-F, 5-CF₃ |
| A-998 | 3-OCH₃ | 2-CF₃, 4-OCH₃ | | A-1028 | 3-OCH₃ | 2-F, 5-CF₃ |
| A-999 | 3-OCF₃ | 2-CF₃, 4-OCH₃ | | A-1029 | 3-OCF₃ | 2-F, 5-CF₃ |
| A-1000 | 3-F | 3-Cl, 4-CF₃ | | A-1030 | 3-F | 3-F, 4-CF₃ |
| A-1001 | 3-Cl | 3-Cl, 4-CF₃ | | A-1031 | 3-Cl | 3-F, 4-CF₃ |
| A-1002 | 3-CH₃ | 3-Cl, 4-CF₃ | | A-1032 | 3-CH₃ | 3-F, 4-CF₃ |
| A-1003 | 3-CF₃ | 3-Cl, 4-CF₃ | | A-1033 | 3-CF₃ | 3-F, 4-CF₃ |
| A-1004 | 3-OCH₃ | 3-Cl, 4-CF₃ | | A-1034 | 3-OCH₃ | 3-F, 4-CF₃ |
| A-1005 | 3-OCF₃ | 3-Cl, 4-CF₃ | | A-1035 | 3-OCF₃ | 3-F, 4-CF₃ |
| A-1006 | 3-F | 2-CF₃, 5-F | | A-1036 | 3-F | 3-Cl, 4-CF₃ |
| A-1037 | 3-Cl | 3-Cl, 4-CF₃ | | A-1049 | 3-Cl | 3-F, 4-Cl |
| A-1038 | 3-CH₃ | 3-Cl, 4-CF₃ | | A-1050 | 3-CH₃ | 3-F, 4-Cl |
| A-1039 | 3-CF₃ | 3-Cl, 4-CF₃ | | A-1051 | 3-CF₃ | 3-F, 4-Cl |
| A-1040 | 3-OCH₃ | 3-Cl, 4-CF₃ | | A-1052 | 3-OCH₃ | 3-F, 4-Cl |
| A-1041 | 3-OCF₃ | 3-Cl, 4-CF₃ | | A-1053 | 3-OCF₃ | 3-F, 4-Cl |
| A-1042 | 3-F | 3-Cl, 4-Cl | | A-1054 | 3-F | 3-CF₃, 4-Cl |
| A-1043 | 3-Cl | 3-Cl, 4-Cl | | A-1055 | 3-Cl | 3-CF₃, 4-Cl |
| A-1044 | 3-CH₃ | 3-Cl, 4-Cl | | A-1056 | 3-CH₃ | 3-CF₃, 4-Cl |
| A-1045 | 3-CF₃ | 3-Cl, 4-Cl | | A-1057 | 3-CF₃ | 3-CF₃, 4-Cl |
| A-1046 | 3-OCH₃ | 3-Cl, 4-Cl | | A-1058 | 3-OCH₃ | 3-CF₃, 4-Cl |
| A-1047 | 3-OCF₃ | 3-Cl, 4-Cl | | A-1059 | 3-OCF₃ | 3-CF₃, 4-Cl |
| A-1048 | 3-F | 3-F, 4-Cl | | | | |

### Table 2

Compounds of the formula (I.A) wherein Q denotes Q-2 and the combination of (R¹)ₙ and (R²)ₘ in each case corresponds to one line of Table B.

### Table 3

Compounds of the formula (I.A) wherein Q denotes Q-3 and the combination of (R¹)ₙ and (R²)ₘ in each case corresponds to one line of Table B.

### Table 4

Compounds of the formula (I.A) wherein Q denotes Q-4 and the combination of (R¹)ₙ and (R²)ₘ in each case corresponds to one line of Table B.

### Table 5

Compounds of the formula (I.A) wherein Q denotes Q-5 and the combination of (R¹)ₙ and (R²)ₘ in each case corresponds to one line of Table B.

### Table 6

Compounds of the formula (I.A) wherein Q denotes Q-6 and the combination of (R¹)ₙ and (R²)ₘ in each case corresponds to one line of Table B.

### Table 7

Compounds of the formula (I.A) wherein Q denotes Q-7 and the combination of (R¹)ₙ and (R²)ₘ in each case corresponds to one line of Table B.

### Table 8

Compounds of the formula (I.A) wherein Q denotes Q-8 and the combination of (R¹)ₙ and (R²)ₘ in each case corresponds to one line of Table B.

### Table 9

Compounds of the formula (I.A) wherein Q denotes Q-9 and the combination of (R¹)ₙ and (R²)ₘ in each case corresponds to one line of Table B.

### Table 10

Compounds of the formula (I.A) wherein Q denotes Q-10 and the combination of (R¹)ₙ and (R²)ₘ in each case corresponds to one line of Table B.

### Table 11

Compounds of the formula (I.A) wherein Q denotes Q-11 and the combination of (R¹)ₙ and (R²)ₘ in each case corresponds to one line of Table B.

### Table 12

Compounds of the formula (I.A) wherein Q denotes Q-12 and the combination of (R¹)ₙ and (R²)ₘ in each case corresponds to one line of Table B.

### Table 13

Compounds of the formula (I.A) wherein Q denotes Q-13 and the combination of (R¹)ₙ and (R²)ₘ in each case corresponds to one line of Table B.

### Table14

Compounds of the formula (I.A) wherein Q denotes Q-14 and the combination of (R¹)ₙ and (R²)ₘ in each case corresponds to one line of Table B.

### Table 15

Compounds of the formula (I.A) wherein Q denotes Q-15 and the combination of (R¹)ₙ and (R²)ₘ in each case corresponds to one line of Table B.

### Table 16

Compounds of the formula (I.A) wherein Q denotes Q-16 and the combination of (R¹)ₙ and (R²)ₘ in each case corresponds to one line of Table B.

### Table 17

Compounds of the formula (I.A) wherein Q denotes Q-17 and the combination of (R¹)ₙ and (R²)ₘ in each case corresponds to one line of Table B.

### Table 18

Compounds of the formula (I.A) wherein Q denotes Q-18 and the combination of (R¹)ₙ and (R²)ₘ in each case corresponds to one line of Table B.

### Table 19

Compounds of the formula (I.A) wherein Q denotes Q-19 and the combination of (R¹)ₙ and (R²)ₘ in each case corresponds to one line of Table B.

### Table 20

Compounds of the formula (I.A) wherein Q denotes Q-20 and the combination of (R¹)ₙ and (R²)ₘ in each case corresponds to one line of Table B.

### Table 21

Compounds of the formula (I.A) wherein Q denotes Q-21 and the combination of (R¹)ₙ and (R²)ₘ in each case corresponds to one line of Table B.

### Table 22

Compounds of the formula (I.A) wherein Q denotes Q-22 and the combination of (R¹)ₙ and (R²)ₘ in each case corresponds to one line of Table B.

### Table 23

Compounds of the formula (I.A) wherein Q denotes Q-23 and the combination of (R¹)ₙ and (R²)ₘ in each case corresponds to one line of Table B.

### Table 24

Compounds of the formula (I.A) wherein Q denotes Q-24 and the combination of (R¹)ₙ and (R²)ₘ in each case corresponds to one line of Table B.

### Table 25.

Compounds of the formula (I.A) wherein Q denotes Q-25 and the combination of (R¹)ₙ and (R²)ₘ in each case corresponds to one line of Table B.

### Table 26

Compounds of the formula (I.A) wherein Q denotes Q-26 and the combination of (R¹)ₙ and (R²)ₘ in each case corresponds to one line of Table B.

### Table 27

Compounds of the formula (I.A) wherein Q denotes Q-27 and the combination of (R¹)ₙ and (R²)ₘ in each case corresponds to one line of Table B.

### Table 28

Compounds of the formula (I.A) wherein Q denotes Q-28 and the combination of (R¹)ₙ and (R²)ₘ in each case corresponds to one line of Table B.

### Table 29

Compounds of the formula (I.A) wherein Q denotes Q-29 and the combination of (R¹)ₙ and (R²)ₘ in each case corresponds to one line of Table B.

### Table 30

Compounds of the formula (I.A) wherein Q denotes Q-30 and the combination of (R¹)ₙ and (R²)ₘ in each case corresponds to one line of Table B.

### Table 31

Compounds of the formula (I.A) wherein Q denotes Q-31 and the combination of (R¹)ₙ and (R²)ₘ in each case corresponds to one line of Table B.

### Table 32

Compounds of the formula (I.A) wherein Q denotes Q-32 and the combination of (R¹)ₙ and (R²)ₘ in each case corresponds to one line of Table B.

### Table 33

Compounds of the formula (I.A) wherein Q denotes Q-33 and the combination of (R¹)ₙ and (R²)ₘ in each case corresponds to one line of Table B.

### Table 34

Compounds of the formula (I.A) wherein Q denotes Q-34 and the combination of (R¹)ₙ and (R²)ₘ in each case corresponds to one line of Table B.

### Table 35

Compounds of the formula (I.A) wherein Q denotes Q-35 and the combination of (R¹)ₙ and (R²)ₘ in each case corresponds to one line of Table B.

### Table 36

Compounds of the formula (I.A) wherein Q denotes Q-36 and the combination of (R¹)ₙ and (R²)ₘ in each case corresponds to one line of Table B.

### Table 37

Compounds of the formula (I.A) wherein Q denotes Q-37 and the combination of (R¹)ₙ and (R²)ₘ in each case corresponds to one line of Table B.

### Table 38

Compounds of the formula (I.A) wherein Q denotes Q-38 and the combination of (R¹)ₙ and (R²)ₘ in each case corresponds to one line of Table B.

### Table 39.

Compounds of the formula (I.A) wherein Q denotes Q-39 and the combination of (R¹)ₙ and (R²)ₘ in each case corresponds to one line of Table B.

### Table 40

Compounds of the formula (I.A) wherein Q denotes Q-40 and the combination of (R¹)ₙ and (R²)ₘ in each case corresponds to one line of Table B.

### Table 41.

Compounds of the formula (I.A) wherein Q denotes Q-41 and the combination of (R¹)ₙ and (R²)ₘ in each case corresponds to one line of Table B.

### Table 42

Compounds of the formula (I.A) wherein Q denotes Q-42 and the combination of (R¹)ₙ and (R²)ₘ in each case corresponds to one line of Table B.

### Table 43

Compounds of the formula (I.A) wherein Q denotes Q-43 and the combination of (R¹)ₙ and (R²)ₘ in each case corresponds to one line of Table B.

### Table 44

Compounds of the formula (I.A) wherein Q denotes Q-44 and the combination of (R¹)ₙ and (R²)ₘ in each case corresponds to one line of Table B.

### Table 45

Compounds of the formula (I.A) wherein Q denotes Q-45 and the combination of (R¹)ₙ and (R²)ₘ in each case corresponds to one line of Table B.

### Table 46

Compounds of the formula (I.A) wherein Q denotes Q-46 and the combination of (R¹)ₙ and (R²)ₘ in each case corresponds to one line of Table B.

### Table 47

Compounds of the formula (I.A) wherein Q denotes Q-47 and the combination of (R¹)ₙ and (R²)ₘ in each case corresponds to one line of Table B.

### Table 48

Compounds of the formula (I.A) wherein Q denotes Q-48 and the combination of (R¹)ₙ and (R²)ₘ in each case corresponds to one line of Table B.

### Table 49

Compounds of the formula (I.A) wherein Q denotes Q-49 and the combination of (R¹)ₙ and (R²)ₘ in each case corresponds to one line of Table B.

### Table 50

Compounds of the formula (I.A) wherein Q denotes Q-50 and the combination of (R¹)ₙ and (R²)ₘ in each case corresponds to one line of Table B.

### Table 51

Compounds of the formula (I.A) wherein Q denotes Q-51 and the combination of (R¹)ₙ and (R²)ₘ in each case corresponds to one line of Table B.

### Table 52

Compounds of the formula (I.A) wherein Q denotes Q-52 and the combination of (R¹)ₙ and (R²)ₘ in each case corresponds to one line of Table B.

### Table 53

Compounds of the formula (I.A) wherein Q denotes Q-53 and the combination of (R¹)ₙ and (R²)ₘ in each case corresponds to one line of Table B.

### Table 54

Compounds of the formula (I.A) wherein Q denotes Q-54 and the combination of (R¹)ₙ and (R²)ₘ in each case corresponds to one line of Table B.

### Table 55

Compounds of the formula (I.A) wherein Q denotes Q-55 and the combination of (R¹)ₙ and (R²)ₘ in each case corresponds to one line of Table B.

The compounds of the formula (I) according to the present invention can be prepared by various routes in analogy to prior art processes known per se for preparing biphenyl compounds. Advantageously, they can be obtained as outlined in schemes 1 and 2.

Generally, quinoline compounds of formula (I) can be prepared by reaction of a compound of formula (II) with a boronic acid derivative of the formula (III) by a Suzuki coupling as shown in scheme 1.

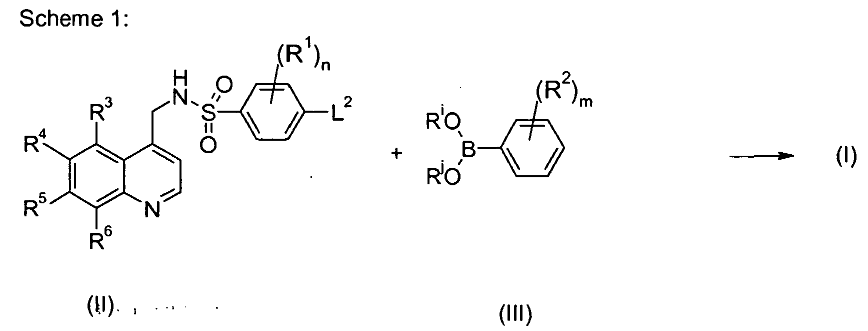

In scheme 1, R¹, R², R³, R⁴, R⁵, R⁶; n and m are as defined above, Rⁱ and R^{j} are each independently hydrogen or C₁-C₄-alkyl, or Rⁱ and R^{j} together form an 1,2-ethylene or 1,2-propylene moiety the carbon atoms of which may be unsubstituted or may all or in part be substituted by methyl groups, and L² is a suitable leaving group.

Suitable leaving groups L² are halogen, preferably chlorine, bromine or iodine, alkylcarboxylate, benzoate, alkylsulfonate, haloalkylsulfonate or arylsulfonate, most preferably chlorine or bromine.

The reaction is usually carried out in the presence of a base and a catalyst, in particular a palladium catalyst, such as for example described in the following literature: Synth. Commun. Vol. 11, p. 513 (1981); Acc. Chem. Res. Vol. 15, pp. 178-184 (1982); Chem. Rev. Vol. 95, pp. 2457-2483 (1995); Organic Letters Vol. 6 (16), p. 2808 (2004); "Metal catalyzed cross coupling reactions", 2nd Edition, Wiley, VCH 2005 (Eds. De Meijere, Diederich); "Handbook of organopalladium chemistry for organic synthesis" (Eds Negishi), Wiley, Interscience, New York, 2002; "Handbook of functionalized organometallics", (Ed. P. Knochel), Wiley, VCH, 2005.

Suitable catalysts are in tetrakis(triphenylphosphine)palladium(0); bis(triphenylphosphine)palladium(II) chloride; bis(acetonitrile)palladium(II) chloride; [1,1'-bis(diphenylphosphino)ferrocene]-palladium(II) chloride/methylene chloride (1:1) complex; bis[bis-(1,2-diphenylphosphino)ethane]palladium(0); bis(bis-(1,2-diphenylphosphino)butane]-palladium(II), chloride; palladium(II) acetate; palladium(II) chloride; and palladium(II) acetate/tri-o-tolylphosphine complex or mixtures of phosphines and Pd salts or phosphines and Pd-complexes e.g. dibenzylideneacetone-palladium and tri-tert-butylphosphine (or its tetrafluoroborate), triscyclohexylphosphine; or a polymer-bound Pd-triphenylphosphine catalyst system.

Suitable bases are, in general, inorganic compounds, such as alkali metal and alkaline earth metal oxides, such as lithium oxide, sodium oxide, calcium oxide and magnesium oxide, alkali metal and alkaline earth metal carbonates, such as lithium carbonate, sodium carbonate, potassium carbonate, caesium carbonate and calcium carbonate, and also alkali metal bicarbonates, such as sodium bicarbonate, alkali metal and alkaline earth metal alkoxides, such as sodium methoxide, sodium ethoxide, potassium ethoxide and potassium tert.-butoxide, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to bases such as sodium carbonate, potassium carbonate, caesium carbonate, triethylamine and sodium bicarbonate.

The base is used in a 1:1 to 1:10, preferably a 1:1.5 to 5 molar ratio relative to 1 mole of compounds (II), the boronic acid is used in a 1:1 to 1: 5 ratio, preferably a 1:1 to 1:2.5 molar ratio relative to 1 mole of compounds (II). In some cases it may be benefical for easy purification to use the boronic acid in a substoechiometric amount of from 0.7:1 to 0.99:1, relative to 1 mole of compounds (II).

The reaction is usually carried out in an inert organic organic solvent. Suitable solvents are aliphatic hydrocarbons, such as pentane, hexane, cyclohexane and petroleum ether, aromatic hydrocarbons, such as toluene, o-, m- and p-xylene, ethers, such as diisopropyl ether, tert.-butyl methyl ether, dioxane, anisole and tetrahydrofuran and dimethoxyethane, ketones, such as acetone, methyl ethyl ketone, diethyl ketone and tert.-butyl methyl ketone, and also dimethyl sulfoxide, dimethylformamide and dimethylacetamide, particularly preferably ethers, such as tetrahydrofuran, dioxane and dimethoxyethane. It is also possible to use mixtures of the solvents mentioned, or mixtures with water.

The reaction is usually carried out at temperatures of from 20°C to 180°C, preferably from 40°C to 120°C.

After completion of the reaction, the compounds of formula (I) can be isolated by employing conventional methods such as adding the reaction mixture to water, extracting with an organic solvent, concentrating the extract an the like. The isolated compounds (I) can be purified by a technique such as chromatography, recrystallization and the like, if necessary.

It is also possible to add a scavenger to the reaction mixtures to remove byproducts or unreacted starting materials by binding to those and simple filtration. For details see "Synthesis and purification catalog", Argonaut, 2003 and literature cited therein.

Boronic acids or esters (III) are commercially available or can be prepared according to "Science of Synthesis" Vol. 6, Thieme, 2005; WO 02/042275; Synlett 2003, (8) p.1204; J. Org. Chem., 2003, 68, p. 3729, Synthesis, 2000, p.442, J. Org. Chem., 1995, 60, p. 750; or "Handbook of functionalized organometallics", (Ed. P. Knochel), Wiley, VCH, 2005.

Compounds (II) can be obtained by reaction of sulfonylchlorides (IV) with quinolines (V) as shown in scheme 2.

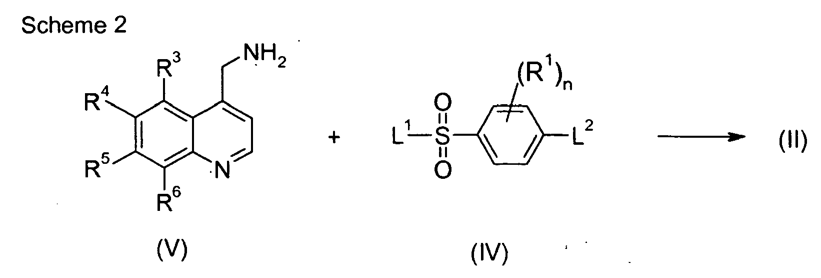

In scheme 2, L², R¹, R², R³, R⁴, R⁵, R⁶, n and m are as defined above, and L¹ is a leaving group such as hydroxy or halogen, preferably chlorine.

The reaction of a sulfonylchloride (IV) with a quinoline (V) can be performed in accordance with standard methods of organic chemistry, see for example, Lieb. Ann. Chem. P. 641, 1990, or WO 2005/033081

This reaction is usually carried out in an inert organic solvent. Suitable solvents are aliphatic hydrocarbons, such as pentane, hexane, cyclohexane and petroleum ether, aromatic hydrocarbons, such as toluene, o-, m- and p-xylene, halogenated hydrocarbons, such as dichloromethane, chloroform and chlorobenzene, ethers, such as diethyl ether, diisopropyl ether, tert.-butyl methyl ether, dioxane, anisole and tetrahydrofuran, nitriles, such as acetonitrile and propionitrile, ketones, such as acetone, methyl ethyl ketone, diethyl ketone and tert.-butyl methyl ketone, and also dimethyl sulfoxide, dimethylformamide and dimethylacetamide, preferably tetrahydrofuran, methyl tert-butyl ether, methylene chloride, chloroform, acetonitrile, toluene or dimethylformamide. It is also possible to use mixtures of the solvents mentioned.

It may be advantageous to carry out the reaction in the presence of a base. Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, disopropylethylamine and N-methylpiperidine, pyridine, substituted pyridines, such as collidine, lutidine and 4-dimethylaminopyridine, and also bicyclic amines. Particular preference is given to pyridine, triethylamine and potassium carbonate. The bases are generally employed in equimolar amounts, in excess or, if appropriate, as solvent. The excess of base is typically 0.5 to 5 molar equivalents relative to 1 mole of compounds (V).

Generally, the reaction is carried out at temperatures of from -30°C to 120°C, preferably from -10°C to 100°C.

The starting materials are generally reacted with one another in equimolar amounts.

If sulfonylchlorides (IV) are not commercially available, they can be obtained according to procedures known in the art.

Quinolines (V) are known from the literature or are commercially available (e.g.: 4-methylenaminoquinoline: CAS-Nr 5632-13-3; 6-chloro-4-methylenaminoquinoline: CAS-Nr 859814-05-5; 6-methoxy-4-methylenaminoquinoline: CAS-Nr 708261-71-6; 8-hydroxy-4-methylenaminoquinoline: CAS-Nr 33976-91-9; 6-methoxy-8-chloro-4-methylenaminoquinoline: CAS-Nr 857207-07-9), or they can be prepared from quinoline precursors (VI) wherein X is as defined in the following scheme by reduction:

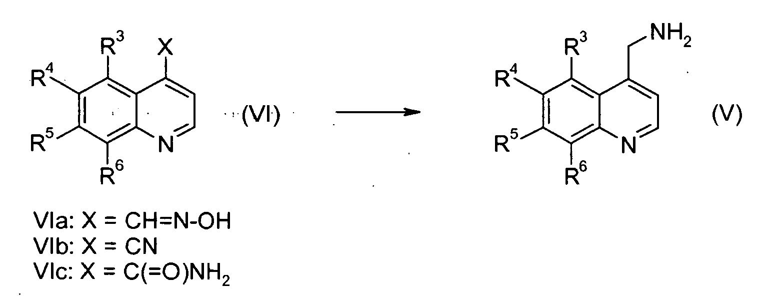

Methods of this reduction can be found in the literature e.g. in Houben-Weyl, Band 10/4, Thieme, Stuttgart, 1968; Band 11/2, 1957; Band E5, 1985; J. Heterocycl. Chem., 1997, 34 (6), pp. 1661-1667; J. Chem. Soc. 1954, p. 1165; Heterocycles, 41(4), pp. 675-688, 1995; J. Org. Chem., 1982, 47, p. 3153; Heterocycles, 1996, 43 (9), pp.1893-1900; J. Prakt. Chem-Chem. Ztg. 336(8), pp. 695-697, 1994; or are known to those skilled in the art.

The oxims (VIa) can be prepared from either the respective aldehyd (X=CHO; compounds (Vld)) or the methylderivative (X=CH₃; compounds (VIe)), as described in Houben-Weyl, Band 10/4, Thieme, Stuttgart, 1968; Band 11/2, 1957; Band E5, 1985; J. Prakt. Chem-Chem. Ztg. 336(8), pp. 695-697, 1994; Tetrahedron Lett. 42(39), pp.6815-6818, 2001; or Heterocycles, 29(9), pp.1741-1760, 1989.

The aldehyds (Vld) are commercially available (e.g 6-chloro-4-quinolinecarbaldehyde, 7-methoxy-4-quinolinecarbaldehyde, quinoline-4-carbaldehyde) or can be synthesized from a 4-methylquinoline as outlined in J. Org. Chem. 51(4), pp. 536-537, 1986, or from a haloderivative (X= halogen, compounds (VIf)) as shown in Eur. J. Org. Chem., 2003, (8), pp. 1576-1588; Tetrahedron Lett. 1999, 40 (19), pp. 3719-3722; Tetrahedron, 1999, 55 (41), pp. 12149-12156.

The methyl derivatives (VIe) are commercially available (e.g. 6-chloro 4-methylquinoline; 6,8-dimethoxy-quinoline) or can be synthesized according to "Science of Synthesis", Vol 15, Thieme, Stuttgart, 2005.

The nitriles (Vlb) can be prepared either from the respective halogen derivative (VIf) (X=halogen, preferably chlorine, bromine or iodine,) by reaction with a cyanide source with or without additional catalysts, as described e.g. in Tetrahedron Lett. 42(38), pp. 6707-6710, 2001; Chem. Eur. J., 2003, 9 (8), pp. 1828-1836; Chem. Commun. (Cambridge), 2004, (12), pp. 1388-1389; J. Organomet. Chem. 2004, 689 (24), pp. 4576-4583; or J. Chem. Soc. Perk. T., 1 (16), pp. 2323-2326, 1999. Alternatively, the amide or oxime may be dehydrated to the corresponding nitrile (Vlb) as outlined in "Synthesis", Stuttgart, (10), pp. 943-944, 1992; or literature cited therein; or Heterocycl. Chem. 1997, 34 (6), pp. 1661-1667.

The 4-halogene quinolines (VIf) are either commercially available or can be synthesized according to "Science of Synthesis", Vol 15, Thieme, Stuttgart, 2005 or e.g. according to the following literature or citations therein: 4-chloro-6,7-dimethoxy-quinoline: Journal Med. Chem. 48(5), p. 1359, 2005; 4-chloro-5,7-dichloro-quinoline: Indian, 187817, 29 Jun 2002; 4-chloro-7-chloro-quinoline: Tetrahedron, 60 (13), p. 3017, 2004; 4-chloro-7-trifluoromethyl-quinoline;Tetrahedron lett., 31(8), p. 1093, 1990; 4-chloro-7,8-dimethoxy-quinoline: Tetrahedron, 41 (15), p.3033, 1985; 4-chloro-8-methoxy quinoline: Chem. Berichte 118(4), p.1556, 1985; 4-chloro-(6 or 7 or 8)-iodo quinoline, 4-bromo-(6 or 7 or 8)-iodo-quinoline, 4-iodo-(6 or 7 or 8)-iodo-quinoline: J. Med. Chem., 21(3), p. 268, 1978.

Further methods to build up appropriate precursors or modify substitution pattern can be found in "Synthesis", Stuttgart (1), pp. 31-32, 1993; Tetrahedron, 1993, 49 (24), pp. 5315-5326; "Methods in Science of Synthesis", Band 15, and literature cited therein; Bioorg. Med. Chem. Lett. 1997, 7 (23), pp. 2935-2940; J. Am. Chem. Soc., 1946, 68, p. 1264; or Org. Synth.1955, III, p. 272.

The N-oxides may be prepared from the compounds (I) according to conventional oxidation methods, for example by treating a compound (I) with an organic peracid such as metachloroperbenzoic acid [Journal of Medicinal Chemistry, 38(11), 1892-1903 (1995); WO 03/64572] or with inorganic oxidizing agents such as hydrogen peroxide, [see Jounal of Heterocyclic Chemistry, 18(7), 1305-8 (1981)] or oxone, see Journal of the American Chemical Society, 123(25), 5962-5973 (2001).

In some cases it can be beneficial in terms of ease of work up or purification to perform the reduction of compounds (VI) to compounds (V) and the reaction of the amine (V) with the compound (IV) in one pot without isolating compounds (V).

Some of the intermediates of formula (II) are novel. These are also subject of this invention.

Especially, the intermediates of formula (II.1) are subject of the present invention: 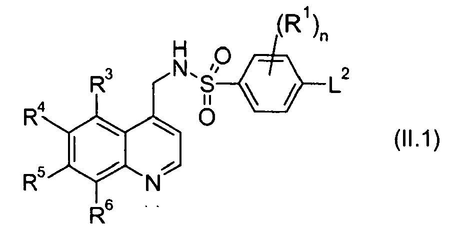 wherein
L² is chlorine, bromine or iodine, and
n, R¹, R³, R⁴, R⁵ and R⁶ have the meanings as defined above for compounds of formula (I), especially those given as preferred ones.

If individual compounds (I) are not obtainable by the routes described above, they can be prepared by derivatization of other compounds (I) or by customary modifications of the synthesis routes described.

The preparation of the compounds of formula (I) may lead to them being obtained as isomer mixtures (stereoisomers, enantiomers). If desired, these can be resolved by the methods customary for this purpose, such as crystallization or chromatography, also on optically active adsorbate, to give the pure isomers.

Due to their excellent activity, the compounds of the general formula (I) as well as the N-oxides and salts thereof may be used for controlling animal pests, selected from harmful insects, arachnids and nematodes.

Accordingly, the invention further relates to agriculturally composition for combating such animal pests, which comprises such an amount of at least one compound of the general formula (I), an N-oxide or an agronomically acceptable salt thereof, and at least one inert liquid and/or solid agronomically acceptable carrier that has a pesticidal action and, if desired, at least one surfactant.

Such a composition may contain a single active compound of the formula (I), an N-oxide or an agronomically acceptable salt thereof, or a mixture of several active compounds of the formula (I), N-oxides or agronomically acceptable salts thereof according to the present invention.

The compounds of the formula (I) as well as N-oxides or agronomically acceptable salts thereof and the pestidicidal compositions comprising them are effective agents for controlling arthropod pests and nematodes. Animal pests controlled by the compounds of formula (I) include for example

Insects from the order of the lepidopterans (Lepidoptera), for example Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutelia xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni and Zeiraphera canadensis;
beetles (Coleoptera), for example Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus and Sitophilus granaria;
dipterans (Diptera), for example Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea and Tipula paludosa;
thrips (Thysanoptera), e.g. Dichromothrips corbetti, Frankliniella fusca, Frankliniella occidentalis, Frankliniella tritici, Scirtothrips citri, Thrips oryzae, Thrips palmi and Thrips tabaci;
hymenopterans (Hymenoptera), e.g. Athalia rosae, Atta cephalotes, Atta sexdens, Atta texana, Hoplocampa minuta, Hoplocampa testudinea, Monomorium pharaonis, Solenopsis geminata and Solenopsis invicta;
heteropterans (Heteroptera), e.g. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis and Thyanta perditor;
homopterans (Homoptera), e.g. Acyrthosiphon onobrychis, Adelges laricis, Aphidula nasturtii, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Bemisia argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzodes persicae, Myzus ascalonicus, Myzus cerasi, Myzus persicae, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Sogatella furcifera Trialeurodes vaporariorum, Toxoptera aurantiiand, and Viteus vitifolii;
termites (Isoptera), e.g. Calotermes flavicollis, Leucotermes flavipes, Reticulitermes flavipes, Reticulitermes lucifugus und Termes natalensis;
orthopterans (Orthoptera), e.g. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus and Tachycines asynamorus ;
Arachnoidea, such as arachnids (Acarina), e.g. of the families Argasidae, Ixodidae and Sarcoptidae, such as Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Dermanyssus gallinae, Psoroptes ovis, Rhipicephalus appendiculatus, Rhipicephalus evertsi, Sarcoptes scabiei, and Eriophyidae spp. such as Aculus schlechtendali, Phyllocoptrata oleivora and Eriophyes sheldoni; Tarsonemidae spp. such as Phytonemus pallidus and Polyphagotarsonemus latus; Tenuipalpidae spp. such as Brevipalpus phoenicis; Tetranychidae spp. such as Tetranychus cinnabarinus, Tetranychus kanzawai, Tetranychus pacificus, Tetranychus telarius and Tetranychus urticae, Panonychus ulmi, Panonychus citri, and oligonychus pratensis ;
Siphonatera, e.g. Xenopsylla cheopsis, Ceratophyllus spp.; The compositions and compounds of formula (I) are useful for the control of nematodes, especially plant parasitic nematodes such as root knot nematodes, Meloidogyne hapla,Meloidogyne incognita, Meloidogyne javanica, and other Meloidogyne species;
cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Paratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species.

In a preferred embodiment of the invention the compounds of formula (I), their N-oxides or their agronomically acceptable salts are used for controlling arthropodes, such as insects or arachnids, in particular insects of the orders Lepidoptera, Coleoptera and Homoptera and arachnids of the order Acarina. The compounds of the formula (I) according to the present invention are particularly useful for controlling insects of the order Thysanoptera and Homoptera.

The compounds of formula (I), their N-oxides, their agronomically acceptable salts, or the pesticidal compositions comprising them may be used to protect growing plants and crops from attack or infestation by animal pests, especially insects, acaridae or arachnids by contacting the plant/crop with a pesticidally effective amount of compounds of formula (I). The term "crop" refers both to growing and harvested crops.

The compounds of formula (I), their N-oxides and their agronomically acceptable salts, can be converted into the customary formulations, for example solutions, emulsions, suspensions, dusts, powders, pastes and granules. The use form depends on the particular intended purpose; in each case, it should ensure a fine and even distribution of the compound according to the invention.

The formulations are prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 and Mollet, H., Grubemann, A., Formulation tech-nology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active com-pound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Examples of suitable solvents are water, aromatic solvents (for example Solvesso products, xylene), paraffins (for example mineral oil fractions), alcohols (for example methanol, butanol, pentanol, benzyl alcohol), ketones (for example cyclohexanone, gamma-butyrolactone), pyrrolidones (N-methyl-pyrrolidones [NMP], N-octyl-pyrrolidone [NOP]), acetates (glycol diacetate), glycols, fatty acid dimethylamides, fatty acids and fatty acid esters. In principle, solvent mixtures may also be used.

Suitable emulsifiers are nonionic and anionic emulsifiers (for example polyoxyethylene fatty alcohol ethers, alkylsulfonates and arylsulfonates).

Examples of dispersants are lignin-sulfite waste liquors and methylcellulose.

Suitable surfactants used are alkali metal, alkaline earth metal and ammonium salts of lignosulfonic acid, naphthalenesulfonic acid, phenolsulfonic acid, dibutylnaphthalene-sulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol ethylene oxide condensates, ethoxylated castor oil, polyoxyethylene alkyl ethers, ethoxylated polyoxypropyl-ene, lauryl alcohol polyglycol ether acetal, sorbitol esters, lignosulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, highly polar solvents, for example dimethyl sulfoxide, N-methylpyrrolidone or water.

Also anti-freezing agents such as glycerin, ethylene glycol, propylene glycol and bacte-ricides such as can be added to the formulation.

Suitable antifoaming agents are for example antifoaming agents based on silicon or magnesium stearate.

A suitable preservative is e.g. dichlorophen.

Seed treatment formulations may additionally comprise binders and optionally colorants.

Binders can be added to improve the adhesion of the active materials on the seeds after treatment. Suitable binders are block copolymers EO/PO surfactants but also polyvinylalcoholsl, polyvinylpyrrolidones, polyacrylates, polymethacrylates, polybute-nes, polyisobutylenes, polystyrene, polyethyleneamines, polyethyleneamides, poly-ethyleneimines (Lupasol®, Polymin®), polyethers, polyurethans, polyvinylacetate, ty-lose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pig-ment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Example of a gelling agent is carrageen (Satiagel®).

Powders, materials for spreading and dustable products can be prepared by mixing or concomitantly grinding the active substances with a solid carrier.

Granules, for example coated granules, impregnated granules and homogeneous granules, can be prepared by binding the active compounds to solid carriers.

Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the active compound(s). In this case, the active compound(s) are employed in a purity of from 90% to 100% by weight, preferably 95% to 100% by weight (according to NMR spectrum).

For seed treatment purposes, respective formulations can be diluted 2-10 fold leading to concentrations in the ready to use preparations of 0.01 to 60% by weight active compound by weight, preferably 0.1 to 40% by weight.

The compounds of formula (I), their N-oxides and their agronomically acceptable salts, can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the active compound(s) according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. However, it is also possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1% per weight.

The active compound(s) may also be used successfully in the ultra-low-volume process (ULV), it being possible to apply formulations comprising over 95% by weight of active compound, or even to apply the active compound without additives.

The following are examples of formulations:
1. Products for dilution with water for foliar applications. For seed treatment purposes, such products may be applied to the seed diluted or undiluted.
   A) Water-soluble concentrates (SL, LS)
      10 parts by weight of the active compound(s) are dissolved in 90 parts by weight of water or a water-soluble solvent. As an alternative, wetters or other auxiliaries are added. The active compound(s) dissolves upon dilution with water, whereby a formulation with 10% (w/w) of active compound(s) is obtained.
   B) Dispersible concentrates (DC)
      20 parts by weight of the active compound(s) are dissolved in 70 parts by weight of cyclohexanone with addition of 10 parts by weight of a dispersant, for example polyvi-nylpyrrolidone. Dilution with water gives a dispersion, whereby a formulation with 20% (w/w) of active compound(s) is obtained.
   C) Emulsifiable concentrates (EC)
      15 parts by weight of the active compound(s) are dissolved in 7 parts by weight of xy-lene with addition of calcium dodecylbenzenesulfonate and castor oil ethoxylate (in each case 5 parts by weight). Dilution with water gives an emulsion, whereby a formulation with 15% (w/w) of active compound(s) is obtained.
   D) Emulsions (EW, EO, ES)
      25 parts by weight of the active compound(s) are dissolved in 35 parts by weight of xylene with addition of calcium dodecylbenzenesulfonate and castor oil ethoxylate (in each case 5 parts by weight). This mixture is introduced into 30 parts by weight of wa-ter by means of an emulsifier machine (e.g. Ultraturrax) and made into a homogeneous emulsion. Dilution with water gives an emulsion, whereby a formulation with 25% (w/w) of active compound(s) is obtained.
   E) Suspensions (SC, OD, FS)
      In an agitated ball mill, 20 parts by weight of the active compound(s) are comminuted with addition of 10 parts by weight of dispersants, wetters and 70 parts by weight of water or of an organic solvent to give a fine active compound(s) suspension. Dilution with water gives a stable suspension of the active compound(s), whereby a formulation with 20% (w/w) of active compound(s) is obtained.
   F) Water-dispersible granules and water-soluble granules (WG, SG)
      50 parts by weight of the active compound(s) are ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the active com-pound(s), whereby a formulation with 50% (w/w) of active compound(s) is obtained.
   G) Water-dispersible powders and water-soluble powders (WP, SP, SS, WS)
      75 parts by weight of the active compound(s) are ground in a rotor-stator mill with addi-tion of 25 parts by weight of dispersants, wetters and silica gel. Dilution with water gives a stable dispersion or solution of the active compound(s), whereby a formulation with 75% (w/w) of active compound(s) is obtained.
   H) Gel-Formulation (GF)
      In an agitated ball mill, 20 parts by weight of the active compound(s) are comminuted with addition of 10 parts by weight of dispersants, 1 part by weight of a gelling agent wetters and 70 parts by weight of water or of an organic solvent to give a fine active compound(s) suspension. Dilution with water gives a stable suspension of the active compound(s), whereby a formulation with 20% (w/w) of active compound(s) is ob-tained.
2. Products to be applied undiluted for foliar applications. For seed treatment purposes, such products may be applied to the seed diluted or undiluted.
   I) Dustable powders (DP, DS)
      5 parts by weight of the active compound(s) are ground finely and mixed intimately with 95 parts by weight of finely divided kaolin. This gives a dustable product having 5% (w/w) of active compound(s)
   J) Granules (GR, FG, GG, MG)
      0.5 parts by weight of the active compound(s) is ground finely and associated with 95.5 parts by weightof carriers, whereby a formulation with 0.5% (w/w) of active com-pound(s) is obtained. Current methods are extrusion, spray-drying or the fluidized bed. This gives granules to be applied undiluted for foliar use.
   K) ULV solutions (UL)
      10 parts by weight of the active compound(s) are dissolved in 90 parts by weight of an organic solvent, for example xylene. This gives a product having 10% (w/w) of active compound(s), which is applied undiluted for foliar use.

The compounds of formula (I) are also suitable for the treatment of seeds. Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter

In a preferred embodiment a FS formulation is used for seed treatment. Typcially, a FS formulation may comprise 1-800 g/l of active ingredient, 1-200 g/l of surfactant, 0 to 200 g/l of antifreezing agent, 0 to 400 g/l of binder, 0 to 200 g/l of a pigment and up to 1 liter of a solvent, preferably water.

Other preferred FS formulations of compounds of formula (I) for seed treatment comprise from 0.5 to 80 wt% of the active ingredient, from 0,05 to 5 wt% of a wetter, from 0.5 to 15 wt% of a dispersing agent, from 0,1 to 5 wt% of a thickener, from 5 to 20 wt% of an anti-freeze agent, from 0,1 to 2 wt% of an anti-foam agent, from 1 to 20 wt% of a pigment and/or a dye, from 0 to 15 wt% of a sticker /adhesion agent, from 0 to 75 wt% of a filler/vehicle, and from 0,01 to 1 wt% of a preservative.

Various types of oils, wetters, adjuvants, herbicides, fungicides, other pesticides, or bactericides may be added to the active ingredients, if appropriate just immediately prior to use (tank mix). These agents usually are admixed with the agents according to the invention in a weight ratio of 1:10 to 10:1.

The compounds of formula (I), their N-oxides and their agronomically acceptable salts, are effective through both contact (via soil, glass, wall, bed net, carpet; plant parts or animal parts), and ingestion (bait, or plant part).

For use against ants, termites, wasps, flies, mosquitos, crickets, or cockroaches, compounds of formula (I), their N-oxides and their agronomically acceptable salts are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics.

The bait employed in the composition is a product, which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

Formulations of compounds of formula (I), their N-oxides and their agronomically acceptable salts, such as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250°C, dimethylformamide, N-methylpyrrolidone, dimethyl sulphoxide (DMSO), aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

The compounds of formula (I), their N-oxides and their agronomically acceptable salts and their respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of formula (I), their N-oxides and their agronomically acceptable salts and its respective compositions also comprise treating surfaces of huts and houses, air spraying and impregnation of curtains, tents, clothing items, bed nets, tsetse-fly trap or the like. Insecticidal compositions for application to fibers, fabric, knitgoods, nonwovens, netting material or foils and tarpaulins preferably comprise a mixture including the insecticide, optionally a repellent and at least one binder. Suitable repellents for example are N,N-diethyl-meta-toluamide (DEET), N,N-diethylphenylacetamide (DEPA), 1-(3-cyclohexen-1-yl-carbonyl)-2-methylpiperine, (2-hydroxymethylcyclohexyl) acetic acid lactone, 2-ethyl-1,3-hexandiol, indalone, methylneodecanamide (MNDA), a pyrethroid not used for insect control such as {(+/-)-3-allyl-2-methyl-4-oxocyclopent-2-(+)-enyl-(+)-trans-chrysantemate (Esbiothrin), a repellent derived from or identical with plant extracts like limonene, eugenol, (+)-Eucamalol (1), (-)-1-epi-eucamalol or crude plant extracts from plants like Eucalyptus maculata, Vitex rotundifolia, Cymbopogan martinii, Cymbopogan citratus (lemon grass), Cymopogan nartdus (citronella). Suitable binders are selected for example from polymers and copolymers of vinyl esters of aliphatic acids (such as such as vinyl acetate and vinyl versatate), acrylic and methacrylic esters of alcohols, such as butyl acrylate, 2-ethylhexylacrylate, and methyl acrylate, mono- and di-ethylenically unsaturated hydrocarbons, such as styrene, and aliphatic diens, such as butadiene.

The impregnation of curtains and bednets is done in general by dipping the textile material into emulsions or dispersions of the insecticide or spraying them onto the nets.

The compounds of formula (I), their N-oxides and their or veterinarily acceptable salts thereof are in particular also suitable for being used for combating parasites in and on animals.

An object of the present invention is therfore also to provide new methods to control parasites in and on animals. Another object of the invention is to provide safer pesticides for animals. Another object of the invention is further to provide pesticides for animals that may be used in lower doses than existing pesticides. And anther object of the invention is to provide pesticides for animals, which provide a long residual control of the parasites.

The invention also relates to compositions containing a parasiticidally effective amount of compounds of formula (I), their N-oxides or veterinarily acceptable salts thereof and an acceptable carrier, for combating parasites in and on animals.

The present invention also relates to a method for treating, controlling, preventing and protecting animals against infestation and infection by parasites, which comprises orally, topically or parenterally administering or applying to the animals a parasiticidally effective amount of a compound of formula (I), the N-oxide or veterinarily acceptable salts thereof or a composition comprising it.

The invention also relates to a process for the preparation of a composition for treating, controlling, preventing or protecting animals against infestation or infection by parasites which comprises a parasiticidally effective amount of a compound of formula (I), the N-oxide or veterinarily acceptable salts thereof or a composition comprising it.

Activity of compounds against agricultural pests does not suggest their suitability for control of endo- and ectoparasites in and on animals which requires, for example, low, non-emetic dosages in the case of oral application; metabolic compatibility with the animal, low toxicity, and a safe handling.

Surprisingly, it has now been found that compounds of formula (I), their N-oxides and the veterinarily acceptable salts thereof are suitable for combating endo- and ectoparasites in and on animals.

Compounds of formula (I), the N-oxides or veterinarily acceptable salts thereof and compositions comprising them are preferably used for controlling and preventing infestations and infections animals including warm-blooded animals (including humans) and fish. They are for example suitable for controlling and preventing infestations and infections in mammals such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels.

Compounds of formula (I), the N-oxide thereof or veterinarily acceptable salts thereof and compositions comprising them are preferably used for controlling and preventing infestations and infections in domestic animals, such as dogs or cats.

Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of formula (I), the N-oxides or veterinarily acceptable salts thereof and compositions comprising them are suitable for systemic and/or non-systemic control of ecto- and/or endoparasites. They are active against all or some stages of development.

The compounds of formula (I), the N-oxides or veterinarily acceptable salts thereof are especially useful for combating ectoparasites.

The compounds of formula (I), the N-oxides or veterinarily acceptable salts thereof are especially useful for combating parasites of the following orders and species, respectively:
fleas (Siphonaptera), e.g. Ctenocephalides felis, Ctenocephalides canis, Xenopsylla cheopis, Pulex irritans, Tunga penetrans, and Nosopsyllus fasciatus,
cockroaches (Blattaria - Blattodea), e.g. Blattella germanica, Blattella asahinae, Periplaneta americana, Periplaneta japonica, Periplaneta brunnea, Periplaneta fuligginosa, Periplaneta australasiae, and Blatta orientalis,
flies, mosquitoes (Diptera), e.g. Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola, and Tabanus similis,
lice (Phthiraptera), e.g. Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus and Solenopotes capillatus.

ticks and parasitic mites (Parasitiformes): ticks (Ixodida), e.g. Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata and parasitic mites (Mesostigmata), e.g. Ornithonyssus bacoti and Dermanyssus gallinae;
Actinedida (Prostigmata) und Acaridida (Astigmata) e.g. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp.,Knemidocoptes spp., Cytodites spp., and Laminosioptes spp;
Bugs (Heteropterida): Cimex lectularius, Cimex hemipterus, Reduvius senilis, Triatoma spp., Rhodnius ssp., Panstrongylus ssp. and Arilus critatus,
Anoplurida, e.g. Haematopinus spp., Linognathus spp., Pediculus spp., Phtirus spp., and Solenopotes spp;
Mallophagida (suborders Arnblycerina and Ischnocerina), e.g. Trimenopon spp., Menopon spp., Trinoton spp., Bovicola spp., Werneckiella spp., Lepikentron spp., Trichodectes spp., and Felicola spp;
Roundworms Nematoda:
   Wipeworms and Trichinosis (Trichosyringida), e.g. Trichinellidae (Trichinella spp.), (Trichuridae) Trichuris spp., Capillaria spp;
   Rhabditida, e.g. Rhabditis spp, Strongyloides spp., Helicephalobus spp;
   Strongylida, e.g. Strongylus spp., Ancylostoma spp., Necator americanus, Bunostomum spp. (Hookworm), Trichostrongylus spp., Haemonchus contortus., Ostertagia spp. , Cooperia spp., Nematodirus spp., Dictyocaulus spp., Cyathostoma spp., Oesophagostomum spp., Stephanurus dentatus, Ollulanus spp., Chabertia spp., Stephanurus dentatus , Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp. Aleurostrongylus abstrusus, and Dioctophyma renal;
   Intestinal roundworms (Ascaridida), e.g. Ascaris lumbricoides, Ascaris suum, Ascaridia galli, Parascaris equorum, Enterobius vermicularis (Threadworm), Toxocara canis, Toxascaris leonine, Skrjabinema spp., and Oxyuris equi;
   Camallanida, e.g. Dracunculus medinensis (guinea worm);
   Spirurida, e.g. Thelazia spp. Wuchereria spp., Brugia spp., Onchocerca spp., Dirofilari spp.a, Dipetalonema spp., Setaria spp., Elaeophora spp., Spirocerca lupi, and Habronema spp.;
   Thorny headed worms (Acanthocephala), e.g. Acanthocephalus spp., Macracanthorhynchus hirudinaceus and Oncicola spp.;
Planarians (Plathelminthes):
   Flukes (Trematoda), e.g. Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp., and Nanocyetes spp,
   Cercomeromorpha, in particular Cestoda (Tapeworms), e.g. Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp., and Hymenolepis spp.

The compounds of formula (I) and compositions containing them are particularly useful for the control of pests from the orders Diptera, Siphonaptera and Ixodida.

Moreover, the use of the compounds of formula (I), the N-oxides or salts thereof and compositions containing them for combating mosquitoes is especially preferred.

The use of the compounds of formula (I), the N-oxides or salts thereof and compositions containing them for combating flies is a further preferred embodiment of the present invention.

Furthermore, the use of the compounds of formula (I), the N-oxides or salts thereof and compositions containing them for combating fleas is especially preferred.

The use of the compounds of formula (I), the N-oxides or salts thereof and compositions containing them for combating ticks is a further preferred embodiment of the present invention.

The compounds of formula (I), the N-oxides or salts thereof also are especially useful for combating endoparasites (roundworms nematoda, thorny headed worms and planarians).

Administration can be carried out both prophylactically and therapeutically.

Administration of the active compounds is carried out directly or in the form of suitable preparations, orally, topically/dermally or parenterally.

For oral administration to warm-blooded animals, the compounds of formula (I), the N-oxides or veterinarily salts thereof may be formulated as animal feeds, animal feed premixes, animal feed concentrates, pills, solutions, pastes, suspensions, drenches, gels, tablets, boluses and capsules. In addition, the compounds of formula (I) the N-oxides or veterinarilly acceptable salts thereof may be administered to the animals in their drinking water. For oral administration, the dosage form chosen should provide the animal with 0.01 mg/kg to 100 mg/kg of animal body weight per day of the formula (I) compound, preferably with 0.5 mg/kg to 100 mg/kg of animal body weight per day.

Alternatively, the compounds of formula (I) may be administered to animals parenterally, for example, by intraruminal, intramuscular, intravenous or subcutaneous injection. The compounds of formula (I) may be dispersed or dissolved in a physiologically acceptable carrier for subcutaneous injection. Alternatively, the compounds of formula (I) may be formulated into an implant for subcutaneous administration. In addition the compounds of formula (I) may be transdermally administered to animals. For parenteral administration, the dosage form chosen should provide the animal with 0.01 mg/kg to 100 mg/kg of animal body weight per day of the compound of formula (I).

The compounds of formula (I) may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds of formula (I). In addition, the compounds of formula (I) may be formulated as ear tags for animals, particularly quadrupeds such as cattle and sheep.

Suitable preparations are:
Solutions such as oral solutions, concentrates for oral administration after dilution, solutions for use on the skin or in body cavities, pouring-on formulations, gels;
Emulsions and suspensions for oral or dermal administration; semi-solid preparations;
Formulations in which the active compound is processed in an ointment base or in an oil-in-water or water-in-oil emulsion base;
Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further ingredients such as acids, bases, buffer salts, preservatives, and solubilizers. The solutions are filtered and filled sterile.

Suitable solvents are physiologically tolerable solvents such as water, alkanols such as ethanol, butanol, benzyl alcohol, glycerol, propylene glycol, polyethylene glycols, N-methyl-pyrrolidone, 2-pyrrolidone, and mixtures thereof.

The active compounds can optionally be dissolved in physiologically tolerable vegetable or synthetic oils which are suitable for injection.

Suitable solubilizers are solvents which promote the dissolution of the active compound in the main solvent or prevent its precipitation. Examples are polyvinylpyrrolidone, polyvinyl alcohol, polyoxyethylated castor oil, and polyoxyethylated sorbitan ester.

Suitable preservatives are benzyl alcohol, trichlorobutanol, p-hydroxybenzoic acid esters, and n-butanol.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on.

Solutions for use on the skin are prepared according to the state of the art and according to what is described above for injection solutions, sterile procedures not being necessary.

Further suitable solvents are polypropylene glycol, phenyl ethanol, phenoxy ethanol, ester such as ethyl or butyl acetate, benzyl benzoate, ethers such as alkyleneglycol alkylether, e.g. dipropylene glycol monomethyl ether, ketones such as acetone, methyl ethyl ketone, aromatic hydrocarbons, vegetable and synthetic oils, dimethylformamide (DMF), dimethylacetamide, transcutol, solketal, propylenecarbonate, and mixtures thereof.

It may be advantageous to add thickeners during preparation. Suitable thickeners are inorganic thickeners such as bentonites, colloidal silicic acid, aluminium monostearate, organic thickeners such as cellulose derivatives, polyvinyl alcohols and their copolymers, acrylates and methacrylates.

Gels are applied to or spread on the skin or introduced into body cavities. Gels are prepared by treating solutions which have been prepared as described in the case of the injection solutions with sufficient thickener that a clear material having an ointment-like consistency results. The thickeners employed are the thickeners given above.

Pour-on formulations are poured or sprayed onto limited areas of the skin, the active compound penetrating the skin and acting systemically.

Pour-on formulations are prepared by dissolving, suspending or emulsifying the active compound in suitable skin-compatible solvents or solvent mixtures. If appropriate, other auxiliaries such as colorants, bioabsorption-promoting substances, antioxidants, light stabilizers, adhesives are added.

Suitable solvents are water, alkanols, glycols, polyethylene glycols, polypropylene glycols, glycerol, aromatic alcohols such as benzyl alcohol, phenylethanol, phenoxyethanol, esters such as ethyl acetate, butyl acetate, benzyl benzoate, ethers such as alkylene glycol alkyl ethers such as dipropylene glycol monomethyl ether, diethylene glycol monobutyl ether, ketones such as acetone, methyl ethyl ketone, cyclic carbonates such as propylene carbonate, ethylene carbonate, aromatic and/or aliphatic hydrocarbons, vegetable or synthetic oils, DMF, dimethylacetamide, n-alkylpyrrolidones such as methylpyrrolidone, n-butylpyrrolidone or n-octylpyrrolidone, N-methylpyrrolidone, 2-pyrrolidone, 2,2-dimethyl-4-oxymethylene-1,3-dioxolane and glycerol formal.

Suitable colorants are all colorants permitted for use on animals and which can be dissolved or suspended.

Suitable absorption-promoting substances are, for example, DMSO, spreading oils such as isopropyl myristate, dipropylene glycol pelargonate, silicone oils and copolymers thereof with polyethers, fatty acid esters, triglycerides, fatty alcohols.

Suitable antioxidants are sulfites or metabisulfites such as potassium metabisulfite, ascorbic acid, butylhydroxytoluene, butylhydroxyanisole, tocopherol.

Suitable light stabilizers are, for example, novantisolic acid.

Suitable adhesives are, for example, cellulose derivatives, starch derivatives, polyacrylates, natural polymers such as alginates, gelatin.

Emulsions can be administered orally, dermally or as injections.

Emulsions are either of the water-in-oil type or of the oil-in-water type.

They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances.

Suitable hydrophobic phases (oils) are:
liquid paraffins, silicone oils, natural vegetable oils such as sesame oil, almond oil, castor oil, synthetic triglycerides such as caprylic/capric biglyceride, triglyceride mixture with vegetable fatty acids of the chain length C₈-C₁₂ or other specially selected natural fatty acids, partial glyceride mixtures of saturated or unsaturated fatty acids possibly also containing hydroxyl groups, mono- and diglycerides of the C₈-C₁₀ fatty acids,
fatty acid esters such as ethyl stearate, di-n-butyryl adipate, hexyl laurate, dipropylene glycol perlargonate, esters of a branched fatty acid of medium chain length with saturated fatty alcohols of chain length C₁₆-C₁₈, isopropyl myristate, isopropyl palmitate, caprylic/capric acid esters of saturated fatty alcohols of chain length C₁₂-C₁₈, isopropyl stearate, oleyl oleate, decyl oleate, ethyl oleate, ethyl lactate, waxy fatty acid esters such as synthetic duck coccygeal gland fat, dibutyl phthalate, diisopropyl adipate, and ester mixtures related to the latter,
fatty alcohols such as isotridecyl alcohol, 2-octyldodecanol, cetylstearyl alcohol, oleyl alcohol, and
fatty acids such as oleic acid and
mixtures thereof.

Suitable hydrophilic phases are: water, alcohols such as propylene glycol, glycerol, sorbitol and mixtures thereof.

Suitable emulsifiers are:
non-ionic surfactants, e.g. polyethoxylated castor oil, polyethoxylated sorbitan monooleate, sorbitan monostearate, glycerol monostearate, polyoxyethyl stearate, alkylphenol polyglycol ether;
ampholytic surfactants such as di-sodium N-lauryl-p-iminodipropionate or lecithin;
anionic surfactants, such as sodium lauryl sulfate, fatty alcohol ether sulfates, mono/dialkyl polyglycol ether orthophosphoric acid ester monoethanolamine salt;
cation-active surfactants, such as cetyltrimethylammonium chloride.

Suitable further auxiliaries are: substances which enhance the viscosity and stabilize the emulsion, such as carboxymethylcellulose, methylcellulose and other cellulose and starch derivatives, polyacrylates, alginates, gelatin, gum arabic, polyvinylpyrrolidone, polyvinyl alcohol, copolymers of methyl vinyl ether and maleic anhydride, polyethylene glycols, waxes, colloidal silicic acid or mixtures of the substances mentioned.

Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers.

Liquid suspending agents are all homogeneous solvents and solvent mixtures.

Suitable wetting agents (dispersants) are the emulsifiers given above.

Other auxiliaries which may be mentioned are those given above.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form.

Suitable excipients are all physiologically tolerable solid inert substances. Those used are inorganic and organic substances. Inorganic substances are, for example, sodium chloride, carbonates such as calcium carbonate, hydrogencarbonates, aluminium oxides, titanium oxide, silicic acids, argillaceous earths, precipitated or colloidal silica, or phosphates. Organic substances are, for example, sugar, cellulose, foodstuffs and feeds such as milk powder, animal meal, grain meals and shreds, starches.

Suitable auxiliaries are preservatives, antioxidants, and/or colorants which have been mentioned above.

Other suitable auxiliaries are lubricants and glidants such as magnesium stearate, stearic acid, talc, bentonites, disintegration-promoting substances such as starch or crosslinked polyvinylpyrrolidone, binders such as starch, gelatin or linear polyvinylpyrrolidone, and dry binders such as microcrystalline cellulose.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like.

The compositions which can be used in the invention can comprise generally from about 0.001 to 95% of the compound of formula (I) or of the N-oxide or salt thereof.

Generally, it is favorable to apply the compounds of formula (I) in total amounts of 0.5 mg/kg to 100 mg/kg per day, preferably 1 mg/kg to 50 mg/kg per day.

Ready-to-use preparations contain the compounds acting against parasites, preferably ectoparasites, in concentrations of 10 ppm to 80 percent by weight, preferably from 0.1 to 65 percent by weight, more preferably from 1 to 50 percent by weight, most preferably from 5 to 40 percent by weight.

Preparations which are diluted before use contain the compounds acting against ectoparasites in concentrations of 0.5 to 90 percent by weight, preferably of 1 to 50 percent by weight.

Furthermore, the preparations comprise the compounds of formula (I) against endoparasites in concentrations of 10 ppm to 2 per cent by weight, preferably of 0.05 to 0.9 percent by weight, very particularly preferably of 0.005 to 0.25 percent by weight.

In a preferred embodiment of the present invention, the compositions comprising the compounds of formula (I) are applied dermally / topically.

In a further preferred embodiment, the topical application is conducted in the form of compound-containing shaped articles such as collars, medallions, ear tags, bands for fixing at body parts, and adhesive strips and foils.

Generally, it is favorable to apply solid formulations which release compounds of formula (I) in total amounts of 10 mg/kg to 300 mg/kg, preferably 20 mg/kg to 200 mg/kg, most preferably 25 mg/kg to 160 mg/kg body weight of the treated animal in the course of three weeks.

For the preparation of the shaped articles, thermoplastic and flexible plastics as well as elastomers and thermoplastic elastomers are used. Suitable plastics and elastomers are polyvinyl resins, polyurethane, polyacrylate, epoxy resins, cellulose, cellulose derivatives, polyamides and polyester which are sufficiently compatible with the compounds of formula (I). A detailed list of plastics and elastomers as well as preparation procedures for the shaped articles is given e.g. in WO 03/086075.

Compositions to be used according to this invention may also contain other active ingredients, for example other pesticides, insecticides, herbicides, fungicides, other pesticides, or bactericides, fertilizers such as ammonium nitrate, urea, potash, and superphosphate, phytotoxicants and plant growth regulators, safeners and nematicides. These additional ingredients may be used sequentially or in combination with the above-described compositions, if appropriate also added only immediately prior to use (tank mix). For example, the plant(s) may be sprayed with a composition of this invention either before or after being treated with other active ingredients.

These agents can be admixed with the agents used according to the invention in a weight ratio of 1:10 to 10:1. Mixing the compounds (I), or the compositions comprising them in the use form as pesticides with other pesticides frequently results in a broader pesticidal spectrum of action.

The following list of pesticides together with which the compounds of formula (I) can be used, is intended to illustrate the possible combinations, but not to impose any limitation:
Organo(thio)phosphates: acephate, azamethiphos, azinphos-ethyl, azinphosmethyl, cadusafos, chlorethoxyfos, chlorfenvinphos, chlormephos, chlorpyrifos, chlorpyrifos-methyl, chlorfenvinphos, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, isoxathion, malathion, mecarbam, methamidophos, methidathion, methyl-parathion, mevinphos, monocrotophos, naled, omethoate, oxydemeton-methyl, paraoxon, parathion, parathion-methyl, phenthoate, phorate, phosalone, phosmet, phosphamidon, phorate, phoxim, pirimiphos, pirimiphos-methyl, profenofos, propetamphos, prothiofos, pyraclofos, pyridaphenthion, quinalphos, sulfotep, sulprophos, tebupirimfos, temephos, terbufos, tetrachlorvinphos, thiometon, triazophos, trichlorfon, vamidothion;
Carbamates: alanycarb, aldicarb, bendiocarb, benfuracarb, butocarboxim, butoxycarboxim, carbaryl, carbofuran, carbosulfan, ethiofoncarb, fenobucarb, fenoxycarb, formethanat, furathiocarb, isoprocarb, methiocarb, methomyl, metolcarb, oxamyl, pirimicarb, propoxur, thiodicarb, thiofanox, triazemate, trimethacarb, XMC, xylylcarb;
Pyrethroids: acrinathrin, allethrin, d-cis-trans allethrin, d-trans allethrin, bifenthrin, bioallethrin, bioallethrin S-cyclopentenyl, bioresmethrin, cycloprothrin, cyfluthrin, beta-cyfluthrin, cyhalothrin, lambda-cyhalothrin, gamma-cyhalothrin, cyphenothrin, cypermethrin, alpha-cypermethrin, beta-cypermethrin, theta-cypermethrin, zeta-cypermethrin, deltamethrin, empenthrin, esfenvalerate, etofenprox, fenpropathrin, fenvalerate, flucythrinate, flumethrin, tau-fluvalinate, halfenprox, imiprothrin, permethrin, phenothrin, prallethrin, profluthrin, pyrethrin I and II, resmethrin, RU 15525, silafluofen, tau-fluvalinate, tefluthrin, tetramethrin, tralomethrin, transfluthrin, dimefluthrin, ZXI 8901;
Growth regulators: a) chitin synthesis inhibitors: benzoylureas; bistrifluron, chlorfluazuron, diflubenzuron, flucycloxuron, flufenoxuron, hexaflumuron, lufenuron, novaluron, noviflumuron, teflubenzuron, triflumuron; buprofezin, diofenolan, hexythiazox, etoxazole, clofentezine; b) ecdysone antagonists: chlormafenozide, halofenozide, methoxyfenozide, tebufenozide, azadirachtin; c) juvenoids: pyriproxyfen, hydroprene, kinoprene, methoprene, fenoxycarb; d) lipid biosynthesis inhibitors: spirodiclofen, spiromesifen, spirotetramat;
Nicotinic receptor agonists/antagonists compounds: acetamiprid, clothianidin, dinotefuran, imidacloprid, nitenpyram, thiacloprid, thiamethoxam, nicotine, bensultap, cartap hydrochloride, thiocyclam, thiosultap-sodium;
the thiazol compound of formula (Γ¹)

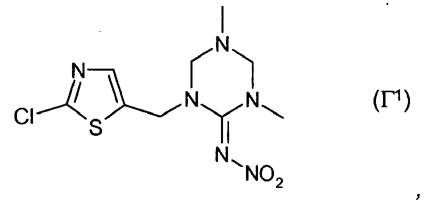

GABA antagonist compounds: acetoprole, chlordane, endosulfan, ethiprole, gamma-HCH (lindane), fipronil, vaniliprole, pyrafluprole, pyriprole, vaniliprole, the phenylpyrazole compound of formula Γ²

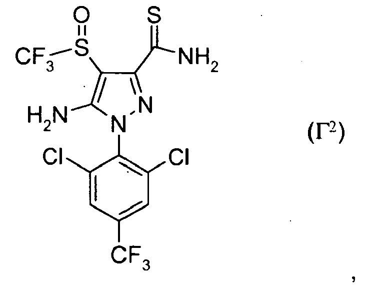

Macrocyclic lactone insecticides: abamectin, emamectin, emamectin benzoate, milbemectin, lepimectin, spinosad.

METI I compounds: fenazaquin, fenpyroximate, flufenerim, pyridaben, pyrimidifen, rotenone, tebufenpyrad, tolfenpyrad;
METI II and III compounds: acequinocyl, fluacryprim, hydramethylnon;
Uncoupler compounds: chlorfenapyr, DNOC;
Oxidative phosphorylation inhibitor compounds: azocyclotin, cyhexatin, diafenthiuron, fenbutatin oxide, propargite, tetradifon;
Moulting disruptor compounds: cyromazine;
Mixed Function Oxidase inhibitor compounds: piperonyl butoxide;
Sodium channel blocker compounds: indoxacarb, metaflumizone;
Inorganic compounds: aluminium phosphide, borax, cryolite, cyanide, sulfuryl fluoride, phosphine;
Microbial disruptors of insect midgut membranes: bacillus thuringiensis subsp. israelensis, bacillus sphaericus, bacillus thuringiensis subsp. aizawai, bacillus thuringiensis subsp. kurstaki, bacillus thuringiensis subsp. tenebrionis;
Various: amitraz, benclothiaz, benzoximat, bifenazate, bromopropylate, cartap, chinomethionat, chloropicrin, flonicamid, methyl bromide, pyridalyl, pymetrozine, rynaxypursulfur, tartar emetic, thiocyclam, tribufosflubendiamide, cyenopyrafen, flupyrazofos, cyflumetofen, amidoflumet, NNI-0101;
N-R'-2,2-dihalo-1-R"-cyclopropanecarboxamide-2-(2,6-dichloro- α,α,α -trifluoro-p-tolyl)hydrazone or N-R'-2,2-di(R"')-propionamide-2-(2,6-dichloro- α,α,α -trifluoro-p-tolyl)-hydrazone, wherein R' is methyl or ethyl, halo is chloro or bromo, R" is hydrogen or methyl and R'" is methyl or ethyl, anthranilamide compounds of formula Γ³ 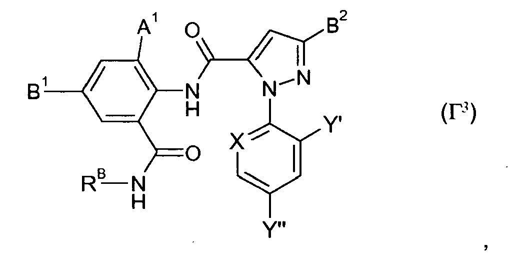 wherein A¹ is CH₃, Cl, Br, I, X is C-H, C-Cl, C-F or N, Y' is F, Cl, or Br, Y" is F, Cl, CF₃, B¹ is hydrogen, Cl, Br, I, CN, B² is Cl, Br, CF₃, OCH₂CF₃, OCF₂H, and R^{B} is hydrogen, CH₃ or CH(CH₃)₂, and malononitrile compounds as described in JP 2002 284608, WO 02/89579, WO 02/90320, WO 02/90321, WO 04/06677, WO 04/20399, or J P 2004 99597.

The compounds of formula (I) of the present invention may also be combined with a fluorinated quinazolinone compound as: 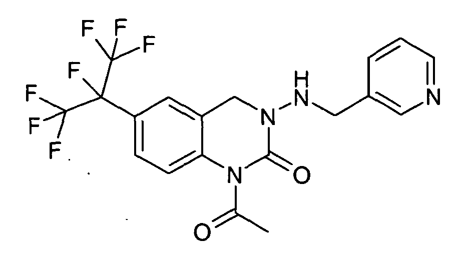 1-acetyl-3-[(pyridin-3-ylmethyl)-amino]-6-(1,2,2,2-tetrafluoro-1-trifluoromethyl-ethyl)-3,4-dihydro- 1H-quinazolin-2-one.

The compounds of formula (I) of the present invention also be combined with a pyrimidinyl alkynylether compounds Γ⁴ or thiadiazolyl alkynylether compounds Γ⁵: 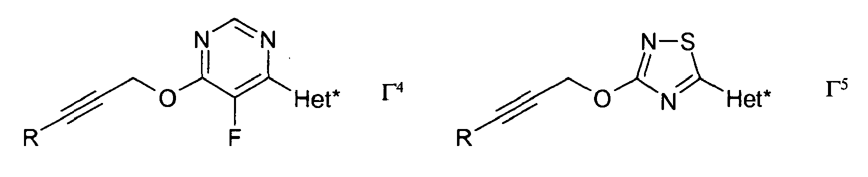 wherein R is methyl or ethyl and Het* is 3,3-dimethylpyrrolidin-1-yl, 3-methylpiperidin-1-yl, 3,5-dimethylpiperidin-1-yl, 4-methylpiperidin-1-yl, hexahydroazepin-1-yl, 2,6-dimethylhexahydroazepin-1-yl or 2,6-dimethylmorpholin-4-yl. These compounds are described e.g. in JP 2006131529.

The afore-mentioned mixing partners are commercially available and may be found in The Pesticide Manual, 13th Edition, British Crop Protection Council (2003) among other publications.

Thiamides of formula Γ² and their preparation have been described in WO 98/28279.

Lepimection is known from Agro Project, PJB Publications Ltd, November 2004. Benclothiaz and its preparation have been described in EP-A1 454621. Methidathion and Paraoxon and their preparation have been described in Farm Chemicals Handbook, Volume 88, Meister Publishing Company, 2001. Acetoprole and its preparation have been described in WO 98/28277. Metaflumizone and its preparation have been described in EP-A1 462 456. Flupyrazofos has been described in Pesticide Science 54, 1988, p.237-243 and in US 4822779. Pyrafluprole and its preparation have been described in JP 2002193709 and in WO 01/00614. Pyriprole and its preparation have been described in WO 98/45274 and in US 6335357. Amidoflumet and its preparation have been described in US 6221890 and in JP 21010907. Flufenerim and its preparation have been described in WO 03/007717 and in WO 03/007718. Cyflumetofen and its preparation have been described in WO 04/080180.

Anthranilamide compounds of formula Γ³ and their preparation have been described in WO 01/70671; WO 02/48137; WO 03/24222, WO 03/15518, WO 04/67528; WO 04/33468; and WO 05/118552.

Fungicidal mixing partners are those selected from the group consisting of
acylalanines such as benalaxyl, metalaxyl, ofurace, oxadixyl,
amine derivatives such as aldimorph, dodine, dodemorph, fenpropimorph, fenpropidin, guazatine, iminoctadine, spiroxamin, tridemorph,
anilinopyrimidines such as pyrimethanil, mepanipyrim or cyrodinyl,
antibiotics such as cycloheximid, griseofulvin, kasugamycin, natamycin, polyoxin or streptomycin,
azoles such as bitertanol, bromoconazole, cyproconazole, difenoconazole, diniconazole, epoxiconazole, fenbuconazole, fluquiconazole, flusilazole, hexaconazole, imazalil, metconazole, myclobutanil, penconazole, propiconazole, prochloraz, prothioconazole, tebuconazole, triadimefon, triadimenol, triflumizol, triticonazole, flutriafol,
dicarboximides such as iprodion, myclozolin, procymidon, vinclozolin,
dithiocarbamates such as ferbam, nabam, maneb, mancozeb, metam, metiram, propineb, polycarbamate, thiram, ziram, zineb,
heterocyclic compounds such as anilazine, benomyl, boscalid, carbendazim, carboxin, oxycarboxin, cyazofamid, dazomet, dithianon, famoxadon, fenamidon, fenarimol, fuberidazole, flutolanil, furametpyr, isoprothiolane, mepronil, nuarimol, probenazole, proquinazid, pyrifenox, pyroquilon, quinoxyfen, silthiofam, thiabendazole, thifluzamid, thiophanate-methyl, tiadinil, tricyclazole, triforine,
copper fungicides such as Bordeaux mixture, copper acetate, copper oxychloride, basic copper sulfate,
nitrophenyl derivatives such as binapacryl, dinocap, dinobuton, nitrophthalisopropyl,
phenylpyrroles such as fenpiclonil or fludioxonil,
sulfur,
other fungicides such as acibenzolar-S-methyl, benthiavalicarb, carpropamid, chlorothalonil, cyflufenamid, cymoxanil, diclomezin, diclocymet, diethofencarb, edifenphos, ethaboxam, fenhexamid, fentin-acetate, fenoxanil, ferimzone, fluazinam, fosetyl, fosetyl-aluminum, iprovalicarb, hexachlorobenzene, metrafenon, pencycuron, propamocarb, phthalide, toloclofos-methyl, quintozene, zoxamid,
strobilurins such as azoxystrobin, dimoxystrobin, fluoxastrobin, kresoxim-methyl, metominostrobin, orysastrobin, picoxystrobin or trifloxystrobin,
sulfenic acid derivatives such as captafol, captan, dichlofluanid, folpet, tolylfluanid,
cinnemamides and analogs such as dimethomorph, flumetover or flumorph.

The animal pest, i.e. arthropodes and nematodes, the plant, soil or water in which the plant is growing can be contacted with the present compound(s) (I), the N-oxides or salts thereof or composition(s) containing them by any application method known in the art. As such, "contacting" includes both direct contact (applying the compounds/compositions directly on the animal pest or plant - typically to the foliage, stem or roots of the plant) and indirect contact (applying the compounds/compositions to the locus of the animal pest or plant).

Moreover, animal pests may be controlled by contacting the target pest, its food supply, habitat, breeding ground or its locus with a pesticidally effective amount of compounds of formula (I), the N-oxides or salts thereof. As such, the application may be carried out before or after the infection of the locus, growing crops, or harvested crops by the pest.

"Locus" means a habitat, breeding ground, plant, seed, soil, area, material or environment in which a pest or parasite is growing or may grow.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

The compounds of formula (I) the N-oxides or salts thereof and compositions comprising them can be used for protecting wooden materials such as trees, board fences, sleepers, etc. and buildings such as houses, outhouses, factories, but also construction materials, furniture, leathers, fibers, vinyl articles, electric wires and cables etc. from ants and/or termites, and for controlling ants and termites from doing harm to crops or human being (e.g. when the pests invade into houses and public facilities). The compounds of formula (I) are applied not only to the surrounding soil surface or into the under-floor soil in order to protect wooden materials but it can also be applied to lumbered articles such as surfaces of the under-floor concrete, alcove posts, beams, plywoods, furniture, etc., wooden articles such as particle boards, half boards, etc. and vinyl articles such as coated electric wires, vinyl sheets, heat insulating material such as styrene foams, etc. In case of application against ants doing harm to crops or human beings, the ant controller of the present invention is applied to the crops or the surrounding soil, or is directly applied to the nest of ants or the like.

The compounds of the invention can also be applied preventively to places at which occurrence of the pests is expected.

The compounds of formula (I), the N-oxides or salts thereof may be also used to protect growing plants from attack or infestation by pests by contacting the plant with a pesticidally effective amount of compounds of formula (I). As such, "contacting" includes both direct contact (applying the compounds/compositions directly on the pest and/or plant - typically to the foliage, stem or roots of the plant) and indirect contact (applying the compounds/compositions to the locus of the pest and/or plant).

In the case of soil treatment or of application to the pests dwelling place or nest, the quantity of active ingredient ranges from 0.0001 to 500 g per 100 m², preferably from 0.001 to 20 g per 100 m².

Customary application rates in the protection of materials are, for example, from 0.01 g to 1000 g of active compound per m² treated material, desirably from 0.1 g to 50 g per m².

Insecticidal compositions for use in the impregnation of materials typically contain from 0.001 to 95 % by weight, preferably from 0.1 to 45% by weight, and more preferably from 1 to 25% by weight of at least one repellent and / or insecticide.

For use in bait compositions, the typical content of active ingredient is from 0.001 % by weight to 15% by weight, desirably from 0.001 % by weight to 5% by weight of active compound.

For use in spray compositions, the content of active ingredient is from 0.001 to 80% by weight, preferably from 0.01 to 50% by weight and most preferably from 0.01 to 15% by weight.

For use in treating crop plants, the rate of application of the active ingredients of this invention may be in the range of 0.1 g to 4000 g per hectare, desirably from 25 g to 600 g per hectare, more desirably from 50 g to 500 g per hectare.

In the treatment of seed, the application rates of the mixture are generally from 0.1 g to 10 kg per 100 kg of seed, preferably from 1 g to 5 kg per 100 kg of seed, in particular from 1 g to 200 g per 100 kg of seed.

The present invention is now illustrated in further detail by the following examples.

### I. Experimental procedures

With due modification of the starting compounds, the protocols shown in the synthesis example below were used for obtaining further compounds (I). The resulting compounds, together with physical data, are listed below in the table C.

The products were characterized by coupled High Performance Liquid Chromatography/ mass spectrometry (HPLC/MS), by ¹H-NMR (400 MHz) in CDCl₃ or d₆-DMSO or by their melting points. HPLC column: RP-18 column (Chromolith Speed ROD from Merck KgaA, Germany). Elution: acetonitrile + 0.1 % trifluoroacetic acid (TFA) / water in a ratio of from 5:95 to 95:5 in 5 minutes at 40°C. MS: Quadrupol electrospray ionisation, 80 V (positiv modus).

### I.1.1 Preparation of bromosulfonamides (II.B)

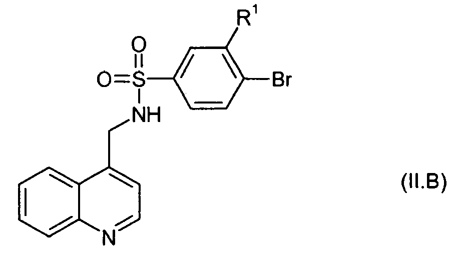

### I.1.1.a) Preparation of 4-bromo-3-fluoro-N-[(quinolin-4-yl)methyl]benzenesulfonamide (R¹ = F)

A solution of quinolinemethylamine (61 g, 0.023 mol) in triethylamine (3.81 ml, 0.027 mol) was added a solution of 3-fluoro-4-bromo-sulphonic acid chloride (6.24 g, 0.023 mol). The mixture was allowed to stir at ambient temperature for 16 h. Water (20 ml) was added; the precipitate was filtered off, rinsed subsequently with water and methyl-tert-butylether and dried to yield the title compound (4.92 g) as a colourless solid. ¹H-NMR (400 MHz, d₆-DMSO): δ = 8.8 (m, 1 h), 8.6 (m, 1 h), 8.1-7.8 (m, 3 H), 7.8.-7.4 (m, 5 h), 4.6 ppm (s, 2 H).

### I.1.1.b) Preparation of 4-bromo-3-trifluoromethyl-N-[(quinolin-4-yl)methyl]-benzene-sulfonamide (R¹ = CF₃)

This compound has been prepared using a method in analogy to the method described under 1.1.1.a). ¹H-NMR (400 MHz, d₆-DMSO): δ = 8.8 (m, 2 H), 8.1 (m, 8 H), 4.6 ppm (s, 2 H).

### I.1.1.c) Preparation of 4-bromo-3-methyl-N-[(quinolin-4-yl)methyl]benzenesulfonamide (R¹ = CH₃)

This compound has been prepared using a method in analogy to the method described under 1.1.1.a). ¹H-NMR (400 MHz, d₆-DMSO): δ = 8.8 (m, 1 H), 8.5 (t, 1 H), 8.2-8.0 (m, 2 H), 7.8-7.5 (m, 6 H), 4.6 (d, 2 H), 2.4 ppm (s, 3 H).

### I.1.1.d) Preparation of 4-bromo-3-chloro-N-[(quinolin-4-yl)methyl]benzenesulfonamide (R¹ = Cl)

This compound has been prepared using a method in analogy to the method described under 1.1.1.a). ¹H-NMR (400 MHz, d₆-DMSO): δ = 8.8 (m, 1 H), 8.6 (t, 1 H), 8.1-7.6 (m, 8 H), 4.6 ppm (d, 2 H).

### I.1.2 Preparation of bromosulfonamides (II.B*)

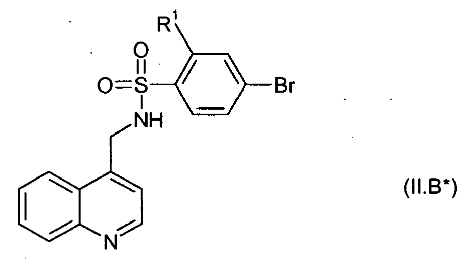

### I.1.2.a) Preparation of 4-bromo-2-fluoro-N[(quinolin-4-yl)methyl]-benzenesulfonamide (R¹ = F)

This compound has been prepared using a method in analogy to the method described under 1.1.1.a). ¹H-NMR (400 MHz, d₈-THF) δ = 8.6 (m, 1 H), 8.1 (m, 1 H), 8.0 (m, 1 H), 7.6-7.4 (m, 5 H), 7.2 (m, 2 H), 4.5 (d, 2 H).

### I.1.2.b) Preparation of 4-bromo-2-methyl-N[(quinolin-4-yl)methyl]-benzenesulfonamide (R¹ = CH₃)

This compound has been prepared using a method in analogy to the method described under 1.1.1.a). ¹H-NMR (400 MHz, d₆-DMSO) δ = 8.8 (m, 1 H), 8.6 (m, 1 H), 8.1-8.0 (m, 2 H), 7.8-7.4 (m, 6 H), 7.2 (m, 2 H), 4.5 (d, 2 H), 2.5 (s, 3H).

### I.1.2.c) Preparation of 4-bromo-2-trifluoromethoxy-N[(quinolin-4-yl)methyl]-benzenesulfonamide (R¹= OCF₃)

This compound has been prepared using a method in analogy to the method described under 1.1.1.a).¹H-NMR (400 MHz, d₆-DMSO) δ = 8.8 (m, 1 H), 8.1-8.0 (m, 2 H), 7.8-7.4 (m, 7 H), 7.2 (m, 2 H), 4.5 (d, 2 H).

### I.1.2.d) Preparation of 4-bromo-2-chloro-N[(quinolin-4-yl)methyl]benzenesulfonamide (R¹ = Cl)

This compound has been prepared using a method in analogy to the method described under 1.1.1.a). ¹H-NMR (400 Mhz, d₆-DMSO) δ = 8.8 (br, 2 H), 8.1-7.9 (m, 2 H), 7.8-7.4 (m, 6 H), 7.2 (m, 2 H), 4.6 (d, 2 H).

### 1.1.2.e) Preparation of 4-bromo-2-trifluoromethyl-N[(quinolin-4-yl)methyl]-benzenesulfonamide (R¹= CF₃)

This compound has been prepared using a method in analogy to the method described under 1.1.1.a).¹H-NMR (400 Mhz, d₆-DMSO) δ = 8.8 (m, 2 H), 8.2-7.6 (m, 5 H), 7.7 (m, 1 H),7.6 (m, 1 H), 7.4 (m, 1 H), 4.5 (d, 2 H).

### 1.2 General procedure for the preparation of compounds (I)

A mixture of a suitable sulfonamide of general formula (II) (0.48 mmol) as described above, Cs₂CO₃ (200 mg), polystyrene-triphenylphosphine-Pd (400 mg, Argonaut) and boronic acid (0.6 mmol) in tetrahydrofuran (3 ml) and water (0.5 ml) was reacted at 75°C. After 20 h the mixture was cooled to ambient temperature and treated with Polystyrene-diethanolamine (100 mg, Novabiochem) for 6 h. The immobilized reactants were filtered off and rinsed with tetrahydrofuran (10 ml). The filtrate was collected and the volatiles were removed in vacuo. The residue was subjected to column chromatography (SiO₂, cyclohexane/ethyl acetate).

Analytical data of representative compounds are given in the table C and in the table D.

According to the procedure described above compounds of formula (I.B) listed in table C have been prepared.

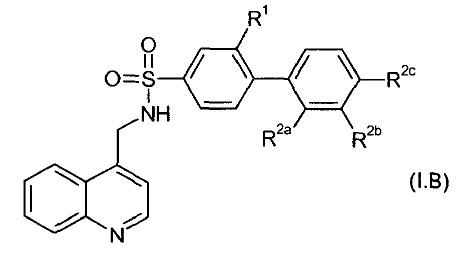

**Table C**

| Compound | R¹ | R^{2a} | R^{2b} | R^{2c} | MS (m/e [M⁺]) | HPLC ^{a)} or ¹H-NMR ^{b)} |
|---|---|---|---|---|---|---|
| B1 | CH₃ | Cl | H | Cl | 456.9 | 3.20 |
| B2 | CH₃ | H | CH₃ | H | 402.5 | 2.91 |
| B3 | CH₃ | H | H | CF₃ | 457.4 | 3.13 |
| B4 | CH₃ | H | F | H | 406.5 | 2.77 |
| B5 | CH₃ | Cl | H | H | 422.9 | 2.86 |
| B6 | CH₃ | F | H | H | 406.5 | 2.68 |
| B7 | CH₃ | H | H | OCF₃ | 473.4 | 3.21 |
| B8 | Cl | H | F | H | 426.9 | 2.81 |
| B9 | F | Cl | H | Cl | 461.3 | 3.08 |
| B10 | F | H | CH₃ | H | 406.5 | 2.88 |
| B11 | F | H | H | CF₃ | 460.4 | 3.07 |
| B12 | F | H | F | H | 410.4 | 2.76 |
| B13 | F | F | H | H | 410.4 | 2.70 |
| B14 | F | H | H | OCF₃ | 476.4 | 3.15 |
| B15 | CF₃ | H | CH₃ | H | 456.5 | 3.06 |
| B16 | CF₃ | H | H | CF₃ | 510.5 | 3.23 |
| B17 | CF₃ | H | F | H | 460.5 | 2.96 |
| B18 | CF₃ | H | H | OCF₃ | 526.5 | 3.32 |
| B19 | CF₃ | H | H | CN | 467.5 | 2.76 |
| B20 | CF₃ | H | H | O-CF=CF₂ | 538.5 | 3.36 |
| B21 | Cl | CF₃ | H | CF₃ | n.d. ^{c)} | 8.8 m, 8.2 m, 8.1 m, |
| | | | | | | 8.0 m, 7.9 m, |
| | | | | | | 7.8-7.7 m, 7.6 m, |
| | | | | | | 7.4m,7.2m,4.8d |
| B22 | Cl | Cl | H | Cl | n.d. ^{c)} | 8.8 m, 8.1 m, 8.1 m, |
| | | | | | | 7.9 m, 7.8 m, 7.6 m, |
| | | | | | | 7.5 m, 7.4 m, 7.2 m, |
| | | | | | | 7.1 m,5.2t,4.8d |
| B23 | Cl | Cl | H | CF₃ | n.d. ^{c)} | 8.8 m, 8.1 m, 8.1 m, |
| | | | | | | 7.9 m, 7.8 m, 7.6 m, |
| | | | | | | 7.5 m, 7.4 m, 7.2 m, |
| | | | | | | 7.1 m,5.2t,4.8d |
| B24 | Cl | H | H | CF₃ | n.d. ^{c)} | 8.8 m, 8.2 m, 8.0 m, |
| | | | | | | 7.8-7.6 m, 7.5 m, |
| | | | | | | 7.4 m, 7.3 m, 7.2 m, |
| | | | | | | 5.2t,4.8d |
| B25 | Cl | H | H | OCH₃ | n.d. ^{c)} | 8.8 m, 8.2 m, 7.9 m, |
| | | | | | | 7.9-7.6 m; 7.3 m, |
| | | | | | | 7.1 m,7.0m,5.2t, |
| | | | | | | 4.8 d |
| B26 | Cl | F | H | H | n.d. ^{c)} | 8.8 m, 8.2 m, 7.9 m, |
| | | | | | | 7.7 m, 7.6 m, |
| | | | | | | 7.5-7.1 m,5.2t,4.8d |
| B27 | Cl | CH₃ | H | OCH₃ | n.d. ^{c)} | 8.8 m, 8.2 m, 8.0 m, |
| | | | | | | 7.7 m , 7.6 m, 7.3 m, |
| | | | | | | 7.2 m, 7.0 m, 6.8 m, |
| | | | | | | 5.2 t, 4.8 d |
| B28 | CH₃ | H | Cl | H | 422.9 | 2.94 |
| B29 | CH₃ | F | H | Br | 485.4 | 3.04 |
| B30 | CH₃ | H | H | CN | 414.0 | 2.6 |
| B31 | CH₃ | F | H | Cl | 440.9 | 2.97 |
| B32 | CH₃ | Cl | H | CF₃ | 490.9 | 3.19 |
| B33 | CH₃ | H | CF₃ | Cl | 490.9 | 3.23 |
| B34 | CH₃ | F | H | OCH₃ | 436.5 | 2.34 |
| B35 | Cl | H | Cl | H | 443.4 | 3.00 |
| B36 | Cl | H | H | CN | 433.9 | 2.63 |
| B37 | Cl | F | H | Cl | 461.3 | 3.03 |
| B38 | Cl | H | CF₃ | Cl | 511.3 | 3.31 |
| B39 | Cl | F | H | OCH₃ | 456.9 | 2.52 |
| B40 | F | H | Cl | H | 426.9 | 2.94 |
| B41 | F | H | H | CN | 417.5 | 2.55 |
| B42 | F | F | H | Cl | 444.9 | 2.97 |
| B43 | F | Cl | H | CF₃ | 494.9 | 3.19 |
| B44 | F | H | CF₃ | Cl | 494.9 | 3.23 |
| B45 | F | F | H | OCH₃ | 440.5 | 2.41 |
| B46 | CH₃ | H | H | O-CF=CF₂ | 484.5 | 3.22 |
| B47 | Cl | H | H | O-CF=CF₂ | 504.9 | 3.26 |
| B48 | F | H | H | O-CF=CF₂ | 488.5 | 3.20 |
| B49 | Cl | F | H | Br | 5058 | 3.11 |
| B50 | CF₃ | H | Cl | H | 476.9 | 3.13 |
| B51 | CF₃ | F | H | H | 460.4 | 2.88 |
| B52 | CF₃ | F | H | Cl | 494.9 | 3.15 |
| B53 | CF₃ | H | CF₃ | Cl | 544.9 | 3.40 |

| | | | | | | |
|---|---|---|---|---|---|---|
| a) retention time for the method described above b) δ [ppm], (CDCl₃,400 MHz) c) not determined | | | | | | |

According to the procedure described above compounds of formula (I.B*) listed in table D have been prepared.

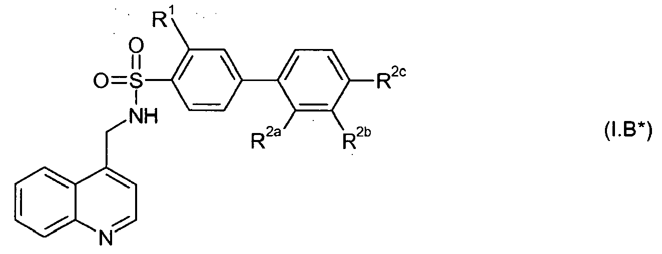

**Table D**

| Compound | R¹ | R^{2a} | R^{2b} | R^{2c} | MS (m/e [M⁺]) | HPLC ^{a)} or ¹H-NMR ^{b)} |
|---|---|---|---|---|---|---|
| B54 | OCF₃ | H | H | Cl | 492.9 | 3.16 |
| B55 | CF₃ | H | H | Cl | 476.9 | 3.1 |
| B56 | CH₃ | H | H | Cl | 422.9 | 2.93 |
| B57 | F | H | H | Cl | 426.9 | 2.86 |
| B58 | OCF₃ | H | H | CF₃ | 526.9 | 3.25 |
| B59 | CF₃ | H | H | CF₃ | 510.5 | 3.21 |
| B60 | CH₃ | H | H | CF₃ | 456.6 | 3.06 |
| B61 | Cl | H | H | CF₃ | 476.9 | 3.04 |
| B62 | F | H | H | CF₃ | 460.4 | 2.96 |
| B63 | CF₃ | H | H | CN | 467.5 | 2.70 |
| B64 | CH₃ | H | H | CN | 413.2 | 2.52 |
| B65 | Cl | H | H | CN | 433.9 | 2.48 |
| B66 | OCF₃ | H | H | OCF₃ | 542.5 | 3.31 |
| B67 | CF₃ | H | H | OCF₃ | 526.5 | 3.27 |
| B68 | CH₃ | H | H | OCF₃ | 472.5 | 3.12 |
| B69 | Cl | H | H | OCF₃ | 492.9 | 3.09 |
| B70 | F | H | H | OCF₃ | 476.4 | 3.04 |
| B71 | OCF₃ | Cl | H | Cl | 527.3 | n.d. ^{c)} |
| B72 | CF₃ | Cl | H | Cl | 511.3 | n.d. ^{c)} |
| B73 | CH₃ | Cl | H | Cl | 457.4 | n.d. ^{c)} |
| B74 | Cl | Cl | H | Cl | 477.8 | n.d. ^{c)} |
| B75 | F | Cl | H | Cl | 461.3 | n.d. ^{c)} |
| B76 | OCF₃ | Cl | H | Cl | 594.5 | n.d. ^{c)} |
| B77 | CF₃ | CF₃ | H | CF₃ | 578.5 | n.d. ^{c)} |
| B78 | CH₃ | CF₃ | H | CF₃ | 524.5 | 3.33 |
| B79 | Cl | CF3 | H | CF₃ | 544.9 | n.d. ^{c)} |
| B80 | F | CF₃ | H | CF₃ | 528.4 | n.d. ^{c)} |
| B81 | OCF₃ | Cl | H | CF₃ | 560.9 | 3.52 |
| B82 | CF₃ | Cl | H | CF₃ | 544.9 | n.d. ^{c)} |
| B83 | CH₃ | Cl | H | CF₃ | 490.9 | n.d. ^{c)} |
| B84 | Cl | Cl | H | CF₃ | 511.3 | 3.27 |
| B85 | F | Cl | H | CF₃ | 494.9 | 3.24 |
| B86 | OCF₃ | CF₃ | H | H | 526.4 | n.d. ^{c)} |
| B87 | CF₃ | CF₃ | H | H | 510.5 | 3.14 |
| B88 | CH₃ | CF₃ | H | H | 456.5 | 3.04 |
| B89 | Cl | CF₃ | H | H | 476.9 | n.d. ^{c)} |
| B90 | F | CF₃ | H | H | 460.4 | 2.95 |
| B91 | OCF₃ | Cl | H | H | 492.9 | 3.16 |
| B92 | CF₃ | Cl | H | H | 476.9 | 3.08 |
| B93 | CH₃ | Cl | H | H | 422.9 | n.d. ^{c)} |
| B94 | Cl | Cl | H | H | 443.4 | n.d. ^{c)} |
| B95 | F | Cl | H | H | 426.9 | 2.88 |
| B96 | OCF₃ | CF₃ | H | Cl | 560.9 | 3.44 |
| B97 | CF₃ | CF₃ | H | Cl | 544.9 | 3.38 |
| B98 | CH₃ | CF₃ | H | Cl | 490.9 | 3.3 |
| B99 | Cl | CF₃ | H | Cl | 511.3 | 3.3 |
| B100 | F | CF₃ | H | Cl | 494.9 | 3.21 |
| B101 | OCF₃ | F | H | F | 494.9 | 3.1 |
| B102 | CF₃ | F | H | F | 478.4 | 3.05 |
| B103 | CH₃ | F | H | F | 424.5 | 2.85 |
| B104 | Cl | F | H | F | 444.9 | 2.83 |
| B105 | F | F | H | F | 428.4 | n.d. ^{c)} |
| B106 | OCF₃ | F | H | H | 476.4 | 3.06 |
| B107 | CF₃ | F | H | H | 460.4 | 3.01 |
| B108 | CH₃ | F | H | H | 406.5 | n.d. ^{c)} |
| B109 | Cl | F | H | H | 426.9 | 2.74 |
| B110 | F | F | H | H | 410.4 | 2.74 |
| B111 | OCF₃ | H | H | CN | 483.5 | 2.78 |
| B112 | CH₃ | H | H | CN | 413.2 | 2.56 |
| B113 | OCF₃ | Cl | H | Cl | 527.3 | n.d. ^{c)} |
| B114 | CF₃ | Cl | H | Cl | 511.3 | 3.34 |
| B115 | CH₃ | Cl | H | Cl | 457.4 | 3.17 |
| B116 | Cl | Cl | H | Cl | 477.8 | 3.18 |
| B117 | F | Cl | H | Cl | 461.3 | 3.10 |
| B118 | CF₃ | Cl | H | CF₃ | 544.9 | n.d. ^{c)} |
| B119 | CH₃ | Cl | H | CF₃ | 490.93 | n.d. ^{c)} |
| B120 | Cl | CF₃ | H | H | 476.9 | 2.96 |
| B121 | CH₃ | Cl | H | H | 422.9 | 2.88 |
| B122 | Cl | Cl | H | H | 443.4 | 2.88 |
| B123 | F | F | H | F | 428.4 | 2.73 |
| B124 | CH₃ | F | H | H | 406.5 | 2.75 |

| | | | | | | |
|---|---|---|---|---|---|---|
| a) retention time for the method described above b) δ [ppm], (CDCl₃,400 MHz) c) not determined | | | | | | |

In analogues way comparative examples of formula (I.B) listed in table E have been prepared.

**Table E**

| Compound | R¹ | R^{2a} | R^{2b} | R^{2c} | HPLC, ¹H-NMR |
|---|---|---|---|---|---|
| C1 | H | H | H | OCF₃ | n.d. ^{c)} |
| C2 | H | H | H | CF₃ | n.d. ^{c)} |

| | | | | | |
|---|---|---|---|---|---|
| c) not determined | | | | | |

### II. Assessment of the biological activity

### II.1 Activity against cotton aphid (aphis gossypii), mixed life stages

The active compounds were formulated in 50:50 acetone:water and 100 ppm Kinetic^{®} surfactant.

Cotton plants at the cotyledon stage were infested prior to treatment by placing a heavily infested leaf from the main aphid colony on top of each cotyledon. The aphids were allowed to transfer overnight and the host leaf was removed. The infested cotyledons were then dipped and agitated in the test solution for 3 seconds and allowed to dry in a fume hood. Test plants were maintained under fluorescent lighting in a 24 h photoperiod at 25°C and 20-40% relative humidity. Aphid mortality on the treated plants, relative to mortality on untreated check plants, was determined after 5 days.

In this test, the compounds B1, B3, B7, B12, B14, B27, B45, B56, B59, B85, B90, B115, B117 and B123 at 300 ppm showed a mortality of at least 75% in comparison with untreated controls.

### II.2 Activity against green peach aphid (Myzus persicae)

The active compounds were formulated in 50:50 acetone:water and 100 ppm Kinetic^{™} surfactant.

Pepper plants in the 2^{nd} leaf-pair stage (variety California Wonder) were infested with approximately 40 laboratory-reared aphids by placing infested leaf sections on top of the test plants. The leaf sections were removed after 24 hr. The leaves of the intact plants were dipped into gradient solutions of the test compound and allowed to dry. Test plants were maintained under fluorescent light (24 hour photoperiod) at about 25°C and 20-40% relative humidity. Aphid mortality on the treated plants, relative to mortality on check plants, was determined after 5 days.

In this test, the compounds B1, B3, B9, B14, B27, B31, B56 and B85 at 300 ppm showed a mortality of at least 75% in comparison with untreated controls.

### II.3 Activity against southern armyworm (spodoptera eridania), 2^{nd}-3^{rd} instar larvae

The active compounds were formulated as a 10.000 ppm solution in a mixture of 35% acetone and water, which was diluted with water, if needed.

Sieva lima bean foliage, expanded to the first true leaves, were dipped and agitated in the test solution for 3 seconds and then allowed to dry in a fume hood. The treated plant was then placed in 25-cm plastic perforated zip enclosure bags, ten 2^{nd}-instar larvae were added, and the bags sealed. After 4 days, observations were made of mortality, plant feeding, and of any interference with larval growth.

In this test, the compounds B1, B3, B8, B10, B12, B26, B31, B32, B45, B60, B84, B90, B98 and B103 at 300 ppm showed a mortality of at least 75% in comparison with untreated controls.

### II.4 Activity against Colorado potato beetle (Leptinotarsa decemlineata)

Potato plants were utilized for bioassays. Excised plant leaves were dipped into 1:1 acetone/water dilutions of the active compounds. After the leaves had dried, they were individually placed onto water-moistened filter paper on the bottoms of Petri dishes. Each dish was infested with 5-7 larvae and covered with a lid. Each treatment dilution was replicated 4 times. Test dishes were held at approximately 27°C and 60% humidity. Numbers of live and morbid larvae were assessed in each dish at 5 days after treatment application, and percent mortality was calculated.

In this test, the compounds B2, B16, B32, B68, B84, B121 and B122 at 2500 ppm showed a mortality of at least 75% in comparison with untreated controls.

### II.5 Activity against diamond back moth (plutella xylostella)

The active compounds were formulated in 50:50 acetone:water and 0.1 % (vol/vol) Alkamuls EL 620 surfactant. A 6 cm leaf disk of cabbage leaves was dipped in the test solution for 3 seconds and allowed to air dry in a Petri plate lined with moist filter paper. The leaf disk was inoculated with 10 third instar larvae and kept at 25-27°C and 50-60% humidity for 3 days. Mortality was assessed after 72 h of treatment.

In this test, the compounds B1, B3, B12, B23, B27 and B115 at 300 ppm showed a mortality of at least 75% in comparison with untreated controls.

### II.6 Activity against two-spotted spider mite (tetranychus urticae, OP-resistant strain)

The active compounds are formulated in 50:50 acetone:water and 100 ppm Kinetic^{™} surfactant.

Sieva lima bean plants with primary leaves expanded to 7-12 cm are infested by placing on each a small piece from an infested leaf (with about 100 mites) taken from the main colony. This is done at about 2 hours before treatment to allow the mites to move over to the test plant to lay eggs. The piece of leaf used to transfer the mites is removed. The newly-infested plants are dipped in the test solution and allowed to dry. The test plants are kept under fluorescent light (24 hour photoperiod) at about 25°C and 20-40% relative humidity. After 5 days, one leaf is removed and mortality counts are made.

In this test, the compounds B1 and B7 at 300 ppm showed a mortality of at least 75% in comparison with untreated controls.

### II.7 Activity against brown planthopper (nilaparvata lugens)

The active compounds were formulated as a 20:80 acetone:water solution. Surfactant (Alkamuls EL 620) was added at the rate of 0.1 % (vol/vol).

Potted rice plants of 3-4 weeks of age are sprayed with 10 ml of the test solution using air driven hand atomizer (Devillbis atomizer) at 1.7 bar. The treated plants are allowed to dry for about 1 hour and covered with Mylar cages. The plants are inoculated with 10 adults of the specie (5 male and 5 females) and kept at 25-27°C and 50-60% humidity for 3 days. Mortality is assed after 24, 48 and 72 hours after treatment. Dead insects are usually found in the water surface. Each treatment is replicated once.

In this test, compounds B16 at 300 ppm showed a mortality of at least 75% in comparison with untreated controls.

### II.8 Activity against Boll weevil (Anthonomus grandis)

The active compounds were formulated in 1:3 DMSO:water. 10 to 15 eggs were placed into microtiterplates filled with 2% agar-agar in water and 300 ppm formaline. The eggs were sprayed with 20 µl of the test solution, the plates were sealed with pierced foils and kept at 24-26°C and 75-85% humidity with a day/night cycle for 3 to 5 days. Mortality was assessed on the basis of the remaining unhatched eggs or larvae on the agar surface and/or quantity and depth of the digging channels caused by the hatched larvae. Tests were replicated 2 times.

In this test, compounds B68, B116 and B117 at 2500 ppm showed a mortality of at least 75% in comparison with untreated controls.

### II.9 Activity against Mediterranean fruitfly (Ceratitis capitata)

The active compounds were formulated in 1:3 DMSO:water. 50 to 80 eggs were placed into microtiterplates filled with 0.5% agar-agar and 14% diet in water. The eggs were sprayed with 5 µl of the test solution, the plates were sealed with pierced foils and kept at 27-29°C and 75-85% humidity under fluorescent light for 6 days. Mortality was assessed on the basis of the agility of the hatched larvae. Tests were replicated 2 times.

In this test, compounds B85 and B98 at 2500 ppm showed at least 75% mortality.

### II.10 Activity against Tobacco budworm (Heliothis virescens)

The active compounds were formulated in 1:3 DMSO:water. 15 to 25 eggs were placed into microtiterplates filled with diet. The eggs were sprayed with 10 µl of the test solution, the plates were sealed with pierced foils and kept at 27-29°C and 75-85% humidity under fluorescent light for 6 days. Mortality was assessed on the basis of the agility and of comparative feeding of the hatched larvae. Tests were replicated 2 times.

In this test, compounds at 2500 ppm showed at least 75% mortality.

### II.11 Activity against Vetch aphid (Megoura viciae)

The active compounds were formulated in 1:3 DMSO:water. Bean leaf disks were placed into microtiterplates filled with 0.8% agar-agar and 2.5 ppm OPUS^{™}. The leaf disks were sprayed with 2.5 µl of the test solution and 5 to 8 adult aphids were placed into the microtiterplates which were then closed and kept at 22-24°C and 35-45% under fluorescent light for 6 days. Mortality was assessed on the basis of vital, reproduced aphids. Tests were replicated 2 times.

In this test, compound at 2500 ppm showed at least 75% mortality compared to 0% mortality of untreated controls.

### II.12 Activity against tobacco budworm (Heliothis virescens)

Two-leaf cotton plants are utilized for bioassays. Excised plant leaves are dipped into 1:1 acetone/water dilutions of the active compounds. After the leaves have dried, they are individually placed onto water-moistened filter paper on the bottoms of Petri dishes. Each dish is infested with 5-7 larvae and covered with a lid. Each treatment dilution is replicated 4 times. Test dishes are held at approximately 27°C and 60% humidity. Numbers of live and morbid larvae are assessed in each dish at 5 days after treatment application, and percent mortality is calculated.

### II.13 Activity against silverleaf whitefly (bemisia argentifolii)

The active compounds were formulated in 50:50 acetone:water and 100 ppm Kinetic^{™} surfactant.

Selected cotton plants were grown to the cotyledon state (one plant per pot). The cotyledons were dipped into the test solution to provide complete coverage of the foliage and placed in a well-vented area to dry. Each pot with treated seedling was placed in a plastic cup and 10 to 12 whitefly adults (approximately 3-5 day old) were introduced. The insects were colleted using an aspirator and an 0.6 cm, non-toxic Tygon tubing connected to a barrier pipette tip. The tip, containing the collected insects, was then gently inserted into the soil containing the treated plant, allowing insects to crawl out of the tip to reach the foliage for feeding. The cups were covered with a re-usable screened lid (150 micron mesh polyester screen PeCap from Tetko Inc). Test plants were maintained in the holding room at about 25°C and 20-40% relative humidity for 3 days avoiding direct exposure to the fluorescent light (24 hour photoperiod) to prevent trapping of heat inside the cup. Mortality was assessed 3 days after treatment of the plants.

In this test, the compounds B56, B84, B85, B115 and B117 at 300 ppm showed a mortality of at least 70% in comparison with untreated controls.

### II.14 Activity against cowpea aphid (aphis craccivora)

The active compounds were formulated in 50:50 acetone:water. Potted cowpea plants colonized with 100-150 aphids of various stages were sprayed after the pest population has been recorded. Population reduction was recorded after 24, 72, and 120 hours.

In this test, the compounds at 300 ppm showed a mortality of at least 80% in comparison with untreated controls.

### II.15 Activity against yellowfever mosquitos (Aedes aegypti)

The test compound (1 vol% in acetone) was applied to water in glass dishes containing 4th instar aedes aegypti. The test dishes were maintained at about 25°C and observed daily for mortality. Each test was replicated in 3 test dishes.

In this test, the compounds at 300 ppm after 6 days showed over 90% mortality compared to untreated controls.

### II.16 Activity againgst eastern subterranean termites (Reticulitérmes flȧvipes)

Toxicant treatments (1.0% test compound w/w) are applied to 4.25 cm (diam.) filter papers in acetone solution. Treatment levels (% test compound) are calculated on basis of a mean weight per filter paper of 106.5 mg. Treatment solutions are adjusted to provide the quantity of toxicant (mg) required per paper in 213 ml of acetone. Acetone only is applied for untreated controls. Treated papers are vented to evaporate the acetone, moistened with 0.25 ml water, and enclosed in 50x9 mm Petri dishes with tight-fit lids.

Termite bioassays are conducted in 100x15 mm Petri dishes with 10 g fine sand spread in a thin layer over the bottom of each dish. An additional 2.5 g sand is piled against the side of each dish. The sand is moistened with 2.8 ml water applied to the piled sand. Water is added to dishes as needed over the course of the bioassays to maintain high moisture content. Bioassays are done with one treated filter (inside enclosure) and 30 termite workers per test dish. Each treatment level is replicated in 2 test dishes. Test dishes are maintained at about 25°C and 85% humidity for 12 days and observed daily for mortality.

### II.17 Activity against orchid thrips (dichromothrips corbetti)

Dichromothrips corbetti adults used for bioassay are obtained from a colony maintained continuously under laboratory conditions. For testing purposes, the test compound is diluted to a concentration of 500 ppm (wt compound: vol diluent) in a 1:1 mixture of acetone:water, plus 0.01 % Kinetic surfactant.

Thrips potency of each compound is evaluated by using a floral-immersion technique. Plastic petri dishes are used as test arenas. All petals of individual, intact orchid flowers are dipped into treatment solution for approximately 3 seconds and allowed to dry for 2 hours. Treated flowers are placed into individual petri dishes along with 10 - 15 adult thrips. The petri dishes are then covered with lids. All test arenas are held under continuous light and a temperature of about 28°C for duration of the assay. After 4 days, the numbers of live thrips are counted on each flower, and along inner walls of each petri dish. The level of thrips mortality is extrapolated from pre-treatment thrips numbers.

### II.18 Activity against Argentine ant, harvester ant, acrobat ant, carpenter ant, fire ant, house fly, stable fly, flesh fly, yellowfever mosquito, house mosquito, malaria mosquito, German cockroach, cat flea, and brown dog tick via glass contact

Glass vials (20 ml scintillation vials) are treated with 0.5 ml of a solution of active ingredient in acetone. Each vial is rolled uncapped for ca. 10 minutes to allow the active ingredient to completely coat the vial and to allow for full drying of the acetone. Insects or ticks are placed into each vial. The vials are kept at 22°C and are observed for treatment effects at various time intervals.

### II.19 Activity against flea beetle (Phylotretta striolata)

Flea bettle (Phylotretta striolata) adults used for bioassay are obtained from a colony maintained continuously under laboratory conditions. For testing purposes, the test compound is diluted to a concentration of 300 ppm (wt compound: vol diluent) in a 1:1 mixture of acetone:water, plus 0.1 % EL 620 surfactant.

Activity of each compound is evaluated using a lip -dip technique. Glass petri dishes (60 X 15 mm) lined with moist filter paper serves as test arenas. All leaf discs are dipped into treatment solution for approximately 3 seconds and allowed to dry for 2 hours. Each treated leaf disc is placed into individual petri dishes and inoculated with 10 adult beetles. Petri dishes are then covered with lids. All test arenas are held under continuous light and a temperature of about 28°C for duration of the assay. After 3 days, percent mortality is observed.

In this test, the compounds B85 at 300 ppm showed a mortality of at least 75% in comparison with untreated controls.

### II.20 Activity against rice green leaf hoppers (Nephotettix virescens)

Leaf hopper adults used for bioassay are obtained from a colony maintained continuously under laboratory conditions. For testing purposes, the test compound is diluted in 300 ppm (wt compound:vol diluent) in a 1:1 mixture of acetone:water, plus 0.1 % EL 620 surfactant.

Hopper activity is evaluated using the foliar spray technique. Potted rice plants (2-3 week old, Variety TN-1) are cleaned and dried prior to application. All plants are treated inside the fume hood using DeVilbiss atomizer at 25 psi with a spray volume of 5 ml /plant. To ensure uniform spray distribution, plants were placed in a rotating flat form inside the fume hood. Treated plants are then placed inside the holding room and allowed to dry for 2 hours. Each plant are caged using Mylar cages (4 inches diameter X 19 inches in height) and inoculated with 10 adults hoppers. All test plants are held under continuous light and a temperature of about 28°C for duration of the assay. Percent mortality is observed after 72 hours.

In this test, the compounds B85 at 300 ppm showed a mortality of at least 75% in comparison with untreated controls.

### III. Comparative data for the biological activity

Comparative data for the biological activity of compounds (I) according to the present invention is compiled below in table F.

**Table F**

| Assessment method | compound | mortality [%] | | |
|---|---|---|---|---|
| | | at 300 ppm | at 100 ppm | at 10 ppm |
| green peach aphid (II.2) | B14 | 90 | 90 | n.d. |
| | C1 | 60 | 0 | n.d. |
| | B24 | 100 | 90 | 90 |
| | C2 | 100 | 60 | 0 |
| diamond back moth (II.5) | B24 | 100 | n.d. | n.d. |
| | C2 | 0 | n.d. | n.d. |
| brown plant hopper (11.7) | B16 | 100 | n.d. | n.d. |
| | C2 | 0 | n.d. | n.d. |

Those results clearly demonstrate the enhanced biological activity of compounds (I) according to the present invention.

## Claims

1. Quinoline compounds of formula (I) 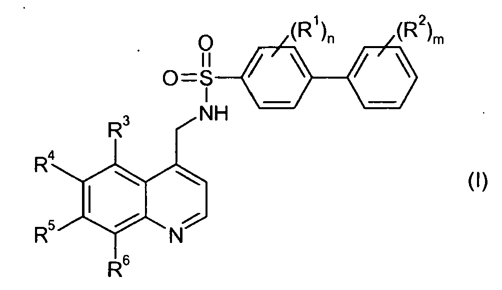 wherein
R¹, R² are each independently halogen, hydroxy, cyano, amino, nitro, C₁-C₆-alkyl, C₂-C₆-alkenyl, C₂-C₆-alkynyl, C₃-C₇-cydoalkyl, C₃-C₇-cydoalkyl-C₁-C₄-alkyl, C₁-C₆-alkoxy, C₂-C₆-alkenyloxy, C₂-C₆-alkynyloxy, C₁-C₄-alkoxy-C₁-C₄-alkoxy, C₃-C₇-cydoalkyl-C₁-C₄-alkoxy, C(OH)(CF₃)₂, C₁-C₆-haloalkyl, C₂-C₆-haloalkenyl, C₁-C₆-haloalkoxy, C₂-C₆-haloalkenyloxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, C(R^{a})=O or C(R^{a})=NOR^{b};
R^{a} is hydrogen or C₁-C₄-alkyl;
R^{b} is hydrogen, C₁-C₄-alkyl, C₂-C₄-alkenyl, C₂-C₄-alkynyl, C₁-C₄-haloalkyl, or C₂-C₄-haloalkenyl;
R³, R⁴, R⁵, R⁶ are each independently hydrogen, halogen, cyano, amino, nitro, hydroxy, C₁-C₆-alkyl, C₁-C₆-alkoxy, C₁-C₆-haloalkyl, C₁-C₆-haloalkoxy, C₁-C₆-alkylthio, C₁-C₆-haloalkylthio, C₁-C₆-alkylsulfinyl, . C₁-C₆-haloalkylsulfinyl, C₁-C₆-alkylsulfonyl, C₁-C₆-haloalkylsulfonyl, or C(=O)OR^{c};
R^{c} is hydrogen, C₁-C₆-alkyl, C₂-C₆-alkenyl, or C₂-C₆-alkynyl;
m is 0,1, 2, 3, 4, or 5;
n is 1 or 2;
and the N-oxides and salts thereof.

2. Quinoline compounds of formula (I) according to claim 1, wherein n is 1.

3. Quinoline compounds of formula (I) according to claim 2, wherein the substituent R¹ is attached to the phenyl group in meta position relative to the sulfonamid moiety.

4. Quinoline compounds of formula (I) according to claim 2, wherein the substituent R¹ is attached to the phenyl group in ortho position relative to the sulfonamid moiety.

5. Quinoline compounds of formula (I) according to any of the preceding claims, wherein R¹ is selected independently from the group consisting of F, Cl, Br, CH₃, CHF₂, CF₃, OCH₃, OCHF₂ and OCF₃.

6. Quinoline compounds of formula (I) according to any of the preceding claims, wherein m is 1, 2 or 3.

7. Quinoline compounds of formula (I) according to any of the preceding claims, wherein R² is selected independently from the group consisting of F, Cl, Br, CH₃, CHF₂, CF₃, OCH₃, OCHF₂ and OCF₃.

8. Quinoline compounds of formula (I) according to any of the proceeding claims, wherein R³, R⁴, R⁵, R⁶ are each hydrogen.

9. A process for the preparation of quinoline compounds of formula (I) as defined in claims 1 to 8, comprising:
reacting a compound of formula (II) with a boronic acid derivative of the formula (III) in the presence of a base and a transition metal catalyst to give quinoline compounds of formula (I), 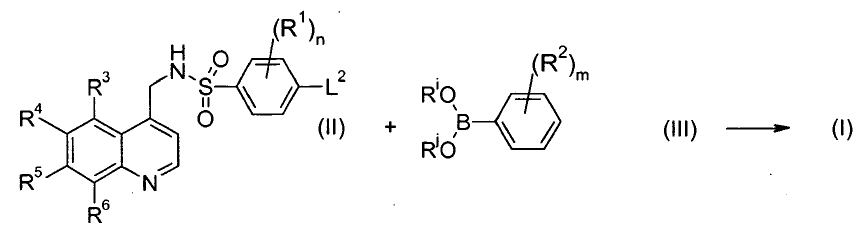 wherein the variables in the above compounds (II) and (III) have the meaning as defined above for quinoline compounds of formula (I), Rⁱ and R^{j} are each independently hydrogen or C₁-C₄-alkyl, or Rⁱ and R^{j} together form an 1,2-ethylene or 1,2-propylene moiety the carbon atoms of which may be unsubstituted or may all or in part be substituted by methyl groups, and L² is a suitable leaving group.

10. A compound of formula (II.1) 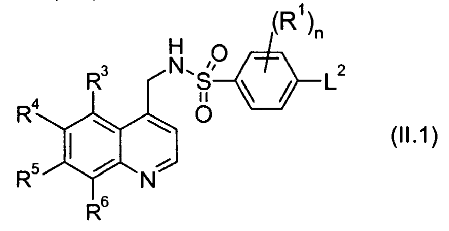 wherein
L² is chlorine, bromine or iodine and n, R¹, R³, R⁴, R⁵ and R⁶ have the meanings given in any of the claims 1 to 8.

11. Compounds of formula (I) and their N-oxides or salts as defined in any of claims 1 to 8 for use in combating arthropod pests or nematodes.

12. Compositions comprising at least one compound of formula (I) and/or a N-oxide or a salt thereof as defined in claims 1 to 8 and a carrier material.

13. A method for protecting growing plants from attack or infestation by arthropod pests or nematodes, which comprises applying to the plants, or to the soil or water in which they are growing, at least one compound of the formula (I) and/or a N-oxide or an agriculturally acceptable salt thereof as defined in claims 1 to 8.

14. A method for protecting of seed comprising contacting the seeds with at least one compound of formulae (I) and/or a N-oxide or an agriculturally acceptable salt thereof as defined in claims 1 to 8 or a composition containing at least one of these compounds in pesticidally effective amounts.

15. Seed, comprising at least one compound of formula (I) and/or a N-oxide or an agriculturally acceptable salt thereof as defined in claims 1 to 8.

16. Compounds of formula (I) as defined in any of claims 1 to 8 for use in a method for treating, controlling, preventing or protecting animals against infestation or infection by parasites which comprises administering or applying to the animals a parasiticidally effective amount of at least one compound of formula (I) and/or a N-oxide or a veterinarily acceptable salt thereof as defined in claims 1 to 8.

17. Synergistic pesticidal mixtures, comprising a compound of formula (I) and/or a N-oxide or a salt thereof as defined in claims 1 to 8 and a pesticide selected from the organo(thio)phosphates, carbamates, pyrethroids, growth regulators, neonicotinoids, nicotinic receptor agonists/antagonists compounds, GABA antagonist compounds, macrocyclic lactone insecticides, METI I, II and III compounds, oxidative phosphorylation inhibitor compounds, moulting disruptor compounds, mixed function oxidase inhibitor compounds, sodium channel blocker compounds, benclothiaz, bifenazate, cartap, flonicamid, pyridalyl, pymetrozine, sulfur, thiocyclam, flubendiamide, cyanopyrafen, flupyrazofos, cyflumetofen, amidoflumet, anthranilamides and N-R'-2,2-dihalo-1-R"-cyclopropanecarboxamide-2-(2,6-dichloro-α,α,α-trifluoro-p-tolyl)hydrazone or N-R'-2,2-di(R"')-propionamide-2-(2,6-dichloro-α,α,α-trifluoro-p-tolyl)-hydrazone, wherein R' is methyl or ethyl, halo is chloro or bromo, R" is hydrogen or methyl and R'" is methyl or ethyl.

## Patentansprüche

1. Chinolinverbindungen der Formel (I) 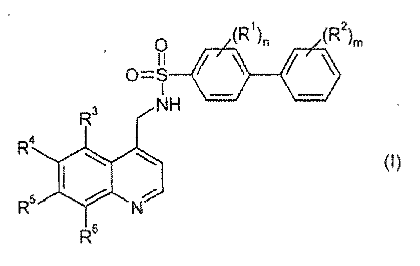 wobei
R¹, R² jeweils unabhängig Halogen, Hydroxy, Cyano, Amino, Nitro, C₁-C₆-Alkyl, C₂-C₆-Alkenyl, C₂-C₆-Alkinyl, C₃-C₇-Cycloalkyl, C₃-C₇-Cycloalkyl-C₁-C₄-alkyl, C₁-C₆-Alkoxy, C₂-C₆-Alkenyloxy, C₂-C₆-Alkinyloxy, C₁-C₄-Alkoxy-C₁-C₄-alkoxy, C₃-C₇-Cycloalkyl-Cl-C4-alkoxy, C (OH) (CF₃)₂, C₁-C₆-Halogenalkyl, C₂-C₆-Halogenalkenyl, C₁-C₆-Halogenalkoxy, C₂-C₆-Halogenalkenyloxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C (R^{a}) =O oder C (R^{a}) =NOR^{b} bedeuten;
R^{a} Wasserstoff oder C₁-C₄-Alkyl bedeutet;
R^{b} Wasserstoff, C₁-C₄-Alkyl, C₂-C₄-Alkenyl, C₂-C₄-Alkinyl, C₁-C₄-Halogenalkyl oder C₂-C₄-Halogenalkenyl bedeutet;
R³, R⁴, R⁵, R⁶ jeweils unabhängig Wasserstoff, Halogen, Cyano, Amino, Nitro, Hydroxy, C₁-C₆-Alkyl, C₁-C₆-Alkoxy, C₁-C₆-Halogenalkyl, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl oder C(=O)OR^{c} bedeuten;
R^{c} Wasserstoff, C₁-C₆-Alkyl, C₂-C₆-Alkenyl oder C₂-C₆-Alkinyl bedeutet;
m 0, 1, 2, 3, 4 oder 5 bedeutet;
n 1 oder 2 bedeutet;
sowie deren N-Oxide und Salze.

2. Chinolinverbindungen der Formel (I) nach Anspruch 1, wobei n 1 bedeutet.

3. Chinolinverbindungen der Formel (I) nach Anspruch 2, wobei der Substituent R¹ an die Phenylgruppe in meta-Stellung in Bezug auf den Sulfonamidrest gebunden ist.

4. Chinolinverbindungen der Formel (I) nach Anspruch 2, wobei der Substituent R¹ an die Phenylgruppe in ortho-Stellung in Bezug auf den Sulfonamidrest gebunden ist.

5. Chinolinverbindungen der Formel (I) nach einem der vorhergehenden Ansprüche, wobei R¹ unabhängig aus der Gruppe bestehend aus F, Cl, Br, CH₃, CHF₂, CF₃, OCH₃, OCHF₂ und OCF₃ ausgewählt ist.

6. Chinolinverbindungen der Formel (I) nach einem der vorhergehenden Ansprüche, wobei m 1, 2 oder 3 bedeutet.

7. Chinolinverbindungen der Formel (I) nach einem der vorhergehenden Ansprüche, wobei R² unabhängig aus der Gruppe bestehend aus F, Cl, Br, CH₃, CHF₂, CF₃, OCH₃, OCHF₂ und OCF₃ ausgewählt ist.

8. Chinolinverbindungen der Formel (I) nach einem der vorhergehenden Ansprüche, wobei R³, R⁴, R⁵ und R⁶ jeweils Wasserstoff bedeuten.

9. Verfahren zur Herstellung von Chinolinverbindungen der Formel (I) nach den Ansprüche 1 bis 8, umfassend die folgenden Schritte:
Umsetzen einer Verbindung der Formel (II) mit einem Boronsäurederivat der Formel (III) in Gegenwart einer Base und eines Übergangsmetallkatalysators, wobei man zu Chinolinverbindungen der Formel (I) gelangt, 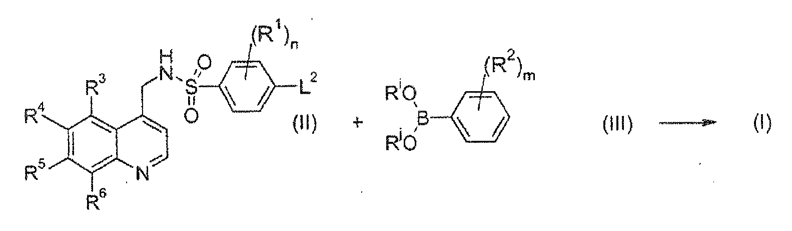 wobei die Variablen in den obengenannten Verbindungen (II) und (III) wie oben für die Chinolinverbindungen der Formel (I) definiert sind, Rⁱ und R^{j} jeweils unabhängig Wasserstoff oder C₁-C₄-Alkyl bedeuten oder Rⁱ und R^{j} gemeinsam einen 1,2-Ethylen- oder 1,2-Propylenrest bilden, dessen Kohlenstoffatome unsubstituiert sein können oder ganz oder teilweise durch Methylgruppen substituiert sein können, und L² eine geeignete Abgangsgruppe bedeutet.

10. Verbindung der Formel (II.1) 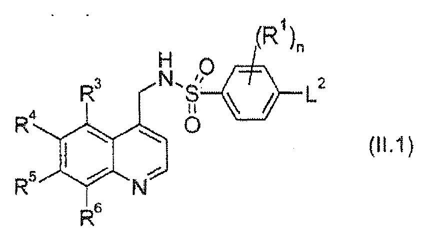 wobei
L² Chlor, Brom oder Iod bedeutet und n, R¹, R³, R⁴, R⁵ und R⁶ die in einem der Ansprüche 1 bis 8 angegebenen Bedeutungen aufweisen.

11. Verbindungen der Formel (I) und ihre N-Oxide oder Salze wie in einem der Ansprüche 1 bis 8 definiert zur Verwendung bei der Bekämpfung von Schadarthropoden oder Nematoden.

12. Zusammensetzungen umfassend mindestens eine Verbindung der Formel (I) und/oder ein N-Oxid oder Salz davon wie in den Ansprüchen 1 bis 8 definiert sowie eine Trägersubstanz.

13. Verfahren zum Schützen von wachsenden Pflanzen gegen Angriff oder Befall durch Schadarthropoden oder Nematoden, bei dem man mindestens eine Verbindung der Formel (I) und/oder ein N-Oxid oder ein landwirtschaftlich annehmbares Salz davon wie in den Ansprüchen 1 bis 8 definiert auf die Pflanzen oder auf den Boden oder das Wasser, in dem diese wachsen, ausbringt.

14. Verfahren zum Schützen von Samen, bei dem man die Samen mit mindestens einer Verbindung der Formel (I) und/oder einem N-Oxid oder einem landwirtschaftlich annehmbaren Salz davon wie in den Ansprüchen 1 bis 8 definiert oder einer Zusammensetzung, die mindestens eine dieser Verbindungen in pestizid wirksamen Mengen enthält, in Kontakt bringt.

15. Samen, umfassend mindestens eine Verbindung der Formel (I) und/oder ein N-Oxid oder ein landwirtschaftlich annehmbares Salz davon wie in den Ansprüchen 1 bis 8 definiert.

16. Verbindungen der Formel (I) wie in einem der Ansprüche 1 bis 8 definiert zur Verwendung in einem Verfahren zum Behandeln oder Schützen von Tieren gegen Befall oder Infektion durch Parasiten oder für die Bekämpfung von bzw. Vorbeugung gegen Befall order Infektion durch Parasiten, bei dem man eine parasitizid wirksame Menge von mindestens einer Verbindung der Formel (I) und/oder einem N-Oxid oder einem tierärztlich annehmbaren Salz davon wie in den Ansprüchen 1 bis 8 definiert den Tieren verabreicht bzw. auf die Tiere ausbringt.

17. Synergistische pestizide Mischungen umfassend eine Verbindung der Formel (I) und/oder ein N-Oxid oder ein Salz davon wie in den Ansprüchen 1 bis 8 definiert sowie ein Pestizid, ausgewählt aus Organo(thio)phosphaten, Carbamaten, Pyrethroiden, Wachstumsregulatoren, Neonikotinoiden, Nikotinrezeptoragonisten/-antagonisten, GABA-Antagonisten, makrozyklischen Lacton-Insektiziden, METI I-, -II- und -III-Verbindungen, Hemmern der oxidativen Phosphorylierung, Verbindungen mit Einwirkung auf die Häutung, Natriumkanalblockern, Benclothiaz, Bifenazat, Cartap, Flonicamid, Pyridalyl, Pymetrozin, Schwefel, Thiocyclam, Flubendiamid, Cyanopyrafen, Flupyrazofos, Cyflumetofen, Amidoflumet, Anthranilamiden und N-R'-2,2-Dihalogen-1-R"-cyclopropancarboxamid-2-(2,6-dichlor-α,α,α-trifluor-p-tolyl)hydrazon oder N-R'2,2-Di(R''')-propionamid-2-(2,6-dichlor-α,α,α-trifluor-p-tolyl)hydrazon, wobei R' Methyl oder Ethyl bedeutet, Halogen Chlor oder Brom bedeutet, R" Wasserstoff oder Methyl bedeutet und R"' Methyl oder Ethyl bedeutet.

## Revendications

1. Composés de quinoléine de formule (I) 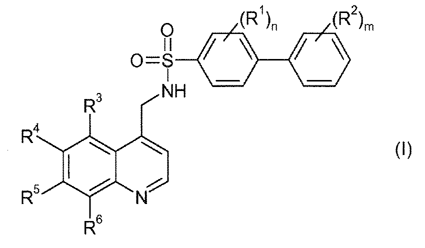 dans laquelle
R¹, R² sont chacun indépendamment halogène, hydroxy, cyano, amino, nitro, C₁-C₆-alkyle, C₂-C₆-alcényle, C₂-C₆-alcynyle, C₃-C₇-cycloalkyle, C₃-C₇-cycloalkyl-C₁-C₄-alkyle, C₁-C₆-alcoxy, C₂-C₆-alcényloxy, C₂-C₆-alcynyloxy, C₁-C₄-alcoxy-C₁-C₄-alcoxy, C₃-C₇-cycloalkyl-C₁-C₄-alcoxy, C (OH) (CF₃) ₂, C₁-C₆-halogénoalkyle, C₂-C₆-halogénoalcényle, C₁-C₆-halogénoalcoxy, C₂-C₆-halogénoalcényloxy, C₁-C₆-alkylthio, C₁-C₆-halogénoalkylthio, C₁-C₆-alkylsulfinyle, C₁-C₆-halogénoalkylsulfinyle, C₁-C₆-alkylsulfonyle, C₁-C₆-halogénoalkylsulfonyle, C(R^{a})=O ou C(R^{a}) =NOR^{b} ;
R^{a} est hydrogène ou C₁-C₄-alkyle ;
R^{b} est hydrogène, C₁-C₄-alkyle, C₂-C₄-alcényle, C₂-C₄-alcynyle, C₁-C₄-halogénoalkyle, ou C₂-C₄-halogénoalcényle ;
R³, R⁴, R⁵, R⁶ sont chacun indépendamment hydrogène, halogène, cyano, amino, nitro, hydroxy, C₁-C₆-alkyle, C₁-C₆-alcoxy, C₁-C₆-halogénoalkyle, C₁-C₆-halogénoalcoxy, C₁-C₆-alkylthio, C₁-C₆-halogénoalkylthio, C₁-C₆-alkylsulfinyle, C₁-C₆-halogénoalkylsulfinyle, C₁-C₆-alkylsulfonyle, C₁-C₆-halogénoalkylsulfonyle, ou C(=O)OR^{c} ;
R^{c} est hydrogène, C₁-C₆-alkyle, C₂-C₆-alcényle, ou C₂-C₆-alcynyle ;
m vaut 0, 1, 2, 3, 4, ou 5 ;
n vaut 1 ou 2 ;
et les N-oxydes et sels de ceux-ci.

2. Composés de quinoléine de formule (I) selon la revendication 1, dans laquelle n vaut 1.

3. Composés de quinoléine de formule (I) selon la revendication 2, dans laquelle le substituant R¹ est fixé au groupement phényle en position *méta* par rapport au motif sulfonamide.

4. Composés de quinoléine de formule (I) selon la revendication 2, dans laquelle le substituant R¹ est fixé au groupement phényle en position ortho par rapport au motif sulfonamide.

5. Composés de quinoléine de formule (I) selon l'une quelconque des revendications précédentes, dans laquelle R¹ est choisi indépendamment parmi le groupe constitué par F, Cl, Br, CH₃, CHF₂, CF₃, OCH₃, OCHF₂ et OCF₃.

6. Composés de quinoléine de formule (I) selon l'une quelconque des revendications précédentes, dans laquelle m vaut 1, 2 ou 3.

7. Composés de quinoléine de formule (I) selon l'une quelconque des revendications précédentes, dans laquelle R² est choisi indépendamment parmi le groupe constitué par F, Cl, Br, CH₃, CHF₂, CF₃, OCH₃, OCHF₂ et OCF₃.

8. Composés de quinoléine de formule (I) selon l'une quelconque des revendications précédentes, dans laquelle R³, R⁴, R⁵, R⁶ sont chacun hydrogène.

9. Procédé de préparation de composés de quinoléine de formule (I) telle que définie selon les revendications 1 à 8, comprenant :
la réaction d'un composé de formule (II) avec un dérivé d'acide boronique de formule (III) en présence d'une base et d'un catalyseur à base de métaux de transition afin de donner les composés de quinoléine de formule (I), 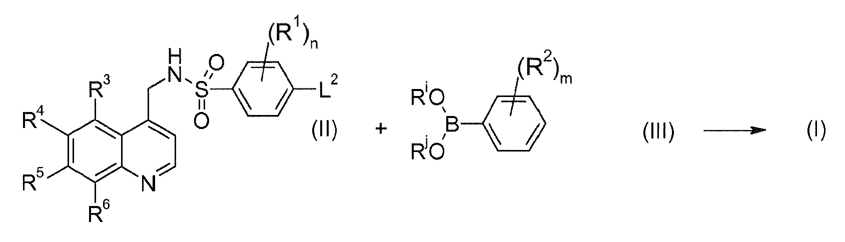
où les variables dans les composés (II) et (III) ci-dessus ont la signification telle que définie ci-dessus pour les composés de quinoléine de formule (I), Rⁱ et R^{j} sont chacun indépendamment hydrogène ou C₁-C₄-alkyle, ou Rⁱ et R^{j} forment ensemble un motif 1,2-éthylène ou 1,2-propylène dont les atomes de carbone peuvent être non substitués ou peuvent être en partie ou totalement substitués par des groupements méthyle, et L² est un groupement partant convenable.

10. Composé de formule (II.1) 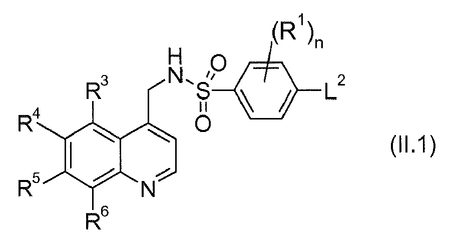 dans laquelle
L² est chlore, brome ou iode et n, R¹, R³, R⁴, R⁵ et R⁶ ont les significations données selon l'une quelconque des revendications 1 à 8.

11. Composés de formule (I) et leurs N-oxydes ou sels tels que définis selon l'une quelconque des revendications 1 à 8, pour une utilisation dans la lutte contre les arthropodes nuisibles ou les nématodes.

12. Compositions comprenant au moins un composé de formule (I) et/ou un N-oxyde ou sel de celui-ci tel que défini selon les revendications 1 à 8 et un matériau véhicule.

13. Méthode de protection de plantes en croissance contre une attaque ou une infestation par des arthropodes nuisibles ou des nématodes, comprenant l'application aux plantes, ou au sol ou à l'eau dans lesquels elles poussent, d'au moins un composé de formule (I) et/ou d'un N-oxyde ou d'un sel acceptable sur le plan agricole de celui-ci tel que défini selon les revendications 1 à 8.

14. Méthode de protection de semences, comprenant la mise en contact des semences avec au moins un composé de Formules (I) et/ou un N-oxyde ou un sel acceptable sur le plan agricole de celui-ci tel que défini selon les revendications 1 à 8, ou une composition contenant au moins l'un de ces composés selon des quantités efficaces sur le plan pesticide.

15. Semence, comprenant au moins un composé de Formule (I) et/ou un N-oxyde ou un sel acceptable sur le plan agricole de celui-ci tel que défini selon les revendications 1 à 8.

16. Composés de formule (I) tels que définis selon l'une quelconque des revendications 1 à 8, pour une utilisation dans une méthode de traitement ou de protection d'animaux contre une infestation ou une infection par des parasites, ou de contrôle ou de prévention d'une infestation ou d'une d'infection d'animaux par des parasites, comprenant l'administration ou l'application aux animaux d'une quantité efficace sur le plan parasiticide d'au moins un composé de Formule (I) et/ou d'un N-oxyde ou d'un sel acceptable sur le plan vétérinaire de celui-ci tel que défini selon les revendications 1 à 8.

17. Mélanges pesticides synergiques, comprenant un composé de Formule (I) et/ou un N-oxyde ou un sel de celui-ci tel que défini selon les revendications 1 à 8, et un pesticide choisi parmi les organo(thio)-phosphates, les carbamates, les pyréthroïdes, les substances de croissance, les néonicotinoïdes, les composés agonistes/antagonistes du récepteur nicotinique, les composés antagonistes du GABA, les insecticides à base de lactone macrocyclique, les composés METI I, II et III, les composés inhibiteurs de la phosphorylation oxydative, les composés perturbant la mue, les composés inhibiteurs de l'oxydase à fonction mixte, les composés bloquant le canal sodique, le benclothiaz, le bifénazate, le cartap, le flonicamide, le pyridalyle, la pymétrozine, le soufre, le thiocyclam, le flubendiamide, le cyanopyrafen, le flupyrazofos, le cyflumetofen, l'amidoflumet, les anthranilamides et la N-R'-2,2-dihalogéno-1-R"-cyclopropanecarboxamide-2-(2,6-dichloro-α,α,α-trifluoro-p-tolyl)hydrazone ou la N-R'-2,2-di(R''')-propionamide-2-(2,6-dichloro-α,α,α-trifluoro-p-tolyl)-hydrazone, où R' est méthyle ou éthyle, halogéno est chloro ou bromo, R" est hydrogène ou méthyle et R"' est méthyle ou éthyle.
